(19) Europäisches Patentamt
European Patent Office
Office européen des brevets

(11) Veröffentlichungsnummer: **0 002 010**
**B1**

# (12) EUROPÄISCHE PATENTSCHRIFT

(45) Veröffentlichungstag der Patentschrift: **14.07.82**

(21) Anmeldenummer: **78101299.2**

(22) Anmeldetag: **03.11.78**

(51) Int. Cl.³: **C 07 D 233/50,** C 07 D 401/12, C 07 D 405/12, C 07 D 409/12, C 07 D 413/12, A 61 K 31/415, C 07 C 83/00, C 07 C 157/09, C 07 C 157/14, C 07 D 207/40, C 07 D 209/48

(54) **Neue 2-Imino-imidazolidin-Derivate, Verfahren und Zwischenprodukte zu deren Herstellung und pharmazeutische Präparate enthaltend ein solches 2-Imino-imidazolidin-Derivat sowie die Herstellung dieser Präparate.**

(30) Priorität: **07.11.77 LU 78467**
**15.09.78 CH 9668/78**

(43) Veröffentlichungstag der Anmeldung: **30.05.79 Patentblatt 79/11**

(45) Bekanntmachung des Hinweises auf die Patenterteilung: **14.07.82 Patentblatt 82/28**

(84) Benannte Vertragsstaaten: **BE CH DE FR LU NL SE**

(56) Entgegenhaltungen:
**DD-A-126 907**
**DE-A-1 545 628**
**DE-A-1 670 274**
**DE-A-1 815 788**
**DE-A-2 058 826**
**DE-A-2 140 405**
**DE-A-2 523 103**
**US-A-3 236 857**

(73) Patentinhaber: **F. HOFFMANN-LA ROCHE & CO. Aktiengesellschaft, CH-4002 Basel (CH)**

(72) Erfinder: **Ramuz, Henri, Dr., Rheinparkstrasse 3, CH-4127 Birsfelden (CH)**

(74) Vertreter: **Lederer, Franz, Dr. et al, Patentanwälte Lederer, Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Neue 2-Imino-imidazolidin-Derivate, Verfahren und Zwischenprodukte zu deren Herstellung und pharmazeutische Präparate enthaltend ein solches 2-Imino-imidazolidin-Derivat sowie die Herstellung dieser Präparate

Die vorliegende Erfindung betrifft neue 2-Imino-imidazolidin-Derivate der allgemeinen Formel

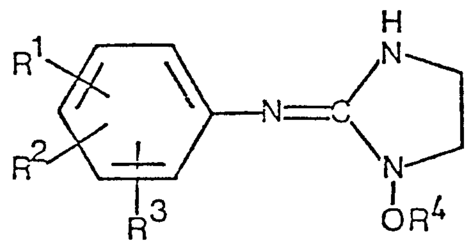

I

worin $R^1$, $R^2$ und $R^3$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy und $R^4$ Wasserstoff, $C_1-C_6$-Alkyl, $C_3-C_6$-Cycloalkyl, $C_2-C_6$-Alkenyl, $C_2-C_6$-Alkinyl, Di-$C_1-C_6$-alkylamino-$C_1-C_6$-alkyl, Carboxy-$C_1-C_6$-alkyl, Carboxy-$C_2-C_6$-alkenyl, Hydroxy-$C_1-C_6$-alkyl, Cyano-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, Mono-$C_1-C_6$-alkylaminocarbonyl-$C_1-C_6$-alkyl,Di-$C_1-C_6$-alkylaminocarbonyl-$C_1-C_6$-alkyl, $C_1-C_6$-Alkoxycarbonyl-$C_2-C_6$-alkenyl, $C_1-C_6$-Alkoxy-$C_1-C_6$-alkyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl, (2,2,8-Trimethyl-4H-m-dioxino[4,5-c]pyridin-5-yl)methyl, [5-Hydroxy-4-(hydroxymethyl)-6-methyl-3-pyridyl]methyl, $\alpha$-Carboxybenzyl, $\alpha$-$C_1-C_6$-Alkoxycarbonyl-$\alpha$-$C_1-C_6$-alkylphenyl oder einen über eine $-CH(R^5)$-Gruppe gebundenen, gegebenenfalls durch $C_1-C_6$-Alkyl oder die Gruppe $-COOR$ substituierten Thienyl-, Furyl- oder Isoxazolylrest oder einen gegebenenfalls durch $C_1-C_6$-Alkyl, $C_1-C_6$-Alkoxy oder die Gruppe $-COOR$ substituierten Pyridylrest und $R^5$ Wasserstoff, Methyl, Äthyl oder n-Propyl und R Wasserstoff oder $C_1-C_6$-Alkyl bedeuten,
sowie pharmazeutisch anwendbare Säureadditionssalze davon.

Die Erfindung betrifft ebenfalls ein Verfahren zur Herstellung dieser Verbindungen.

Aus der DD-A 126 907 und der US-A 3 236 857 sind 2-Imino-imidazolidin-Derivate bekannt, welche an einem Imidazolidin-Stickstoffatom durch Alkyl oder Acyl substituiert sein können. Diese Verbindungen besitzen wertvolle pharmakologische Eigenschaften, insbesondere blutdrucksenkende, sedative und teilweise auch vasokonstriktive Eigenschaften. In der am 7. September 1978 offengelegten DE-A 2 709 720 sind gewisse, unter die obige Formel I fallende Verbindungen als Blutdrucksenker beschrieben. Im Hinblick auf diese Druckschrift gelten für die Bundesrepublik Deutschland eingeschränkte Patentansprüche.

Der Ausdruck »$C_1-C_6$-Alkyl« — allein oder in Kombination — bedeutet im Rahmen der vorliegenden Erfindung geradkettige und verzweigte Alkylgruppen mit 1—6 Kohlenstoffatomen, wie Methyl, Äthyl, n-Propyl, Isopropyl, n-Butyl, Isobutyl, tert.-Butyl, Amyl, Hexyl u. dgl. Der Ausdruck »$C_3-C_6$-Cycloalkyl« bezieht sich auf cyclische, gesättigte Kohlenwasserstoffreste mit 3—6 Kohlenstoffatomen, wie Cyclopropyl, Cyclohexyl u. dgl. Der Ausdruck »$C_1-C_6$-Alkoxy« bedeutet $C_1-C_6$-Alkyläthergruppen, worin der Ausdruck »$C_1-C_6$-Alkyl« die obige Bedeutung hat. Der Ausdruck »$C_2-C_6$-Alkenyl« umfaßt geradkettige und verzweigte Kohlenwasserstoffgruppen mit 2—6 Kohlenstoffatomen, worin mindestens eine Kohlenstoff-Kohlenstoffbindung ungesättigt ist, wie Allyl, Butenyl u. dgl. Der Ausdruck »$C_2-C_6$-Alkinyl« bezieht sich in ähnlicher Weise auf geradkettige und verzweigte Kohlenwasserstoffgruppen mit 2—6 Kohlenstoffatomen, worin mindestens eine Kohlenstoff-Kohlenstoff-Dreifachbindung vorhanden ist, wie Propargyl u. dgl. Es ist darauf hinzuweisen, daß sich eine Doppel- bzw. Dreifachbindung nicht in der 1,2-Stellung befinden kann. Der Ausdruck »Halogen« umfaßt die vier Halogenatome Fluor, Chlor, Brom und Jod. Der im Nachfolgenden verwendete Ausdruck »austretende Gruppe« bedeutet im Rahmen der vorliegenden Erfindung bekannte Gruppen wie Halogen, vorzugsweise Chlor oder Brom, Arylsulfonyloxy wie etwa Mesyloxy, quaternäre Ammonium- und Sulfoniumsalze u. dgl.

Eine bevorzugte Klasse von Verbindungen der Formel I sind solche, worin $R^3$ Wasserstoff bedeutet. Weiters sind solche Verbindungen der Formel I bevorzugt, worin $R^1$ und $R^2$ in 2,6-Stellung des Phenylringes lokalisiert sind. $R^1$ und $R^2$ haben bevorzugt die gleiche Bedeutung und bedeuten vorzugsweise Halogen, besonders bevorzugt Chlor.

Eine weitere Klasse von bevorzugten Verbindungen der Formel I sind solche, worin $R^4$ $C_2-C_6$-Alkinyl, vorzugsweise mit 3 bis 5 Kohlenstoffatomen, Carboxy-$C_1-C_6$-alkyl, vorzugsweise Carboxymethyl oder Carboxypropyl, $C_1-C_6$-Alkoxycarbonyl-$C_2-C_6$-alkenyl, vorzugsweise Äthoxycarbonyl-$C_2-C_6$-alkenyl, oder einen über eine $-CH(R^5)$-Gruppe gebundenen, gegebenenfalls durch $C_1-C_6$-Alkyl oder die Gruppe $-COOR$ substituierten Thienyl-, Furyl-, Isoxazolyl- oder Pyridylrest und $R^5$ Wasserstoff, Methyl, Äthyl oder n-Propyl und R Wasserstoff oder $C_1-C_6$-Alkyl bedeuten;

vorzugsweise handelt es sich um Pyridyl oder einen unsubstituierten Thienyl-, Furyl- oder Isoxazolylrest. Ganz besonders bevorzugt ist $R^4$ in der Bedeutung Propargyl, Carboxymethyl, unverzweigtes Äthoxycarbonyl-$C_2-C_6$-alkenyl und über $-CH(R^5)-$ gebundenes Pyridyl oder Furyl. $R^5$ bedeutet vorzugsweise Wasserstoff oder Methyl.

Aus dem obigen folgt, daß diejenigen Verbindungen der Formel I ganz besonders bevorzugt sind, worin $R^3$ Wasserstoff, $R^1$ und $R^2$ Halogen, vorzugsweise Chlor, in 2,6-Stellung, $R^4$ Propargyl, Carboxymethyl, unverzweigtes Äthoxycarbonyl-$C_2-C_6$-alkenyl oder über $-CH(R^5)-$ gebundenes Pyridyl oder Furyl und $R^5$ Wasserstoff oder Methyl bedeuten.

Die bevorzugtesten Verbindungen der Formel I sind:

2-[(2,6-Dichlorphenyl)imino]-1-hydroxyimidazolidin,
2-[(2,6-Dibromphenyl)imino]-1-hydroxyimidazolidin,
1-Hydroxy-2-[(2-iodphenyl)imino]imidazolidin,
Äthyl 4-[/2-[(2,6-dichlorphenyl)imino]-1-imidazolidinyl/oxy]butyrat,
4-[/2-[(2,6-Dichlorphenyl)imino]-1-imidazolidinyl/oxy]buttersäure,
Äthyl 2-[/2-[(2,6-dichlorphenyl)imino]-1-imidazolidinyl/oxy]butyrat,
2-[(2,6-Dichlorphenyl)imino]-1-(2-propinyloxy)-imidazolidin,
[/2-[(2,6-Dichlorphenyl)imino]-1-imidazolidinyl/oxy]essigsäure,
Äthyl 4-[/2-[(2,6-dichlorphenyl)imino]-1-imidazolidinyl/oxy]crotonat,
2-[(2,6-Dichlorphenyl)imino]-1-äthoxyimidazolidin,
1-(2-Butinyloxy)-2-[(2,6-dichlorphenyl)imino]imidazolidin,
2-[(2,6-Dichlorphenyl)imino]-1-(furfuryloxy)-imidazolidin,
2-[/⟨2-[(2,6-Dichlorphenyl)imino]-1-imidazolinyl⟩oxy]methyl/pyridin,
3-[/⟨2-[(2,6-Dichlorphenyl)imino]-1-imidazolinyl⟩oxy]methyl/pyridin,
2-[/⟨2-[(2,6-Dichlor-4-fluorphenyl)imino]-1-imidazolinyl⟩oxy]methyl/pyridin,
5-[/⟨2-[(2,6-Dichlorphenyl)imino]-1-imidazolinyl⟩oxy]methyl/-3-hydroxy-2-methyl-4-pyridinmethanol.

Die Verbindungen der obigen Formel I und deren pharmazeutisch anwendbare Säureadditionssalze können erfindungsgemäß so hergestellt werden, daß man

a) eine Verbindung der allgemeinen Formel

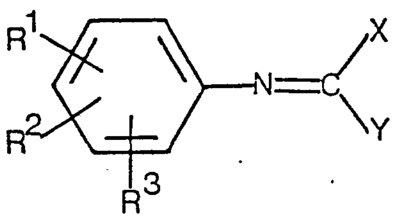

II

worin X und Y je Halogen, eine Sulfhydryl-, Amino-, Methoxy- oder Alkylthiogruppe mit 1–3 Kohlenstoffatomen bedeuten und $R^1$, $R^2$ und $R^3$ die obige Bedeutung besitzen, oder eine Verbindung der allgemeinen Formel

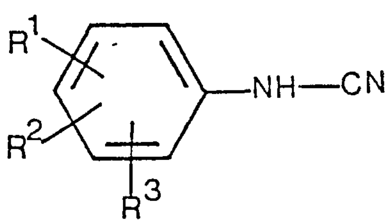

III

worin $R^1$, $R^2$ und $R^3$ die obige Bedeutung besitzen, mit einer Verbindung der allgemeinen Formel

$$H_2N-CH_2-CH_2-NH-OR^4 \qquad IV$$

worin $R^4$ die obige Bedeutung besitzt, umsetzt, oder

b) zur Herstellung von Verbindungen der Formel I, worin $R^4$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl-

oder Pyridylrest bedeutet und $R^1$, $R^2$, $R^3$ und $R^5$ die obige Bedeutung besitzen, eine Verbindung der allgemeinen Formel

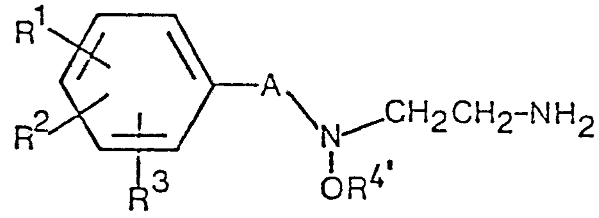

V

worin A die Gruppe

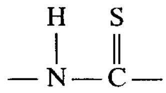

oder

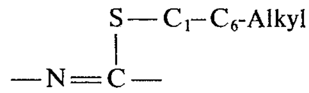

und $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest bedeutet und $R^1$, $R^2$, $R^3$ und $R^5$ die obige Bedeutung besitzen,
cyclisiert, oder

c) zur Herstellung von Verbindungen der Formel I, worin $R^4$ Wasserstoff bedeutet und $R^1$, $R^2$ und $R^3$ die obige Bedeutung besitzen, in einer Verbindung der allgemeinen Formel

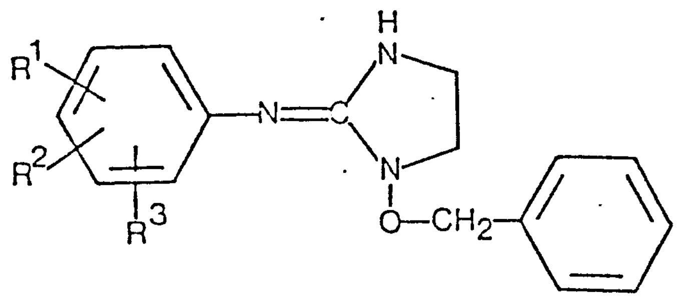

Ia

worin $R^1$, $R^2$ und $R^3$ die obige Bedeutung besitzen, die Benzylgruppe abspaltet, oder

d) zur Herstellung von Verbindungen der allgemeinen Formel

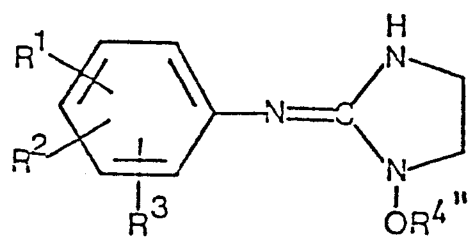

Ib

worin $R^{4''}$ $C_1-C_6$-Alkyl, $C_3-C_6$-Cycloalkyl, $C_2-C_6$-Alkenyl, $C_2-C_6$-Alkinyl, Di-$C_1-C_6$-alkylamino-$C_1-C_6$-alkyl, Carboxy-$C_1-C_6$-alkyl, Hydroxy-$C_1-C_6$-alkyl, Cyano-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, Mono-$C_1-C_6$-alkylaminocarbonyl-$C_1-C_6$-alkyl, Di-$C_1-C_6$-alkylaminocarbonyl-$C_1-C_6$-alkyl, $C_1-C_6$-Alkoxycarbonyl-$C_2-C_6$-alkenyl, $C_1-C_6$-Alkoxy-$C_1-C_6$-alkyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl oder einen über eine $-CH(R^5)$-Gruppe gebundenen, gegebenenfalls durch $C_1-C_6$-Alkyl oder die Gruppe $-COOR$ substituierten Thienyl-, Furyl-, Isoxazolyl- oder Pyridylrest bedeutet und R, $R^1$, $R^2$, $R^3$ und $R^5$ die obige Bedeutung besitzen,
eine Verbindung der allgemeinen Formel

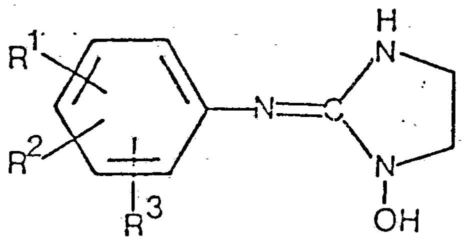

Ic

worin $R^1$, $R^2$ und $R^3$ die obige Bedeutung besitzen, mit einer Verbindung der allgemeinen Formel

$R^{4''}-X$ VI

worin X eine austretende Gruppe bedeutet und $R^{4''}$ die obige Bedeutung besitzt, umsetzt, oder

e) zur Herstellung von Verbindungen der Formel I, worin $R^4$ Carboxy-$C_1-C_6$-alkyl oder Carboxy-$C_2-C_6$-alkenyl bedeutet und $R^1$, $R^2$ und $R^3$ die obige Bedeutung besitzen, eine entsprechende Verbindung der Formel I, worin $R^4$ $C_1-C_6$-Alkoxycarbonyl-$C_1-C_6$-alkyl oder $C_1-C_6$-Alkoxycarbonyl-$C_2-C_6$-alkenyl bedeutet und $R^1$, $R^2$ und $R^3$ die obige Bedeutung besitzen, hydrolysiert, oder

f) zur Herstellung von Verbindungen der Formel I, worin $R^4$ $C_1-C_6$-Alkoxycarbonyl-$C_1-C_6$-alkyl oder $C_1-C_6$-Alkoxycarbonyl-$C_2-C_6$-alkenyl bedeutet und $R^1$, $R^2$ und $R^3$ die obige Bedeutung besitzen, eine entsprechende Verbindung der Formel I, worin $R^4$ $C_1-C_6$-Alkoxycarbonyl-$C_1-C_6$-alkyl oder $C_1-C_6$-Alkoxycarbonyl-$C_2-C_6$-alkenyl bedeutet, wobei jedoch der Alkoxyrest verschieden ist, und $R^1$, $R^2$ und $R^3$ die obige Bedeutung besitzen, mit einem $C_1-C_6$-Alkanol umestert, oder

g) zur Herstellung von Verbindungen der obigen Formel I, worin $R^4$ Aminocarbonyl-$C_1-C_6$-alkyl bedeutet und $R^1$, $R^2$ und $R^3$ die obige Bedeutung besitzen, eine entsprechende Verbindung der Formel I, worin $R^4$ $C_1-C_6$-Alkoxycarbonyl-$C_1-C_6$-alkyl bedeutet und $R^1$, $R^2$ und $R^3$ die obige Bedeutung besitzen, mit Ammoniak, primärem Amin oder sekundärem Amin umsetzt, oder

h) zur Herstellung von Verbindungen der Formel I, worin $R^4$ Wasserstoff bedeutet und $R^1$, $R^2$ und $R^3$ die obige Bedeutung besitzen, eine Verbindung der allgemeinen Formel

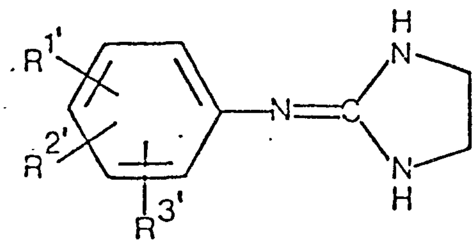

VII

worin $R^{1'}$, $R^{2'}$ und $R^{3'}$ unabhängig voneinander je Wasserstoff, $C_1-C_6$-Alkyl, $C_1-C_6$-Alkoxy, Halogen oder Trifluormethyl bedeuten, mit Kaliumpersulfat in konz. Schwefelsäure oxydiert, oder

i) zur Herstellung von Verbindungen der obigen Formel I, worin $R^4$ [5-Hydroxy-4-(hydroxymethyl)-6-methyl-3-pyridyl]-methyl bedeutet und $R^1$, $R^2$ und $R^3$ die in Anspruch 1 angegebene Bedeutung besitzen, in einer Verbindung der allgemeinen Formel

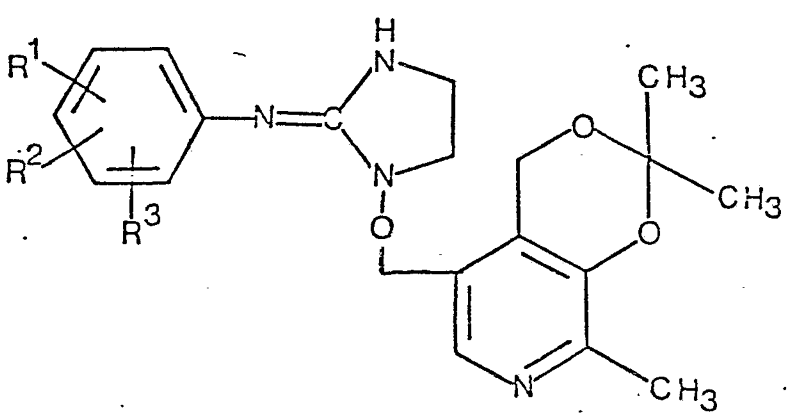

Id

worin $R^1$, $R^2$ und $R^3$ die obige Bedeutung besitzen, die Ketalgruppe abspaltet, und

j) erwünschtenfalls eine erhaltene Verbindung oder ein pharmazeutisch nicht anwendbares Säureadditionssalz davon in ein pharmazeutisch anwendbares Säureadditionssalz davon überführt.

Die Umsetzung einer Verbindung der Formel II bzw. III mit einer Verbindung der Formel IV kann in an sich bekannter Weise durchgeführt werden. Zweckmäßig erfolgt die Reaktion in einem unter den Reaktionsbedingungen inerten organischen Lösungsmittel, oder für die Umsetzung einer Verbindung der Formel II auch in einem Zweiphasensystem wie Wasser/Toluol u. dgl., bei einer Temperatur zwischen etwa 0°C und 180°C, gegebenenfalls in Abhängigkeit von den Resten X und Y. Als Lösungsmittel kommen allenfalls in Abhängigkeit von den Resten X und Y unpolare oder polare aprotische Lösungsmittel, oder für die Umsetzung einer Verbindung der Formel II auch polare protische Lösungsmittel in Frage wie etwa Äther, z. B. Diäthyläther, Tetrahydrofuran, Dioxan u. dgl., aromatische Kohlenwasserstoffe, z. B. Benzol, Toluol, Xylol u. dgl., halogenierte Kohlenwasserstoffe, z. B. Methylenchlorid, Chloroform u. dgl., Ester von Alkansäuren, z. B. Essigester u. dgl., Alkohole, z. B. tert. Butanol, Amylalkohol u. dgl., Acetonitril u. dgl. Die Reaktion erfolgt vorzugsweise in Gegenwart eines säurebindenden Mittels wie Triäthylamin, N-Äthyl-N,N-diisopropylamin, Pyridin und Carbonate u. dgl., falls einer der Reste X und Y ein Halogenatom bedeutet. Die Reaktionsdauer richtet sich nach der Reaktivität der eingesetzten Ausgangsstoffe und liegt zwischen einigen Minuten und mehreren Stunden.

Die Cyclisation einer Verbindung der Formel V erfolgt ebenfalls in an sich bekannter Weise, beispielsweise durch Erwärmen auf Temperaturen zwischen etwa 25° und 200°C, vorzugsweise zwischen etwa 35° und 120°C. Die Reaktion kann in Abwesenheit oder Gegenwart eines Lösungsmittels durchgeführt werden. Für diesen Zweck geeignete Lösungsmittel sind chlorierte Kohlenwasserstoffe wie Methylenchlorid, Äther wie Tetrahydrofuran, aromatische Kohlenwasserstoffe wie Toluol, Acetonitril, Dimethylformamid u. dgl. Die Cyclisation kann auch dadurch erwirkt werden, daß man die Verbindungen der Formel V, vorzugsweise diejenigen, worin A die Gruppe

$$\begin{array}{l} -N{=}C- \\ \qquad\;\; | \\ \qquad\;\; S-C_1\text{–}C_6\text{-Alkyl} \end{array}$$

bedeutet, in einem polaren Lösungsmittel mit einem geeigneten basischen Kondensationsmittel bei einer Temperatur zwischen etwa 0° C und der Rückflußtemperatur des Reaktionsgemisches behandelt. Als geeignete basische Kondensationsmittel können Ammoniumacetat, Methylamin, Pyridin u. dgl. genannt werden. Als polare Lösungsmittel kommen Wasser, Alkohole wie Methanol und aprotische Lösungsmittel wie Acetonitril u. dgl. in Frage.

Die Abspaltung der Benzylgruppe aus einer Verbindung der Formel Ia erfolgt nach an sich bekannten Methoden durch Behandlung mit einer Halogenwassserstoffsäure, vorzugsweise Bromwasserstoffsäure, oder durch katalytische Hydrierung. Die Umsetzung mit einer Halogenwasserstoffsäure erfolgt zweckmäßig in einem unter den Reaktionsbedingungen inerten Lösungsmittel, vorzugsweise Wasser, bei einer Temperatur zwischen etwa Raumtemperatur und 180° C, vorzugsweise 100°C. Die katalytische Hydrierung wird bevorzugt in Gegenwart eines Katalysators wie Palladium, Palladium/Kohle oder Platinoxyd in einem inerten Lösungsmittel wie einem Alkohol, z. B. Methanol, Äthanol, einer Carbonsäure wie Essigsäure, Wasser oder Gemische davon und bei einer Temperatur zwischen etwa 15° und 50° C durchgeführt. Aus Zweckmäßigkeitsgründen arbeitet man bevorzugt bei Raumtemperatur.

Die O-Substitution einer Verbindung der obigen Formel Ic erfolgt nach an sich bekannten Methoden in Gegenwart einer Base wie Natriumhydrid, Natriumamid, Kalium-t.-butylat, Natriumäthylat, Thalliumäthylat u. dgl. in einem aprotisch polaren Lösungsmittel wie Dimethylformamid, Dimethylsulfoxyd, Acetonitril u. dgl., einem aprotisch apolaren Lösungsmittel wie Äther, z. B. Tetrahydrofuran oder Diäthyläther, aromatische Kohlenwasserstoffe, z. B. Toluol oder Xylol, Cyclohexan u. dgl., einem protisch polaren Lösungsmittel wie Alkohole, z. B. t.-Butanol oder Isopropanol u. dgl. oder flüssigem Ammoniak u. dgl. Die Wahl des Lösungsmittels hängt von der verwendeten Base ab; so wird die Reaktion bei Verwendung von Alkoholaten in einem protisch polaren oder aprotisch apolaren Lösungsmittel, bei Verwendung von Natriumhydrid in einem aprotisch polaren Lösungsmittel und bei Verwendung von Natriumamid in einem aprotisch polaren Lösungsmittel oder flüssigem Ammoniak durchgeführt. Die Umsetzung erfolgt bei einer Temperatur zwischen etwa −50° und 100°C, vorzugsweise zwischen etwa 20° und 45°C, in Abhängigkeit von der verwendeten Base. Bei Verwendung von Thalliumäthylat als Base bedeutet X in der Verbindung der Formel VI vorzugsweise Jod. Die O-Substitution kann auch unter Anwendung der sogenannten Phasentransfer-Katalysé (PTC) durchgeführt werden (vgl. z. B. Angew. Chem. 89, 521 [1975]). In diesem Falle wird als Base vorzugsweise Natriumhydroxyd oder Natriumcarbonat in einem Zweiphasensystem, z. B. Methylenchlorid/Wasser, in Gegenwart eines Salzes wie Tetrabutylammoniumhydrogensulfat verwendet. Aus Zweckmäßigkeitsgründen arbeitet man bevorzugt bei Raumtemperatur.

Die Esterhydrolyse gemäß Verfahrensvariante e) wird nach bekannten Methoden durch Behandlung

mit einer Säure oder Base durchgeführt. Für diesen Zweck geeignete Säuren und Basen sind Schwefelsäure, eine Halogenwasserstoffsäure wie die Chlorwasserstoffsäure u. dgl. und Alkalimetallhydroxyde, z. B. Natriumhydroxyd oder Kaliumhydroxyd, u. dgl. Die Hydrolyse wird zweckmäßig in einem inerten Lösungsmittel durchgeführt. Geeignete Lösungsmittel sind Alkohole, z. B. Methanol, Äthanol, Äther, z. B. Dioxan, Tetrahydrofuran, Dimethylformamid u. dgl., in Kombination mit Wasser. Vorzugsweise wird die Hydrolyse bei einer Temperatur zwischen Raumtemperatur und der Rückflußtemperatur des Reaktionsgemisches durchgeführt.

Die Umesterung gemäß Verfahrensvariante f) erfolgt ebenfalls in an sich bekannter Weise durch Umsetzen mit einem Alkohol durch Erwärmen auf eine Temperatur zwischen etwa 25° und 150°C. Vorzugsweise wird die Umesterung in Gegenwart einer Säure wie Chlorwasserstoffsäure, Schwefelsäure u. dgl. durchgeführt. Die Umesterung wird in einem inerten organischen Lösungsmittel wie einem Kohlenwassserstoff, z. B. Benzol oder Toluol, einem Äther, z. B. Dioxan oder Tetrahydrofuran, Dimethylformamid u. dgl. durchgeführt. Ist der eingesetzte Alkohol bei der Reaktionstemperatur flüssig, so kann auch überschüssiger Alkohol als Reaktionsmedium dienen.

Die Ammonolyse bzw. Aminolyse gemäß Verfahrensvariante g) wird nach bekannten Methoden in Gegenwart oder Abwesenheit eines Lösungsmittels durchgeführt. Geeignete Lösungsmittel sind Kohlenwasserstoffe wie Hexan, Benzol, Toluol, Äther wie Tetrahydrofuran, Alkohole wie Methanol, Äthanol, Dimethylformamid u. dgl. Diese Reaktion wird bevorzugt bei einer Temperatur zwischen etwa 20° und 150°C, bevorzugt zwischen etwa 50 und 100°C, bei Atmosphärendruck oder einem Druck oberhalb Atmosphärendruck durchgeführt.

Die Oxydation gemäß Verfahrensvariante h) wird mit Kaliumpersulfat in konz. Schwefelsäure bei einer Temperatur zwischen etwa −10° und 50°C, vorzugsweise bei Raumtemperatur, durchgeführt. Die Reaktion ist stark exotherm, weshalb nötigenfalls gekühlt werden muß, damit die Reaktionstemperatur die obere Grenze von 50°C nicht überschreitet.

Die Ketalspaltung gemäß Verfahrensvariante i) wird nach bekannten Methoden durch Behandlung mit einer Säure durchgeführt. Für diesen Zweck geeignete Säuren sind Schwefelsäure, Halogenwasserstoffsäuren, wie die Chlorwasserstoffsäure, eine organische Säure, wie die Ameisensäure u. dgl. Die Reaktion wird zweckmäßig in einem Lösungsmittel durchgeführt. Geeignete Lösungsmittel sind Alkohole, z. B. Methanol oder Äthanol, Äther, z. B. Dioxan, Tetrahydrofuran, Wasser u. dgl. oder Gemische der genannten Lösungsmittel mit Wasser. Die Ketalspaltung wird bei einer Temperatur zwischen etwa 0° und 50°C, vorzugsweise bei Raumtemperatur durchgeführt.

Die Verbindungen der obigen Formel I können in pharmazeutisch anwendbare Säureadditionssalze übergeführt werden, beispielsweise durch Behandlung mit einer anorganischen Säure, wie einer Halogenwasserstoffsäure, z. B. Chlorwasserstoff- oder Bromwasserstoffsäure, Schwefelsäure, Phosphorsäure u. dgl., oder mit einer organischen Säure wie Oxalsäure, Weinsäure, Zitronensäure, Methansulfonsäure u. dgl. Ein pharmazeutisch nicht anwendbares Säureadditionssalz einer Verbindung der Formel I kann in bekannter Weise, z. B. durch Behandlung mit Alkali, in die freie Base übergeführt werden und diese, erwünschtenfalls, in ein pharmazeutisch anwendbares Säureadditionssalz umgewandelt werden.

Die als Ausgangsmaterial verwendeten Verbindungen der obigen Formeln II und III sind zum Teil bekannt und zum Teil neu. Die neuen Verbindungen können in an sich bekannter Weise, d. h. in zur Herstellung der bekannten Verbindungen analoger Weise hergestellt werden.

Die als Ausgangsmaterial verwendeten Verbindungen der obigen Formel IV sind neu und ebenfalls Gegenstand der vorliegenden Erfindung. Sie können beispielsweise dadurch hergestellt werden, daß man ein O-Benzylhydroxylamin der Formel

$$H_2N-OR^{4'''} \qquad \text{VIII}$$

worin $R^{4'''}$ Benzyl bedeutet,
mit einer Verbindung der allgemeinen Formel

$$X-CH_2-CH_2-Z \qquad \text{IX}$$

worin Z eine Phthalimido- oder Succinimidogruppe bedeutet und X die obige Bedeutung besitzt, oder mit Äthylenimin umsetzt.

Die Umsetzung mit einer Verbindung der obigen Formel IX erfolgt nach an sich bekannten Methoden in Gegenwart oder Abwesenheit eines Lösungsmittels bei einer Temperatur zwischen etwa 50° und 100°C, vorzugsweise zwischen etwa 80° und 90°C und liefert eine Verbindung der allgemeinen Formel

$$Z-CH_2CH_2-HN-O-R^{4'''} \qquad \text{Xa}$$

worin $R^{4'''}$ und Z die obige Bedeutung besitzen.

Für diese Umsetzung geeignete Lösungsmittel sind Acetonitril, Tetrahydrofuran u. dgl.

Eine erhaltene Verbindung der Formel Xa kann erwünschtenfalls durch Abspaltung der

Benzylgruppe und allfällige O-Substitution in eine entsprechende Verbindung, worin $R^{4'''}$ die Bedeutung von $R^4$ besitzt, jedoch von Benzyl verschieden ist, überführt werden. Die Abspaltung der Benzylgruppe sowie die allfällige O-Substitution kann unter den für die reduktive Abspaltung der Benzylgruppe aus einer Verbindung der Formel Ia — oder in einer bevorzugten Ausführungsform mit Bortribromid oder Bortrichlorid in einem inerten organischen Lösungsmittel wie Methylenchlorid u. dgl. — bzw. unter den für die O-Substitution einer Verbindung der Formel Ic angegebenen Reaktionsbedingungen durchgeführt werden.

Die so erhaltenen Verbindungen der allgemeinen Formel

$$Z-CH_2CH_2-HN-OR^4 \qquad X$$

worin $R^4$ und Z die obige Bedeutung besitzen,
können nach bekannten Methoden in die entsprechenden Verbindungen der Formel IV umgewandelt werden, beispielsweise durch Hydrazinolyse oder Aminolyse. Die Reaktion kann in Gegenwart oder Abwesenheit eines Lösungsmittels bei einer Temperatur zwischen etwa 0° und 40°C, vorzugsweise zwischen etwa Raumtemperatur und 35°C durchgeführt werden.

In einem Alternativverfahren kann die Verbindung der Formel VIII in an sich bekannter Weise auch mit Äthylenimin zur Verbindung der Formel IV, worin $R^4$ Benzyl bedeutet, umgesetzt werden. Die Umsetzung erfolgt zweckmäßig in einem unter den Reaktionsbedingungen inerten organischen Lösungsmittel wie einem aromatischen Kohlenwasserstoff, z. B. Benzol, Toluol u. dgl. in Gegenwart einer schwachen aprotischen Lewis-Säure wie Bortrifluorid oder einer Säure mit einem schwach nucleophilen Anion wie Tetrafluorborsäure, Perchlorsäure u. dgl. bei einer Temperatur zwischen etwa −20° und 50°C, vorzugsweise zwischen 0°C und Raumtemperatur. Eine erhaltene Verbindung der Formel IV, worin $R^4$ Benzyl bedeutet, kann dann erwünschtenfalls — nach vorheriger Überführung in die entsprechende Verbindung der Formel Xa — wie oben beschrieben in die übrigen Verbindungen der Formel IV, worin $R^4$ von Benzyl verschieden ist, umgewandelt werden.

Die als Ausgangsmaterial verwendeten Verbindungen der obigen Formel V sind neu und ebenfalls Gegenstand der vorliegenden Erfindung. Sie können beispielsweise dadurch hergestellt werden, daß man ein Isothiocyanat der allgemeinen Formel

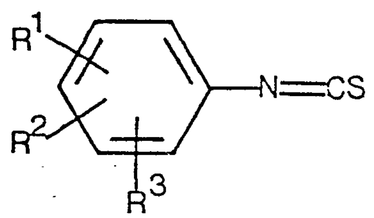

XI

worin $R^1$, $R^2$ und $R^3$ die obige Bedeutung besitzen, mit einer Verbindung der allgemeinen Formel

$$R^{4'}O-NH-CH_2CH_2-Z \qquad XII$$

worin $R^{4'}$ und Z die obige Bedeutung besitzen, zu einer Verbindung der allgemeinen Formel

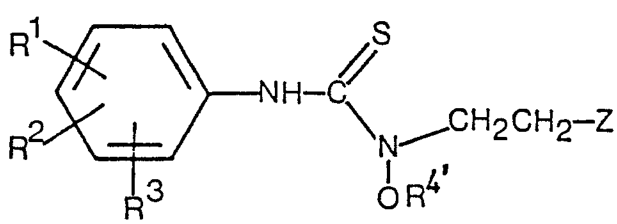

XIII

worin $R^1$, $R^2$, $R^3$, $R^{4'}$ und Z die obige Bedeutung besitzen,
umsetzt. Die Umsetzung erfolgt nach an sich bekannten Methoden in einem unter den Reaktionsbedingungen inerten organischen Lösungsmittel, wie aromatische Kohlenwasserstoffe, z. B. Benzol oder Toluol, Äther, z. B. Tetrahydrofuran, Alkanole, z. B. Äthanol, chlorierte Kohlenwasserstoffe, z. B. Methylenchlorid, u. dgl. Die Umsetzung erfolgt bei einer Temperatur zwischen etwa Raumtemperatur und der Rückflußtemperatur des Reaktionsgemisches.

Die Verbindungen der Formel XIII können nach ebenfalls bekannten Methoden in Verbindungen der allgemeinen Formel

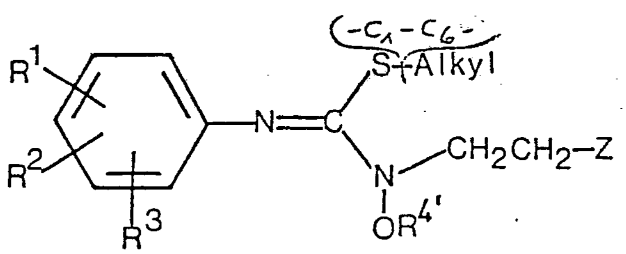

XIV

worin $R^1$, $R^2$, $R^3$, $R^{4'}$ und Z die obige Bedeutung besitzen, überführt werden, beispielsweise durch Umsetzen einer Verbindung der Formel XIII mit einem Trialkyloxoniumtetrafluoroborat, wie Triäthyloxoniumtetrafluoroborat, in einem organischen Lösungsmittel, wie einem chlorierten Kohlenwasserstoff, z. B. Methylenchlorid, einem aromatischen Kohlenwasserstoff, z. B. Benzol oder Toluol, u. dgl. bei einer Temperatur zwischen etwa 0° und 40° C, vorzugsweise bei der Raumtemperatur.

Die Verbindungen der Formeln XIII und XIV können unter den für die Überführung einer Verbindung der Formel X in eine Verbindung der Formel IV, worin $R^4$ Benzyl bedeutet, angegebenen Bedingungen in die entsprechenden Verbindungen der Formel V umgewandelt werden, wobei manchmal sofort Ringschluß eintritt.

Die Verbindungen der Formeln VI, VII, IX und XI sind bekannt oder können in zur Herstellung der bekannten Verbindungen analoger Weise hergestellt werden.

Die Verbindungen der Formel XII sind neu und ebenfalls Gegenstand der vorliegenden Erfindung. Sie können beispielsweise aus den Verbindungen der Formel IV, worin $R^4$ von Wasserstoff verschieden ist, hergestellt werden, indem man diese in an sich bekannter Weise in einem inerten organischen Lösungsmittel, wie einem aromatischen Kohlenwasserstoff, z. B. Toluol oder Xylol, Acetonitril, Dimethylformamid u. dgl. bei einer Temperatur zwischen etwa 80° und 150° C, vorzugsweise bei der Rückflußtemperatur des Reaktionsgemisches, mit Phthalsäureanhydrid bzw. Bernsteinsäureanhydrid umsetzt. Diese Umsetzung kann durch eine Base wie Triäthylamin u. dgl. katalysiert werden. Die Verbindungen der Formel XII, worin $R^{4'}$ Wasserstoff bedeutet, können aus den entsprechenden Verbindungen der Formel XII, worin $R^4$ Benzyl bedeutet, durch Abspaltung der Benzylgruppe hergestellt werden. Die Abspaltung der Benzylgruppe kann unter den für die reduktive Abspaltung der Benzylgruppe aus einer Verbindung der Formel Ia angegebenen Reaktionsbedingungen durchgeführt werden, vorzugsweise jedoch mit Bortribromid oder Bortrichlorid in einem inerten organischen Lösungsmittel wie Methylenchlorid u. dgl.

Die Verbindungen der Formel I sowie deren pharmazeutisch anwendbare Säureadditionssalze besitzen eine wertvolle cardiovaskuläre Wirksamkeit, insbesondere eine wertvolle blutdrucksenkende Wirkung, mit oder ohne zentrale sympathico-inhibitorische Eigenschaften, und können demnach zur Behandlung von Hypertonien Anwendung finden.

Die blutdrucksenkende Wirkung kann nach den folgenden zwei Methoden bestimmt werden:

A) Der systolische Blutdruck und die Herzfrequenz werden vor Substanzgabe mehrmals an wachen, spontan hypertonen weiblichen Ratten gemessen. Pro Dosis werden 5 Versuchstiere mit einem Körpergewicht von ca. 300 g eingesetzt. Die Substanzgabe erfolgt mittels Magensonde. Beide Parameter werden 1, 3, 6 und 16 Stunden nach der Applikation gemessen und die prozentuale Veränderung zu den Kontrollwerten berechnet. Der systolische Blutdruck wird indirekt an der Schwanzarterie der Ratte nach der Methode von Gerold et al. gemessen (Helv. Physiol. Acta 24, 58—69, 1966; Arzneimittelforschung 18, 1285—1287, 1968).

B) Der systolische Blutdruck und die Herzfrequenz werden vor Substanzgabe an wachen weiblichen Hunden mit einem Körpergewicht von 10—15 kg gemessen. Benutzt wird eine modifizierte Carotisschlingen-Technik nach van Leersum (Pflügers's Arch. ges. Physiol. 142, 377—381, 1911). Die Substanz wird Tieren verabreicht, die über Nacht nicht gefüttert worden sind. Beide Parameter werden 0,5, 1, 1,5, 2, 3, 4, 6 und 16 Stunden nach der Applikation gemessen und die prozentuale Veränderung zu den Kontrollwerten berechnet. Die gleichzeitig durchgeführte Beobachtung der Versuchstiere erstreckt sich 24 Stunden über den Zeitpunkt hinaus, an dem die beiden Parameter die Kontrollwerte erreichen.

Die zentrale sympathico-inhibitorische Wirkung kann nach der folgenden Methode bestimmt werden:

Die Wirkung der Testverbindungen auf die Aktivität im sympathischen Nervensystem wird an Katzen in Urethannarkose untersucht. Die präganglionäre Sympathicusaktivität wird mittels bipolaren Platinelektroden vom Nervus spalnchnicus, die postganglionäre von einem Nervenast zur Niere nach der Methode von G. Häusler abgeleitet (Naunyn-Schmiedeberg's Arch. Pharmacol. 286, 97—111, 1974). Außerdem wird der arterielle Blutdruck aus der arteria femoralis sowie die Herzfrequenz gemessen. Die Testsubstanz wird i. v. injiziert. Wenn eine Testsubstanz in blutdrucksenkenden Dosen die Sympathicusaktivität während mehr als 30 Minuten um mehr als 30% hemmt, so wird sie als »mit

zentraler symphatico-inhibitorischer Wirkung« qualifiziert.

In der nachfolgenden Tabelle sind die erhaltenen Resultate zusammengestellt, wobei jeweils die maximalen prozentualen Abweichungen von den Kontrollwerten bzw., ob die Verbindungen eine zentrale symphatico-inhibitorische Wirkung besitzen oder nicht, angegeben sind.

| Verbindung | Spontan hypertone Ratte | | | Wacher Hund | | | Zentrale sympathico-inhibitorische Wirkung |
|---|---|---|---|---|---|---|---|
| | Dosis mg/kg p.o. | Blutdruck △ % | Herzfrequenz △ % | Dosis mg/kg p.o. | Blutdruck △ % | Herzfrequenz △ % | |
| A | 3 | −14 | −15 | 3 | −19 | −41 | Nein |
| A | 10 | −26 | −24 | | | | |
| B | 3 | −14 | − 5 | 10 | −17 | −47 | Nein |
| C | 3 | −21 | −20 | 10 | −16 | − 6 | Nein |
| D | 3 | −17 | + 9 | 30 | −20 | −36 | Nein |
| E | 10 (i.p.) | −10 | −17 | 10 | −20 | − 7 | Nein |
| F | 3 | −15 | + 5 | 3 | −18 | −23 | Nein |
| G | 30 | −15 | −54 | 1 | −24 | − 9 | Ja |
| H | 3 | −14 | −11 | 10 | −16 | −47 | Ja |
| I | 30 | −19 | −21 | 3 | −20 | −13 | Ja |
| K | 10 | −15 | −19 | 3 | −14 | −37 | Ja |
| L | 3 | −13 | − 7 | 1 | −20 | − 8 | Ja |
| M | 1 | −13 | −19 | 0,1 | −21 | −13 | Ja |
| | | | | 1 | −32 | − 3 | |

A 2-[(2,6-Dichlorphenyl)imino]-1-hydroxyimidazolidin-hydrobromid
B 2-[(2,6-Dibromphenyl)imino]-1-hydroxyimidazolidin-hydrobromid
C 1-Hydroxy-2-[(2-iodphenyl)imino]imidazolidin-hydrobromid
D Äthyl 4[/2-[(2,6-dichlorphenyl)imino]-1-imidazolidin/oxy]butyrat-hydrochlorid
E 4-[/2-[(2,6-Dichlorphenyl)imino]-1-imidazolidin/oxy]buttersäure
F Äthyl 2-[/2-[(2,6-dichlorphenyl)imino]-1-imidazolidin/oxy]butyrat-hydrochlorid
G 2-[(2,6-Dichlorphenyl)imino]-1-(2-propinyloxy)-imidazolidin-hydrochlorid
H [/2-[(2,6-Dichlorphenyl)imino]-1-imidazolidin/oxy]essigsäure
I Äthyl 4-[/2-[(2,6-dichlorphenyl)imino]-1-imidazolidin/oxy]crotonat-hydrochlorid
K 2-[(2,6-Dichlorphenyl)imino]-1-äthoxyimidazolidin-hydrochlorid
L 1-(2-Butinyloxy)-2-[(2,6-dichlorphenyl)imino]imidazolidin-hydrochlorid
M 2-[(2,6-Dichlorphenyl)imino]-1-(furfuryloxy)-imidazolidin-hydrochlorid

Die Verfahrensprodukte und deren pharmazeutisch verwendbaren Salze können als Heilmittel z. B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z. B. Wasser, Gelatine, Gummi arabicum, Milchzucker, Stärke, Magnesiumstearat, Talk, pflanzliche Öle, Polyalkylenglykole, Vaseline, usw. enthalten. Die pharmazeutischen Präparate können in fester Form, z. B. als Tabletten, Dragées, Suppositorien, Kapseln oder in flüssiger Form, z. B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten.

Die tägliche Dosis bei oraler Verabreichung liegt zwischen etwa 1 und 200 mg; bei i.v.-Verabreichung zwischen etwa 0,1 und 20 mg. Die angegebenen Dosierungen sind jedoch nur als Beispiele zu verstehen und können je nach der Schwere des zu behandelnden Falles und nach Ermessen des behandelnden Arztes abgeändert werden.

Die nachfolgenden Beispiele erläutern die Erfindung. Alle Temperaturen sind in Celsiusgraden angegeben. Die Schmelzpunkte sind nicht korrigiert.

## Beispiel 1

Zu einer Lösung von 24,93 g (150 mMol) N-(Benzyloxy)-äthylen-diamin, 75 ml N-Äthyl-N,N-diisopropylamin und 300 ml abs. Essigester wird unter Rühren eine Lösung von 36,44 g (150 mMol) 2,6-Dichlorphenylimidocarbonylchlorid so zugetropft, daß die Temperatur 30° nicht übersteigt. Nach beendeter Zugabe wird über Nacht bei Raumtemperatur weitergerührt. Das Gemisch wird fünfmal mit Wasser extrahiert, und die Essigesterphase wird über Natriumsulfat getrocknet. Nach dem Eindampfen des Lösungsmittels wird der Rückstand aus Isopropyläther umkristallisiert, wobei man 1-Benzyloxy-2-[(2,6-dichlorphenyl)imino]-imidazolidin erhält. Smp. 115–117°.

Das Ausgangsmaterial kann wie folgt hergestellt werden:

A) Ein Gemisch aus 12,30 g (48,4 mMol) 2-Bromäthylphthalimid und 12,31 g (100 mMol) O-Benzylhydroxylamin wird während 48 Stunden auf 80° erwärmt. Danach wird Essigester zugegeben, und der gebildete Niederschlag abgenutscht. Die Mutterlauge wird im Vakuum eingedampft. Der ölige Rückstand wird mit 35 ml Methanol versetzt und über Nacht bei Raumtemperatur stehengelassen. Der kristalline Niederschlag wird abgenutscht und getrocknet. Das erhaltene Rohprodukt schmilzt bei 90–91°. Durch Umkristallisation aus Methanol erhält man analysenreines N-/2-[(Benzyloxy)amino]äthyl/phthalimid, Smp. 92–94°.

B) Ein Gemisch aus 50,0 g (197 mMol) 2-Bromäthylphthalimid, 100 g (812 mMol) O-Benzylhydroxylamin und 500 ml Acetonitril wird während 24 Stunden zum Rückfluß erhitzt. Danach werden nochmals 100 g (812 mMol) O-Benzylhydroxylamin dazu gegeben und wiederum 24 Stunden zum Rückfluß erhitzt. Das Lösungsmittel wird danach im Vakuum abgedampft und das überschüssige O-Benzylhydroxylamin im Hochvakuum (0,02 mm Hg) abdestilliert. Der Rückstand wird aus Methanol kristallisiert, wobei man N-/2-[(Benzyloxy)amino]äthyl/phthalimid erhält, Smp. 89–91°. Umkristallisieren aus Methanol erhöht den Schmelzpunkt auf 92–94°.

C) 26,96 g (91 mMol) N-/2-[(Benzyloxy)amino]äthyl/phthalimid werden in 300 ml abs. Alkohol suspendiert und mit 40,5 ml Hydrazinhydrat versetzt. Nach 3stündigem Rühren bei Raumtemperatur wird der gebildete Niederschlag abfiltriert und die Mutterlauge vollständig eingedampft. Der Rückstand und der Niederschlag werden vereinigt und mit 600 ml 1 N Salzsäure versetzt, und das Gemisch während 3 Stunden stark gerührt. Der gebildete Niederschlag wird abfiltriert und getrocknet. Die Mutterlauge wird mit 1 N Natronlauge auf pH 9 eingestellt, mit Natriumchlorid gesättigt und mehrere Male mit Diäthyläther extrahiert. Nach dem Trocknen über Natriumsulfat wird das Lösungsmittel im Vakuum eingedampft. Der ölige Rückstand wird im Hochvakuum destilliert, wobei man N-(Benzyloxy)äthylendiamin erhält, Sdp. 110–115°/0,02 mm Hg. Das

entsprechende Hydrochlorid schmilzt bei 157—159° C (aus Methanol/Acetonitril).

D) 29,6 g (100 mMol) N-/2-[(Benzyloxy)amino]äthyl/phthalimid werden mit 200 ml 40%igem Methylamin versetzt und während 48 Stunden bei Raumtemperatur gerührt. Die erhaltene Lösung wird dreimal mit Methylenchlorid extrahiert. Die organischen Extrakte werden im Vakuum eingedampft und der Rückstand in Äther aufgenommen. Die ätherische Lösung wird mit 1 M Weinsäure extrahiert. Die sauren Extrakte werden mit Eis versetzt und dann mit Natronlauge auf pH 11 eingestellt. Nach fünfmaliger Extraktion mit Äther werden die organischen Extrakte über Natriumsulfat getrocknet und im Vakuum zur Trockene eingedampft, wobei man N-(Benzyloxy)-äthylendiamin erhält.

E) 2,15 g (50 mMol) Aziridin werden in 30 ml Methylenchlorid gelöst und bei 5° mit 5,8 ml einer 9,47 N Lösung (55 mMol) von Tetrafluorborsäure in Äther versetzt. Zu diesem Gemisch gibt man 6,18 g O-Benzylhydroxylamin in 40 ml Methylenchlorid zu. Nach 24 Stunden wird die Lösung auf eine 5%ige Natriumcarbonatlösung gegossen. Die organische Phase wird mit einer gesättigten Natriumchloridlösung gewaschen, getrocknet und im Vakuum eingedampft. Der Rückstand wird durch Destillation im Hochvakuum gereinigt, wobei man N-(Benzyloxy)äthylendiamin erhält.

F) 500 g (3,0 Mol) 2,6-Dichloranilin werden in 1000 ml Ameisensäure gelöst und während 4 Stunden zum Rückfluß erhitzt. Nach dem Abkühlen auf 5° werden die gebildeten Kristalle abfiltriert und mit ca. 1000 ml eiskaltem Isopropanol gewaschen. Man erhält 2,6-Dichlorformanilid, Smp. 180—182°. Aus der Mutterlauge kann noch weiteres Material vom gleichen Schmelzpunkt erhalten werden.

G) Zu einem Gemisch aus 910 ml Thionylchlorid und 220 ml Sulfonylchlorid (2,72 Mol) werden innerhalb von 45 Minuten 485 g (2,55 Mol) 2,6-Dichloranilid bei einer Temperatur von etwa 10° zugegeben. Die Lösung wird dann während 9 Stunden zum Rückfluß erhitzt und dann über Nacht bei Raumtemperatur stehengelassen. Nach dem Eindampfen des Lösungsmittels unter reduziertem Druck wird der ölige Rückstand im Vakuum destilliert, wobei man (2,6-Dichlorphenyl)imidocarbonylchlorid erhält, Sdp. 135—138°/13,5 mm Hg.

## Beispiel 2

Eine Suspension von 45,3 g (134,7 mMol) 1-Benzyloxy-2-[(2,6-dichlorphenyl)imino]-imidazolidin in 450 ml 48%iger Bromwasserstoffsäure wird 4 Stunden auf 80° erwärmt. Die erhaltene Lösung wird auf Eis gegossen und mit Äther extrahiert. Die wässerige Phase wird im Vakuum eingedampft. Der Rückstand wird mit einem Gemisch aus Äthanol/Essigester fünfmal azeotrop destilliert. Durch Kristallisation aus einem Gemisch aus Äthanol/Essigester/Äther erhält man 2-[(2,6-Dichlorphenyl)imino]-1-hydroxyimidazolidin-hydrobromid. Eine analysenreine Probe wird durch Umkristallisation aus Methanol/Acetonitril erhalten, Smp. 233—234°.

## Beispiel 3

0,6 g (1,8 mMol) 2-[(2,6-Dichlorphenyl)imino]-1-hydroxyimidazolidin-hydrobromid werden in wenig Wasser gelöst und mit 1 N Natronlauge auf pH 10 eingestellt. Der gebildete Niederschlag wird mit Methylenchlorid extrahiert. Die organischen Extrakte werden über Natriumsulfat getrocknet und zur Trockene eingedampft. Der Rückstand wird aus Acetonitril umkristallisiert, wobei man 2-[(2,6-Dichlorphenyl)imino]-1-hydroxyimidazolidin erhält, Smp. 215° (Zers.).

## Beispiel 4

12,5 g (150 mMol) O-Methylhydroxylamin-hydrochlorid werden in 50 ml Acetonitril suspendiert. Bei 25° werden 12,92 g (300 mMol) Aziridin zugetropft. Nach 1 Stunde gibt man 75 ml N-Äthyl-N,N-diisopropylamin zu und kühlt das Gemisch auf 10°. Unter weiterer Kühlung tropft man 36 g (148 mMol) 2,6-(Dichlorphenyl)imidocarbonylchlorid zu, wobei die Temperatur 15° nicht überschreiten soll. Das Gemisch wird danach während 4 Stunden bei Raumtemperatur weitergerührt. Dann gießt man auf Wasser und extrahiert viermal mit Äther. Die organischen Extrakte werden über Natriumsulfat getrocknet und im Vakuum eingedampft. Der Rückstand wird an 400 g Kieselgel chromatographiert (Eluierungsmittel Chloroform/Äther 9 : 1), wobei man 2-[(2,6-Dichlorphenyl)imino]-1-methoxyimidazolidin erhält, welches nach Umkristallisation aus Isopropyläther bei 123—124° schmilzt.

Die Base wird in Acetonitril gelöst und mit Chlorwasserstoffsäure in Dioxan auf pH 1 eingestellt. Das erhaltene Hydrochlorid schmilzt bei 228—229°.

Beispiel 5

370 mg (1 mMol) 1-(2-Aminoäthyl)-1-benzyloxy-3-(2,6-dichlorphenyl)-2-thioharnstoff werden auf 120° erhitzt. Nach Abkühlen auf Raumtemperatur wird der Rückstand aus Isopropyläther kristallisiert, wobei man 1-Benzyloxy-2-[(2,6-dichlorphenyl)imino]-imidazolidin erhält, Smp. 115–117°.

Das Ausgangsmaterial kann wie folgt hergestellt werden:

6,12 g (30 mMol) 2,6-Dichlorphenylisothiocyanat werden in 150 ml abs. Benzol gelöst und mit 8,89 g (30 mMol) N-/2-[(Benzyloxy)amino]äthyl/phthalimid versetzt. Nach 24 Stunden bei Raumtemperatur wird der kristalline Niederschlag abfiltriert, wobei man 1-Benzyloxy-3-(2,6-dichlorphenyl)-1-(2-phthalimidoäthyl)-2-thioharnstoff erhält, Smp. 180–182°.

4 g (8 mMol) 1-Benzyloxy-3-(2,6-dichlorphenyl)-1-(2-phthalimidoäthyl)-2-thioharnstoff werden in 30 ml Äthanol und 4 ml Hydrazinhydrat suspendiert. Nach ungefähr 1 Stunde entsteht eine klare Lösung. Es wird noch 3 Stunden weitergerührt, wobei sich ein Niederschlag bildet, der abfiltriert wird. Die Mutterlauge wird im Vakuum eingedampft und der Rückstand sowie der abfiltrierte Niederschlag in 60 ml 1 N Salzsäure suspendiert. Nach 16stündigem Rühren bei Raumtemperatur wird der Niederschlag abfiltriert, und die saure Lösung mit 60 ml 1 N Natronlauge neutralisiert. Der dabei entstehende Niederschlag wird abfiltriert, getrocknet und aus Methanol/Isopropyläther umkristallisiert, wobei man 1-(2-Aminoäthyl)-1-benzyloxy-3-(2,6-dichlorphenyl)-2-thioharnstoff erhält, Smp. 108–109°.

2,0 g Base werden in 10 ml Essigester gelöst und mit Chlorwasserstoffsäure in Dioxan auf pH 3 eingestellt. Die ölige Suspension wird im Vakuum eingedampft und der Rückstand aus Acetonitril umkristallisiert. Das erhaltene Hydrochlorid schmilzt bei 170–171°.

Beispiel 6

200 mg (0,5 mMol) 3-(2-Aminoäthyl)-3-benzyloxy-1-(2,6-dichlorphenyl)-2-äthyl-2-isothioharnstoff werden mit 20 ml Methanol und 250 mg Ammoniumacetat bei Raumtemperatur gerührt. Das Reaktionsgemisch wird danach im Vakuum zur Trockene eingedampft. Der Rückstand wird in Essigester gelöst, und die organische Lösung wird mit Wasser gewaschen, über Natriumsulfat getrocknet und im Vakuum eingedampft. Der Rückstand wird aus Isopropyläther umkristallisiert, wobei man 1-Benzyloxy-2-[(2,6-dichlorphenyl)imino]-imidazolidin erhält, Smp. 117–118°.

Das Ausgangsmaterial kann wie folgt hergestellt werden:

10,0 g (20 mMol) 1-Benzyloxy-3-(2,6-dichlorphenyl)-1-(2-phthalimidoäthyl)-2-thioharnstoff werden in 100 ml Methylenchlorid gelöst und mit 4,18 g (22 mMol) Triäthyloxoniumtetrafluoroborat versetzt. Nach 3stündigem Rühren bei Raumtemperatur wird die Lösung auf eine 5%ige Natriumcarbonatlösung gegossen. Die organische Phase wird mit Wasser gewaschen, über Natriumsulfat getrocknet und im Vakuum eingedampft. Der Rückstand wird in 200 ml heißem Isopropyläther gelöst, und die Lösung wird auf 100 ml eingeengt. Man erhält 3-Benzyloxy-1-(2,6-dichlorphenyl)-2-äthyl-3-(2-phthalimidoäthyl)-2-isothioharnstoff vom Schmelzpunkt 102° (Zers.).

1,06 g (2 mMol) 3-Benzyloxy-1-(2,6-dichlorphenyl)-2-äthyl-3-(2-phthalimidoäthyl)-2-isothioharnstoff werden mit 6 ml 40%igem Methylamin über Nacht bei Raumtemperatur gerührt. Die ölige Suspension wird mit Äther extrahiert. Die organischen Extrakte werden mit Wasser gewaschen, über Natriumsulfat getrocknet und im Vakuum eingedampft. Der Rückstand wird in Äther gelöst und mit Chlorwasserstoffsäure in Dioxan versetzt. Der ausgefallene Niederschlag wird abfiltriert und aus Acetonitril umkristallisiert, wobei man 3-(2-Aminoäthyl)-3-benzyloxy-1-(2,6-dichlorphenyl)-2-äthyl-2-isothioharnstoff-dihydrochlorid erhält, Smp. 148° (Zers.).

Beispiel 7

0,34 g (1 mMol) 1-Benzyloxy-2-[(2,6-dichlorphenyl)imino]imidazolidin werden in 5 ml Äthanol und 2 ml 1 N Salzsäure in Gegenwart von 0,05 g Palladium/Kohle (5%) unter Normaldruck hydriert. Nach beendeter Wasserstoffaufnahme wird der Katalysator abfiltriert, und die Lösung eingedampft. Der Rückstand wird in Wasser gelöst, die Lösung mit Natronlauge auf pH 10 eingestellt und erst mit Methylenchlorid und dann mit Essigester extrahiert. Die organischen Extrakte werden über Natriumsulfat getrocknet und im Vakuum zur Trockene eingedampft. Der Rückstand wird aus Acetonitril umkristallisiert, wobei man 2-[(2,6-Dichlorphenyl)imino]-1-hydroxyimidazolidin erhält, Smp. 196°. Umkristallisation aus Methanol/Acetonitril erhöht den Schmelzpunkt auf 215°.

Beispiel 8

1,0 g (3 mMol) 1-Benzyloxy-2-[(2,6-dichlorphenyl)imino]imidazolidin wird in 10 ml Methylenchlorid gelöst und bei Raumtemperatur vorsichtig mit Bortribromid (1 ml) versetzt. Nach 1 Stunde wird die

Lösung im Vakuum eingedampft. Der Rückstand wird in Wasser gelöst, die Lösung mit Natronlauge auf pH 10 eingestellt und erst mit Methylenchlorid und dann mit Essigester extrahiert. Die organischen Extrakte werden über Natriumsulfat getrocknet und im Vakuum eingedampft. Der Rückstand wird aus Acetonitril umkristallisiert, wobei man 2-[(2,6-Dichlorphenyl)imino]-1-hydroxyimidazolidin erhält, Smp. 211° (Zers.).

## Beispiel 9

8,4 g (25 mMol) 1-Benzyloxy-2-[(2,6-dichlorphenyl)imino]imidazolidin in 30 ml Eisessig und 2,5 ml konz. Schwefelsäure werden in Gegenwart von 300 mg Platinoxid unter Normaldruck hydriert. Nach beendeter Wasserstoffaufnahme wird der Katalysator abfiltriert, und das Filtrat eingedampft. Der Rückstand wird in Wasser gelöst, und die Lösung mit Natriumcarbonat alkalisch gestellt. Der entstandene Niederschlag wird abfiltriert, mit etwas Wasser gewaschen und im Vakuum getrocknet, wobei man 2-[(2,6-Dichlorphenyl)imino]-1-hydroxyimidazolidin erhält, Smp. 215—216°.

## Beispiel 10

31,0 g (63,0 mMol) 1-Benzyloxy-3-(3,4-dimethoxyphenyl)-1-(2-phthalimidoäthyl)-2-thioharnstoff werden in 600 ml Äthanol suspendiert und mit 34 ml Hydrazinhydrat versetzt. Nach 8—12stündigem Rühren bei Raumtemperatur wird das Gemisch mit konz. Salzsäure angesäuert und über Nacht bei Raumtemperatur gerührt. Der Niederschlag wird abfiltriert. Die wässerige Lösung wird mit Natriumcarbonat alkalisch gestellt und mit Essigester extrahiert. Der organische Extrakt wird getrocknet und im Vakuum eingedampft. Der Rückstand wird an 450 g Kieselgel chromatographiert (Eluierungsmittel Chloroform/Essigester 3 : 2). Das erhaltene Öl wird in Äther gelöst und mit einem Überschuß an L-Weinsäure in Äther versetzt, wobei man 1-Benzyloxy-2-[(3,4-dimethoxyphenyl)imino]imidazolidin-L-tartrat erhält, Smp. 90—95°.

Das Ausgangsmaterial kann wie folgt hergestellt werden:

Eine Lösung von 12,44 g (42 mMol) N-[2-(Benzyloxy)aminoäthyl]phthalimid in 50 ml Benzol wird bei Raumtemperatur mit 8,20 g (42 mMol) 3,4-Dimethoxyphenylisothiocyanat versetzt. Nach 36 Stunden wird der entstandene Niederschlag abfiltriert, wobei man 1-Benzyloxy-3-(3,4-dimethoxyphenyl)-1-(2-phthalimidoäthyl)-2-thioharnstoff erhält, Smp. 120—122°.

## Beispiel 11

143,5 g (0,6 Mol) N-(Benzyloxy)äthylendiamin-dihydrochlorid werden mit 400 ml Wasser versetzt. Zu dieser Suspension werden nach und nach vorsichtig 207,0 g (1,5 Mol) Kaliumcarbonat gegeben. Danach werden unter starkem Rühren 200 ml Toluol und dann langsam 145,7 g (0,6 Mol) 2,6-Dichlorphenylimidocarbonylchlorid in 100 ml Toluol innert 45 Minuten zugetropft. Nach 15 Minuten beginnt sich ein Produkt abzuscheiden. Das Gemisch wird über Nacht weitergerührt und danach auf 1 Liter n-Hexan gegossen. Der Niederschlag wird abfiltriert und mit Wasser und mit n-Hexan gewaschen, wobei man 1-Benzyloxy-2-[(2,6-dichlorphenyl)imino]imidazolidin erhält, Smp. 114—117°.

## Beispiel 12

In analoger Weise wie in Beispiel 1 beschrieben wurden die folgenden Verbindungen hergestellt:

- Aus 2,6-Dimethylphenylimidocarbonylchlorid und N-(Benzyloxy)äthylendiamin erhält man 1-Benzyloxy-2-[(2,6-dimethylphenyl)imino]imidazolidin-hydrobromid vom Schmelzpunkt 188—189° (aus Acetonitril/Diäthyläther);
- aus Phenylimidocarbonylchlorid und N-(Benzyloxy)-äthylendiamin erhält man 1-Benzyloxy-2-(phenylimino)-imidazolidin vom Schmelzpunkt 78—79° (aus Isopropyläther);
- aus 2-Isopropylphenylimidocarbonylchlorid und N-(Benzyloxy)-äthylendiamin erhält man 1-Benzyloxy-2-[(2-isopropylphenyl)-imino]imidazolidin als ein Öl;
- aus 3,4-Dichlorphenylimidocarbonylchlorid und N-(Benzyloxy)-äthylendiamin erhält man 1-Benzyloxy-2-[(3,4-dichlorphenyl)imino]imidazolidin-hydrochlorid vom Schmelzpunkt 178—180° (aus Methanol/Acetonitril);
- aus 2-Chlor-6-methylphenylimidocarbonylchlorid und N-(Benzyloxy)äthylendiamin erhält man 1-Benzyloxy-2-[(6-chlor-2-methylphenyl)imino]imidazolidin-hydrochlorid vom Schmelzpunkt 188—191° (aus Acetonitril);
- aus 2,6-Dibromphenylimidocarbonylchlorid und N-(Benzyloxy)-äthylendiamin erhält man 1-Benzyloxy-2-[(2,6-dibromphenyl)imino]imidazolidin vom Schmelzpunkt 89—90° (aus Diisopropyl-

äther/Cyclohexan);

- aus 4-Chlor-2,6-diäthylphenylimidocarbonylchlorid und N-(Benzyloxy)äthylendiamin erhält man 1-Benzyloxy-2-[(4-chlor-2,6-diäthylphenyl)imino]imidazolidin als ein Öl;
- aus 2-Iodphenylimidocarbonylchlorid und N-(Benzyloxy)-äthylendiamin erhält man 1-Benzyloxy-2-[(2-iodphenyl)imino]imidazolidin-hydrochlorid vom Schmelzpunkt 192—194° (aus Methanol/Acetonitril);
- aus 2-Chlorphenylimidocarbonylchlorid und N-(Benzyloxy)-äthylendiamin erhält man 1-Benzyloxy-2-[(2-chlorphenyl)imino]imidazolidin-hydrochlorid vom Schmelzpunkt 198—201° (aus Methanol/Acetonitril/Aceton);
- aus 2-Trifluormethylphenylimidocarbonylchlorid und N-(Benzyloxy)äthylendiamin erhält man 1-Benzyloxy-2-[(2-trifluormethylphenyl)imino]imidazolidin-hydrochlorid vom Schmelzpunkt 191—193° (aus Acetonitril);
- aus 2-Bromphenylimidocarbonylchlorid und N-(Benzyloxy)-äthylendiamin erhält man 1-Benzyloxy-2-[(2-bromphenyl)imino]imidazolidin-hydrochlorid vom Schmelzpunkt 199—200° (aus Acetonitril).

Beispiel 13

In analoger Weise wie in Beispiel 11 beschrieben wurden die folgenden Verbindungen hergestellt:

- Aus 2-Trifluormethylphenylimidocarbonylchlorid und N-(Benzyloxy)äthylendiamin-dihydrochlorid erhält man 1-Benzyloxy-2-[(2-trifluormethylphenyl)imino]imidazolidin-hydrochlorid vom Schmelzpunkt 191—193° (aus Acetonitril);
- aus 2-Bromphenylimidocarbonylchlorid und N-(Benzyloxy)äthylendiamin-dihydrochlorid erhält man 1-Benzyloxy-2-[(2-bromphenyl)imino]imidazolidin-hydrochlorid vom Schmelzpunkt 199—200° (aus Acetonitril).

Beispiel 14

In analoger Weise wie in Beispiel 5, Absatz 2 bzw. Beispiel 10, Absatz 3 beschrieben wurden die folgenden Verbindungen hergestellt:

- Aus 3,4-Dimethylphenylisothiocyanat und N-/2-[(Benzyloxy)amino]äthyl/phthalimid erhält man 1-Benzyloxy-3-(3,4-dimethylphenyl)-1-(2-phthalimidoäthyl)-2-thioharnstoff, Smp. 90—91° (aus Diäthyläther);
- aus 3-Trifluormethylphenylisothiocyanat und N-/2-[(Benzyloxy)amino]äthyl/phthalimid erhält man 1-Benzyloxy-1-(2-phthalimidoäthyl)-3-(α,α,α-trifluor-m-tolyl)-2-thioharnstoff, Smp. 123—124° (aus Benzol);
- aus 4-Methylthiophenylisothiocyanat und N-/2-[(Benzyloxy)amino]äthyl/phthalimid erhält man 1-Benzyloxy-3-[4-(methylthio)phenyl]-1-(2-phthalimidoäthyl)-2-thioharnstoff.

Beispiel 15

In analoger Weise wie in Beispiel 10 beschrieben wurden die folgenden Verbindungen hergestellt:

- Aus 1-Benzyloxy-1-(2-phthalimidoäthyl)-3-(α,α,α-trifluor-m-tolyl)-2-thioharnstoff und Hydrazinhydrat erhält man 1-Benzyloxy-2-[(α,α,α-trifluor-m-tolyl)imino]imidazolidin-hydrochlorid, Smp. 154—155° (aus Äthanol/Essigester);
- aus 1-Benzyloxy-3-[4-(methylthio)phenyl]-1-(2-phthalimidoäthyl)-2-thioharnstoff und Hydrazinhydrat erhält man 1-Benzyloxy-2-/[4-(methylthio)phenyl]imino/imidazolidin, Smp. 96—97° (aus Methylenchlorid/Diisopropyläther);
- aus 1-Benzyloxy-3-(3,4-dimethylphenyl)-1-(2-phthalimidoäthyl)-2-thioharnstoff und Hydrazinhydrat erhält man 1-Benzyloxy-2-[(3,4-dimethylphenyl)imino]imidazolidin.

Beispiel 16

In analoger Weise wie in Beispiel 2 beschrieben wurden die folgenden Verbindungen hergestellt:

- Aus 1-Benzyloxy-2-[(2,6-dimethylphenyl)imino]imidazolidin-hydrobromid und Bromwasserstoffsäure erhält man 1-Hydroxy-2-[2,6-dimethylphenyl)imino]imidazolidin-hydrobromid, Smp. 177—179° (aus Aceton);

— aus 1-Benzyloxy-2-(phenylimino)imidazolidin und Bromwasserstoffsäure erhält man 1-Hydroxy-2-(phenylimino)imidazolidin-hydrobromid, Smp. 152—154° (aus Acetonitril);
— aus 1-Benzyloxy-2-[(2-isopropylphenyl)imino]imidazolidin und Bromwasserstoffsäure erhält man 1-Hydroxy-2-[(2isopropylphenyl)imino]imidazolidin-hydrobromid, Smp. 142—144° (aus Aceton);
— aus 1-Benzyloxy-2-[(α,α,α-trifluor-m-tolyl)imino]imidazolidin und Bromwasserstoffsäure erhält man 1-Hydroxy-2-[(α,α,α-trifluor-m-tolyl)imino]imidazolidin-hydrobromid, Smp. 217° unter Zersetzung (aus Acetonitril/Diäthyläther);
— aus 1-Benzyloxy-2-[(3,4-dichlorphenyl)imino]imidazolidin-hydrochlorid und Bromwasserstoffsäure erhält man 2-[(3,4-Dichlorphenyl)imino]-1-hydroxyimidazolidin-hydrobromid, Smp. 204—206° unter Zersetzung (aus Methanol/Acetonitril);
— aus 1-Benzyloxy-2-[(3,4-dimethylphenyl)imino]imidazolidin und Bromwasserstoffsäure erhält man 1-Hydroxy-2-[(3,4-dimethylphenyl)imino]imidazolidin-hydrobromid, Smp. 198—200° (aus Acetonitril/Diäthyläther);
— aus 1-Benzyloxy-2-[(6-chlor-2-methylphenyl)imino]imidazolidin und Bromwasserstoffsäure erhält man 2-[(6-Chlor-2-methylphenyl)imino]-1-hydroxyimidazolidin-hydrobromid, Smp. 206—207° (aus Acetonitril);
— aus 1-Benzyloxy-2-[(2,6-dibromphenyl)imino]imidazolidin und Bromwasserstoffsäure erhält man 2-[(2,6-Dibromphenyl)imino]-1-hydroxyimidazolidin-hydrobromid, Smp. 260° (aus Methanol/Acetonitril);
— aus 1-Benzyloxy-2-[(4-chlor-2,6-diäthylphenyl)imino]imidazolidin und Bromwasserstoffsäure erhält man 2-[(4-Chlor-2,6-diäthylphenyl)imino]-1-hydroxyimidazolidin, Smp. 170—171° (aus Diisopropyläther);
— aus 1-Benzyloxy-2-[(2-iodphenyl)imino]imidazolidin und Bromwasserstoffsäure erhält man 1-Hydroxy-2-[(2-iodphenyl)imino]imidazolidin-hydrobromid, Smp. 189—190° (aus Acetonitril);
— aus 1-Benzyloxy-2-[(2-chlorphenyl)imino]imidazolidin und Bromwasserstoffsäure erhält man 2-[(2-Chlorphenyl)imino]-1-hydroxyimidazolidin-hydrobromid, Smp. 198—200° (aus Methanol/Acetonitril);
— aus 1-Benzyloxy-2-[(2-bromphenyl)imino]imidazolidin und Bromwasserstoffsäure erhält man 2-[(2-Bromphenyl)imino]-1-hydroxyimidazolidin-hydrobromid, Smp. 194—196° (aus Acetonitril).

## Beispiel 17

In analoger Weise wie in Beispiel 7 beschrieben wurde die folgende Verbindung hergestellt:

— Aus 1-Benzyloxy-2-[(2-trifluormethylphenyl)imino]imidazolidin und Wasserstoff in Gegenwart von Palladium/Kohle in äthanolischer Bromwasserstoffsäure erhält man 1-Hydroxy-2-[(2-trifluormethylphenyl)imino]imidazolidin-hydrobromid, Smp. 182—183° (aus Acetonitril).

## Beispiel 18

327 mg 1 mMol) 2-[(2,6-Dichlorphenyl)imino]-1-hydroxyimidazolidin-hydrobromid werden in 5 ml abs. Dimethylformamid gelöst und bei 5° mit 90 mg (2,06 mMol) Natriumhydrid versetzt. Man rührt bei Raumtemperatur bis die Lösung klar wird, kühlt wieder bis auf 5° und gibt 1,2 ml (1,2 mMol) einer 1,0molaren Methyljodidlösung in Dimethylformamid zu. Nach 30 Minuten wird das Reaktionsprodukt auf Wasser gegossen und die wässerige Phase zweimal mit Äther extrahiert. Die organischen Extrakte werden getrocknet und im Vakuum eingedampft. Der Rückstand wird in 1 ml Acetonitril gelöst und mit Chlorwasserstoffsäure in Dioxan versetzt. Das ausgefallene 2-[(2,6-Dichlorphenyl)imino]-1-methoxyimidazolidin-hydrochlorid schmilzt bei 228°.

## Beispiel 19

In zu Beispiel 18 analoger Weise wird aus 2-[(2,6-Dichlorphenyl)imino]-1-hydroxyimidazolidin-hydrobromid und Diäthylaminoäthylchlorid das 2-[(2,6-Dichlorphenyl)imino]-1-[2-(dimethylamino)äthoxy]imidazolidin hergestellt. Das entsprechende Hydrochlorid schmilzt bei 213—215° (aus Acetonitril/Essigester).

## Beispiel 20

Zu einer Lösung von 6,15 g (30 mMol) 2-[(2,6-Dichlorphenyl)imino]-1-hydroxyimidazolidin in 45 ml abs. Dimethylformamid werden bei einer Temperatur von 25° 1,31 g (30 mMol) Natriumhydrid (55%ige Suspension) zugegeben. Nach 30 Minuten werden 4,16 g (35 mMol; 2,37 ml, d = 1,579) Propargylbromid zugetropft. Nach 30 Minuten wird auf 500 ml einer gesättigten Kochsalzlösung gegossen und die wässerige Phase mit Äther extrahiert. Die Ätherextrakte werden mit wässeriger Salzsäure gewaschen. Die wässerigen Extrakte werden dann mit 3 N-Natronlauge basisch gestellt und mit Äther extrahiert. Nach dem Eindampfen des Lösungsmittels wird der Rückstand in Äther gelöst und mit Chlorwasserstoffsäure in Dioxan versetzt. Der Rückstand wird aus Methanol/Acetonitril umkristallisiert, wobei man 2-[(2,6-Dichlorphenyl)imino]-1-(2-propinyloxy)-imidazolidin-hydrochlorid erhält, Smp. 198—199° (aus Methanol/Acetonitril).

## Beispiel 21

In analoger Weise wie in Beispiel 20 beschrieben wurden die folgenden Verbindungen hergestellt:

— Aus 2-[(2,6-Dichlorphenyl)imino]-1-hydroxyimidazolidin und $\beta$-Phenäthylbromid erhält man 2-[(2,6-Dichlorphenyl)imino]-1-(phenäthyloxy)imidazolidin, Smp. 111—112° (aus Diisopropyläther/Hexan).
Das entsprechende Hydrochlorid wurde aus Aceton/Essigester umkristallisiert, Smp. 167—169°;

— aus 2-[(2,6-Dichlorphenyl)imino]-1-hydroxyimidazolidin und 3-(Diäthylamino)-2,2-dimethylpropylchlorid erhält man 2-[(2,6-Dichlorphenyl)imino]-1-[3-(diäthylamino)-2,2-dimethylpropoxy]imidazolidin. Das entsprechende (R,R)-tartrat (1 : 1), welches mit 1,1 Mol Säure kristallisiert, wurde aus Aceton/Äther kristallisiert und schmilzt bei 156—157°.

## Beispiel 22

6,15 g (25 mMol) 2-[(2,6-Dichlorphenyl)imino]-1-hydroxyimidazolidin werden in 25 ml Dimethylformamid und 45 ml Toluol bei Raumtemperatur mit 1,2 g (30,7 mMol) Natriumamid gerührt. Nach einer Stunde wird das Gemisch bei 50° bis zum Erhalt einer klaren Lösung erwärmt. Danach wird das Gemisch auf Raumtemperatur gekühlt und mit 3,4 ml 2-Chloräthylmethylsulfid versetzt. Dann erwärmt man das Gemisch auf 60° und läßt 16 Stunden reagieren. Das Reaktionsgemisch wird danach auf Wasser gegossen und mit Äther extrahiert. Die ätherischen Extrakte werden dann mit wässeriger Salzsäure extrahiert. Die wässerigen Extrakte werden dann mit 3 N Natronlauge wiederum basisch gestellt und mit Äther extrahiert. Nach dem Trocknen und dem Eindampfen des Lösungsmittels wird der ölige Rückstand an Kieselgel chromatographiert (Eluierungsmittel Chloroform/Essigester 9 : 1). Das erhaltene gelbe Öl wird dann in Aceton gelöst und mit Chlorwasserstoffsäure in Dioxan versetzt. Der gebildete Niederschlag wird aus Aceton umkristallisiert, wobei man 2-[(2,6-Dichlorphenyl)imino]-1-[2-(methylthio)äthoxy]imidazolidin-hydrochlorid erhält, Smp. 156° (Zers.).

In analoger Weise wurde die folgende Verbindung hergestellt:

— Aus 2-[(2,6-Dichlorphenyl)imino]-1-hydroxyimidazolidin und 4-Brombuttersäureäthylester erhält man Äthyl 4-[/2-[(2,6-dichlorphenyl)imino]-1-imidazolidinyl/oxy]butyrat. Das entsprechende Hydrochlorid schmilzt bei 113—115° (aus Isopropanol/Aceton).

## Beispiel 23

4,80 g (13,32 mMol) Äthyl 4-[/2-[(2,6-dichlorphenyl)imino]-1-imidazolidinyl/oxy]butyrat werden in 100 ml Äthanol gelöst, bei Raumtemperatur mit 26,6 ml 1 N Natronlauge versetzt und über Nacht stehengelassen. Die Lösung wird danach mit 26,6 ml 1 N Salzsäure neutralisiert und im Vakuum eingedampft. Der getrocknete Rückstand wird mit einem heißen 1 : 1 : 1-Gemisch aus Acetonitril/Essigester/Chloroform digeriert. Die Lösung wird dann vom Salz abfiltriert und im Vakuum eingedampft. Der Rückstand wird aus heißem Acetonitril umkristallisiert, wobei man 4-[/2-[(2,6-Dichlorphenyl)imino]-1-imidazolidinyl/oxy]-buttersäure erhält, Smp. 115—116° (aus Acetonitril).

## Beispiel 24

38,80 g (107,7 mMol) Äthyl 4-[/2-[(2,6-dichlorphenyl)imino]-1-imidazolidinyl/oxy]butyrat und 50 ml 25%ige Salzsäure werden während 45 Minuten auf 125° erwärmt. Die abgekühlte Lösung wird mit Natriumacetat auf pH 5 eingestellt und mit Chloroform extrahiert. Die organischen Extrakte werden über Magnesiumsulfat getrocknet und im Vakuum eingedampft. Der Rückstand wird aus Chloroform/Äther umkristallisiert, wobei man 4-[/2-[(2,6-Dichlorphenyl)imino]-1-imidazolidinyl/oxy]-buttersäure erhält, Smp. 115°.

Beispiel 25

In analoger Weise wie in Beispiel 22 beschrieben wurde die folgende Verbindung hergestellt:

— Aus 2-[(2,6-Dichlorphenyl)imino]-1-hydroxyimidazolidin und 4-[2-Tetrahydropyranyl)oxy]-brompropan erhält man 3-[/2-[(2,6-Dichlorphenyl)imino]-1-imidazolidinyl/oxy]-1-propanol, Smp. 115—117° (aus Methylenchlorid/Diisopropyläther). Das entsprechende Hydrochlorid schmilzt bei 150—152° (aus Isopropanol/Essigester).

Beispiel 26

In analoger Weise wie in Beispiel 20 beschrieben wurde aus 2-[(2,6-Dichlorphenyl)imino]-1-hydroxyimidazolidin und $\alpha$-Bromphenylessigsäure-t.-butylester t.-Butyl//2-[(2,6-dichlorphenyl)imino]-1-imidazolidinyl/oxy/phenylacetat hergestellt, Smp. 88—90° (aus Cyclohexan).

Beispiel 27

In zu Beispiel 24 analoger Weise, aber unter Verwendung von 1 N äthanolischer Salzsäure erhält man aus t.-Butyl //2-[(2,6-dichlorphenyl)imino]-1-imidazolidinyl/oxy/phenylacetat das //2-[(2,6-Dichlorphenyl)imino]-1-imidazolidinyl/oxy/phenylessigsäure-hydrochlorid, Smp. 184—186° (aus Chloroform/Acetonitril).

Beispiel 28

In analoger Weise wie in Beispiel 22 beschrieben wurden die folgenden Verbindungen hergestellt:

— Aus 2-[(2,6-Dichlorphenyl)imino]-1-hydroxyimidazolidin und Allylbromid erhält man 1-(Allyloxy)-2-[(2,6-dichlorphenyl)imino]imidazolidin-hydrochlorid, Smp. 169—171° (aus Acetonitril);
— aus 2-[(2,6-Dichlorphenyl)imino]-1-hydroxyimidazolidin und Benzylbromid erhält man 1-Benzyloxy-2-[(2,6-dichlorphenyl)imino]imidazolidin, Smp. 115—117° (aus Diisopropyläther);
— aus 2-[(2,6-Dichlorphenyl)imino]-1-hydroxyimidazolidin und Bromessigester erhält man Äthyl//2-[(2,6-dichlorphenyl)imino]-1-imidazolidin/oxy/acetat-hydrochlorid, Smp. 171—173° (aus Acetonitril/Diäthyläther);
— aus 2-[(2,6-Dichlorphenyl)imino]-1-hydroxyimidazolidin und Bromessigester erhält man Äthyl//2-[(2,6-dichlorphenyl)imino]-1-imidazolidin/oxy/acetat-hydrochlorid, Smp. 171—173° (aus Acetonitril/Diäthyläther):
— aus 2-[(2,6-Dichlorphenyl)imino]-1-hydroxyimidazolidin und Propylbromid erhält man 2-[(2,6-Dichlorphenyl)imino]-1-propoxyimidazolidin-hydrochlorid, Smp. 217—218° (aus Methylenchlorid/Essigester);
— aus 2-[(2,6-Dichlorphenyl)imino]-1-hydroxyimidazolidin und 5-Bromvaleronitril erhält man 5-[/2-[(2,6-Dichlorphenyl)imino]-1-imidazolidinyl/oxy]valeronitril-hydrochlorid, Smp. 183—185° (aus Aceton/Diäthyläther);
— aus 2-[(2,6-Dichlorphenyl)imino]-1-hydroxyimidazolidin und $\beta$-Methoxyäthyl-$\beta$-chloräthyläther erhält man 2-[(2,6-Dichlorphenyl)imino]-1-[2-(2-methoxyäthoxy)äthoxy]imidazolidin-methansulfonat (1 : 1), Smp. 103—104° (aus Aceton/Diäthyläther);
— aus 2-[(2,6-Dichlorphenyl)imino]-1-hydroxyimidazolidin und 4-Brom-1-buten erhält man 1-(3-Butenyl)-2-[(2,6-dichlorphenyl)imino]imidazolidin-methansulfonat, Smp. 155—157° (aus Aceton/Diäthyläther);
— aus 2-[(2,6-Dichlorphenyl)imino]-1-hydroxyimidazolidin und 5-Brom-1-penten erhält man 2-[(2,6-Dichlorphenyl)imino]-1-(4-pentenyl)-imidazolidin-hydrochlorid, Smp. 203—207° (aus Aceton);
— aus 2-[(2,6-Dichlorphenyl)imino]-1-hydroxyimidazolidin und Brombuttersäureisopropylester erhält man Isopropyl 4-[/2-[(2,6-dichlorphenyl)imino]-1-imidazolidinyl/oxy]-butyrat-hydrochlorid, Smp. 134—135° (aus Essigester);
— aus 2-[(2,6-Dichlorphenyl)imino]-1-hydroxyimidazolidin und Homoveratrylchlorid erhält man 2-[(2,6-Dichlorphenyl)imino]-1-[(3,4-dimethoxyphenäthyl)oxy]imidazolidin-oxalat (1 : 1), Smp. 167—168° (aus Acetonitril);
— aus 2-[(2,6-Dichlorphenyl)imino]-1-hydroxyimidazolidin und 4-Chlorbutyronitril erhält man 4-[/2-(2,6-Dichlorphenyl)imino]-1-imidazolidinyl/oxy]butyronitril als ein Öl; das entsprechende Hydrochlorid schmilzt bei 183—184° (aus Acetonitril/Essigester);
— aus 2-[(2,6-Dichlorphenyl)imino]-1-hydroxyimidazolidin und 5-Bromvaleriansäureäthylester erhält man Äthyl 5-[/2-[(2,6-dichlorphenyl)imino]-1-imidazolidinyl/oxy]-valerat-oxalat (1 : 1), Smp. 96° (aus Essigester);

- aus 1-Hydroxy-2-(phenylimino)imidazolidin und Brombuttersäureäthylester erhält man Äthyl 4-/[2-(phenylimino)-1-imidazolidinyl]oxy/butyrat als ein Öl;
- aus 2-[(6-Chlor-2-methylphenyl)imino]-1-hydroxyimidazolidin und 4-Brombuttersäureäthylester erhält man Äthyl 4-[/2-[(6-chlor-2-methylphenyl)imino]-1-imidazolidinyl/oxy]-butyrat als ein Öl;
- aus 1-Hydroxy-2-[(2,6-dimethylphenyl)imino]imidazolidin und 4-Brombuttersäureäthylester erhält man Äthyl 4-[/2-[(2,6-dimethylphenyl)imino]-1-imidazolidinyl/oxy]butyrat als ein Öl:
- aus 2-[(2,6-Dichlorphenyl)imino]-1-hydroxyimidazolidin und δ-Chlorbutyl-(2-tetrahydropyranyl)-äther und anschließender Behandlung mit 3 N Salzsäure erhält man 4-[/2-[(2,6-Dichlorphenyl)-imino]-1-imidazolidinyl/oxy]-1-butanol-hydrochlorid, Smp. 175—176° (aus Methanol/Acetonitril).

## Beispiel 29

In zu Beispiel 23 analoger Weise wurden die folgenden Verbindungen hergestellt:

- Aus Äthyl 5-[/2-[(2,6-dichlorphenyl)imino]-1-imidazolidinyl/oxy/valerat und Natronlauge erhält man 5-[/2-[(2,6-Dichlorphenyl)imino]-1-imidazolidinyl/oxy]-valeriansäure, Smp. 148—150° (aus Acetonitril/Essigester);
- aus Äthyl [/2-[(2,6-dichlorphenyl)imino]-1-imidazolidinyl/oxy]acetat und Natronlauge erhält man [/2-[(2,6-Dichlorphenyl)imino]-1-imidazolidinyl/oxy]essigsäure, Smp. 175—176° (aus Acetonitril);
- aus Äthyl 4-/[2-(phenylimino)-1-imidazolidinyl]oxy/butyrat und Natronlauge erhält man 4-[(2-Phenylimino)-1-imidazolidinyl]oxy/buttersäure als ein glasiges Material;
- aus Äthyl 4-[/2-[(6-chlor-2-methylphenyl)imino]-1-imidazolidinyl/oxy]butyrat und Natronlauge erhält man 4-[/2-[(6-Chlor-2-methylphenyl)imino]-1-imidazolidinyl/oxy]buttersäure, Smp. 101—103° (Zers.) (aus Acetonitril);
- aus Äthyl-4-[/2-[(2,6-dimethylphenyl)imino]-1-imidazolidinyl/oxy]butyrat und Natronlauge erhält man 4-/[2-(2,6-dimethylphenyl)-1-imidazolidinyl]oxy/buttersäure, Smp. 130—132° (aus Acetonitril).

## Beispiel 30

5,51 g (15 mMol) Äthyl 4-[/2-[(2,6-dichlorphenyl)imino]-1-imidazolidinyl/oxy]butyrat, 200 mg Natriummethylat und methanolischer Ammoniak werden unter Druck während 24 Stunden erwärmt. Die Lösung wird danach im Vakuum eingedampft. Der Rückstand wird aus Methylenchlorid/Diäthyläther und dann aus Benzol/Cyclohexan (1 : 1) umkristallisiert, wobei man 4-[/2-[(2,6-Dichlorphenyl)imino]-1-imidazolidinyl/oxy]-buttersäureamid erhält, Smp. 140—141°.

## Beispiel 31

1,0 g Äthyl 4-[/2-[(2,6-dichlorphenyl)imino]-1-imidazolidinyl/oxy]butyrat wird bei Raumtemperatur über Nacht in 20 ml methanolischer Salzsäure stehengelassen. Das Reaktionsgemisch wird danach mit Toluol zur Trockene eingedampft. Der Rückstand wird mit Natriumcarbonat und Äther versetzt. Die organische Phase wird über Natriumsulfat getrocknet und zur Trockene eingedampft. Umkristallisation des Rückstandes aus Cyclohexan liefert Methyl 4-[/2-[(2,6-dichlorphenyl)imino]-1-imidazolidinyl/oxy]butyrat, Smp. 95—96°.

## Beispiel 32

In analoger Weise wie in Beispiel 22 beschrieben wurden die folgenden Verbindungen hergestellt:

- Aus 2-[(2,6-Dichlorphenyl)imino]-1-hydroxyimidazolidin und 4-Bromcrotonsäureäthylester erhält man Äthyl 4-[/2-[(2,6-dichlorphenyl)imino]-1-imidazolidinyl/oxy]-crotonat, Smp. 137—138° (aus Acetonitril); das entsprechende Hydrochlorid schmilzt bei 139—140° (aus Aceton/Diäthyläther);
- aus 2-[(2-Chlorphenyl)imino]-1-hydroxyimidazolidin und Propargylbromid erhält man 2-[(2-Chlorphenyl)imino]-1-(2-propynyloxy)imidazolidin-hydrochlorid, Smp. 174—175° (aus Methanol/Acetonitril);
- aus 2-[(2-Chlorphenyl)imino]-1-hydroxyimidazolidin und 4-Brombuttersäureäthylester erhält man Äthyl 4-[/2-[(2-chlorphenyl)imino]-1-imidazolidinyl/oxy]butyrat als ein Öl;
- aus 2-[(2,6-Dichlorphenyl)imino]-1-hydroxyimidazolidin und 4-Brom-2-methylbuttersäureäthylester erhält man Äthyl 4-[/2-[(2,6-dichlorphenyl)imino]-1-imidazolidinyl/oxy]-2-methylbutyrat als ein Öl;
- aus 2-[(2,6-Dichlorphenyl)imino]-1-hydroxyimidazolidin und Isopropyljodid erhält man 2-[(2,6-Dichlorphenyl)imino]-1-isopropoxy-imidazolidin-hydrochlorid, Smp. 204—205° (aus Acetonitril);
- aus 2-[(2,6-Dichlorphenyl)imino]-1-hydroxyimidazolidin und 2-Brombuttersäureäthylester erhält man Äthyl 2-[/2-[(2,6-dichlorphenyl)imino]-1-imidazolidinyl/oxy]-butyrat-hydrochlorid, Smp. 176—177° (aus Acetonitril);
- aus 1-Hydroxy-2[(2-jodphenyl)imino]imidazolidin und 4-Brombuttersäureäthylester erhält man Äthyl 4-[/2-[(2-jodphenyl)imino]-1-imidazolidinyl/oxy]butyrat als ein Öl;
- aus 2-[(2,6-Dichlorphenyl)imino]-1-hydroxyimidazolidin und α-Bromisobuttersäureäthylester erhält man Äthyl2-[/2-[(2,6-dichlorphenyl)imino]-1-imidazolidinyl/oxy]-2-methylpropionat-hydrochlorid, Smp. 171 —172° (aus Essigester/Chlorwasserstoffsäure in Dioxan/Diäthyläther);
- aus 2-[(2,6-Dichlorphenyl)imino]-1-hydroxyimidazolidin und 3-Methoxypropylchlorid erhält man 2-[(2,6-Dichlorphenyl)imino]-1-(3-methoxypropoxy)imidazolidin, Smp. 95—96° (aus Isopropyläther);
- aus 2-[(2,6-Dichlorphenyl)imino]-1-hydroxyimidazolidin und Äthyljodid erhält man 2-[(2,6-Dichlorphenyl)imino]-1-äthoxyimidazolidin-hydrochlorid, Smp. 219—221° (aus Aceton);
- aus 2-[(2,6-Dichlorphenyl)imino]-1-hydroxyimidazolidin und trans-Cinnamylbromid erhält man 1-(Cinnamyloxy)-2-[(2,6-dichlorphenyl)imino]imidazolidin-hydrochlorid, Smp. 123—125° (aus Acetonitril/Chlorwasserstoffsäure in Dioxan/Diäthyläther).

## Beispiel 33

In analoger Weise wie in Beispiel 23 beschrieben wurden die folgenden Verbindungen hergestellt:

- Aus Äthyl 4-[/2-[(2-jodphenyl)imino]-1-imidazolidinyl/oxy]butyrat und Natronlauge erhält man 4-[/2-[(2-jodphenyl)imino]-1-imidazolidinyl/oxy]buttersäure als ein Schaum;
- aus Äthyl 2-[/2-[(2,6-dichlorphenyl)imino]-1-imidazolidinyl/oxy]butyrat und Natronlauge erhält man 2-[/2-[(2,6-Dichlorphenyl)imino]-1-imidazolidinyl/oxy]buttersäure als ein Schaum;
- aus Äthyl 2-[/2-[(2,6-dichlorphenyl)imino]-1-imidazolidinyl/oxy]-2-methylpropionat und Natronlauge erhält man 2-[/2-[(2,6-Dichlorphenyl)imino]-1-imidazolidinyl/oxy]-2-methylpropionsäure, Smp. 194—195° (aus Chloroform);
- aus Äthyl 4-[/2-[(2-chlorphenyl)imino]-1-imidazolidinyl/oxy]butyrat und Natronlauge erhält man 4-[/2-[(2-chlorphenyl)imino]-1-imidazolidinyl/oxy]buttersäure als ein Schaum;
- aus Äthyl 4-[/2-[(2,6-dichlorphenyl)imino]-1-imidazolidinyl/oxy]-2-methylbutyrat und Natronlauge erhält man 4-[/2-[(2,6-Dichlorphenyl)imino]-1-imidazolidinyl/oxy]-2-methylbuttersäure; Schmelzpunkt des Hydrochlorids 202—203° (aus Acetonitril);
- aus Äthyl 2-/[/2-[(2,6-dichlorphenyl)imino]-1-imidazolidinyl/oxy]methyl/-3-furoat und Natronlauge erhält man 2-/[/2-[(2,6-Dichlorphenyl)imino]-1-imidazolidinyl/oxy]methyl/-3-furansäure, Smp. 175° (Zers.) (aus Acetonitril).

## Beispiel 34

1,2 g (0,5 mMol) N-(Benzyloxy)äthylendiamin-dihydrochlorid werden mit 20 ml 48%iger Bromwasserstoffsäure wähernd 3 Stunden auf 80° erwärmt. Die warme wässerige Lösung wird danach rasch mit Cyclohexan gewaschen. Aus dieser sauren Lösung kristallisiert das N-Hydroxyäthylendiamin-dihydrobromid, Smp. 172° (Zers.).

2,38 g (10 mMol) N-Hydroxyäthylendiamin-dihydrobromid, 15 ml Eisessig und 5,6 g (~40 mMol) Kaliumcarbonat werden auf dem Dampfbad bis zum Abschluß der $CO_2$-Entwicklung erwärmt. Das Gemisch wird danach abgekühlt, mit 2,66 g (11 mMol) 2,6-Dichlorphenylimidocarbonylchlorid versetzt, dann während 3 Minuten auf dem Dampfbad stark erwärmt und anschließend 1 Stunde bei Raumtemperatur stehengelassen. Das Reaktionsprodukt wird auf 100 ml Wasser und 40 ml 3 N Schwefelsäure gegossen. Das überschüssige 2,6-Dichlorphenylimidocarbonylchlorid wird mit Methylenchlorid extrahiert. Die wässerige Lösung wird mit konz. Natronlauge auf pH 6 eingestellt und danach mit 50 ml gesättigter Natriumcarbonatlösung versetzt, wobei 2-[(2,6-Dichlorphenyl)imino]-1-hydroxyimidazolidin vom Schmelzpunkt 219—220° als kristalliner Niederschlag ausfällt. Das erhaltene Produkt ist mit dem in Beispiel 3 erhaltenen Produkt identisch.

## Beispiel 35

In zu Beispiel 11 analoger Weise werden die folgenden Verbindungen hergestellt:

- Aus 2,5-Dichlorphenylimidocarbonylchlorid und N-(Benzyloxy)äthylendiamin erhält man 1-(Benzyloxy-2-[2,5-dichlorphenyl)imino]-imidazolidin-hydrochlorid, Smp. 206—207° (aus Acetonitril);
- aus 2,3-Dichlorphenylimidocarbonylchlorid und N-(Benzyloxy)äthylendiamin erhält man 1-(Benzyl-

oxy)-2-[(2,3-dichlorphenyl)imino]-imidazolidin-hydrochlorid, Smp. 200–201° (aus Acetonitril/Chlorwasserstoffsäure in Dioxan);

- aus 2,4-Dichlorphenylimidocarbonylchlorid und N-(Benzyloxy)äthylendiamin erhält 1-(Benzyloxy)-2-[(2,4-dichlorphenyl)imino]-imidazolidin-hydrochlorid, Smp. 178–180° (aus Aceton/Chlorwasserstoffsäure in Dioxan).

Beispiel 36

In zu Beispiel 2 analoger Weise wurden die folgenden Verbindungen hergestellt:

- Aus 1-(Benzyloxy)-2-[(2,5-dichlorphenyl)imino]-imidazolidin erhält man 2-[(2,5-Dichlorphenyl)imino]-1-hydroxyimidazolidin-hydrobromid, Smp. 214–215° (aus Methanol/Acetonitril);
- aus 1-(Benzyloxy)-2-[(2,3-dichlorphenyl)imino]-imidazolidin erhält man 2-[(2,3-Dichlorphenyl)imino]-1-hydroxyimidazolidin-hydrobromid, Smp. 190–191° (aus Methanol/Acetonitril);
- aus 1-(Benzyloxy)-2-[(2,4-dichlorphenyl)imino]-imidazolidin enthält man 2-[(2,4-Dichlorphenyl)-imino]-1-hydroxyimidazolidin-hydrobromid, Smp. 217–218° (aus Methanol/Acetonitril).

Beispiel 37

In zu Beispiel 1, Absatz D, analoger Weise erhält man

- aus N-/2-[/2-[(2,6-Dichlorphenyl)imino]-1-imidazolidinyl/oxy]äthyl/phthalimid 1-(2-Aminoäthoxy)-2-[(2,6-dichlorphenyl)imino]imidazolidin, Smp. 129–130° (aus Acetonitril); das entsprechende Dihydrochlorid schmilzt bei 238–240° (Zers.) (aus Acetonitril/Diäthyläther);
- aus N-/4-[/2-[(2,6-Dichlorphenyl)imino]-1-imidazolidinyl/oxy]butyl/phthalimid 1-(4-Aminobutoxy)-2-[(2,6-dichlorphenyl)imino]imidazolidin; das entsprechende Dihydrochlorid schmilzt bei 234–237° (aus Acetonitril/Methanol).

Beispiel 38

In analoger Weise wie in Beispiel 20 beschrieben wurden die folgenden Verbindungen hergestellt:

- Aus 2-[(2,6-Dichlorphenyl)imino]-1-hydroxyimidazolidin und 1-Brom-2-butin erhält man unter Verwendung von Benzol als Lösungsmittel 1-(2-Butinyloxy)-2-[(2,6-dichlorphenyl)imino]imidazolidin-hydrochlorid, Smp. 198° (Zers.) (aus Acetonitril/Chlorwasserstoffsäure in Dioxan);
- aus 2-[(2,6-Dichlorphenyl)imino]-1-hydroxyimidazolidin und 2-Chlormethylfuran erhält man unter Verwendung von Toluol als Lösungsmittel 2-[(2,6-Dichlorphenyl)imino]-1-(furfuryloxy)-imidazolidin, Smp. 123–124° (aus Isopropyläther); das entsprechende Hydrochlorid schmilzt bei 162–164° (aus Acetonitril/Dioxan/Diäthyläther);
- aus 2-[(2,6-Dichlorphenyl)imino]-1-hydroxyimidazolidin und Äthyl 2-chlormethyl-3-furoat erhält man unter Verwendung von Toluol als Lösungsmittel Äthyl 2-/[/2-[(2,6-dichlorphenyl)imino]-1-imidazolidinyl/oxy]methyl/-3-furoat, Smp. 99° (aus Isopropyläther); das entsprechende Fumarat schmilzt bei 154–156° (aus Methanol/Acetonitril);
- aus 2-[(2,6-Dichlorphenyl)imino]-1-hydroxyimidazolidin und 2-Chlormethylthiophen erhält man unter Verwendung von Toluol als Lösungsmittel 2-[(2,6-Dichlorphenyl)imino]-1-(2-thienylmethoxy)-imidazolidin, Smp. 101–102° (aus Methylenchlorid/Cyclohexan); das entsprechende Hydrochlorid schmilzt bei 211° (Zers.) (aus Aceton/Chlorwasserstoffsäure in Dioxan).

Beispiel 39

In zu Beispiel 20 analoger Weise erhält man

- aus 2-[(2,6-Dichlorphenyl)imino]-1-hydroxyimidazolidin und $\beta$-Methoxyäthylchlorid 2-[(2,6-Dichlorphenyl)imino]-1-(2-methoxyäthoxy)imidazolidin-hydrochlorid, Smp. 125–127° (aus Aceton/Chlorwasserstoffsäure in Dioxan/Diäthyläther);
- aus 2-[(2,6-Dichlorphenyl)imino]-1-hydroxyimidazolidin und 4-Brom-3-methylcrotonsäuremethylester (cis-trans-Gemisch) Methyl 4-[/2-[(2,6-dichlorphenyl)imino]-imidazolidinyl/oxy]-3-methylcrotonat als ein cis-trans-Gemisch 1 : 1, Smp. 84–85° (aus Isopropyläther).

Beispiel 40

14,12 g (41,6 mMol) Tetrabutylammoniumhydrogensulfat, 9,84 g (40 mMol) 2-[(2,6-Dichlorphenyl)-imino]-1-hydroxyimidazolidin, 7,97 g (44 mMol) α-Brompropionsäureäthylester und 140 ml Methylenchlorid werden bei Raumtemperatur gerührt. Dazu werden langsam 42 ml 2,0 N Natronlauge gegossen, wobei eine sofortige Reaktion eintritt. Das Methylenchlorid wird im Vakuum abdestilliert und durch Äther ersetzt. Die ätherische Lösung wird mit Wasser gewaschen, über Magnesiumsulfat getrocknet und im Vakuum eingedampft. 15 g eines kristallinen Produkts werden erhalten und aus Cyclohexan umkristallisiert, wobei man Äthyl 2-[/2-[(2,6-dichlorphenyl)imino]-1-imidazolidinyl/oxy]-propionat vom Schmelzpunkt 79—80° erhält.

Das Hydrochlorid schmilzt bei 185—186° (aus Acetonitril/Chlorwasserstoffsäure in Dioxan/Diäthyläther).

In analoger Weise wurden die folgenden Verbindungen hergestellt:

— Aus 2-[(2,6-Dichlorphenyl)imino]-1-hydroxyimidazolidin und 3-Brommethylthiophen erhält man 2-[(2,6-Dichlorphenyl)imino]-1-(3-thienylmethoxy)-imidazolidin-hydrochlorid, Smp. 204—206° (aus Aceton/Chlorwasserstoffsäure in Dioxan/Diäthyläther);
— aus 2-[(2,6-Dichlorphenyl)imino]-1-hydroxyimidazolidin und 4-Brom-2-methyl-2-buten erhält man 2-[(2,6-Dichlorphenyl)imino]-1-[(3-methyl-2-butenyl)oxy]imidazolidin-hydrochlorid, Smp. 153—154° (aus Acetonitril/Chlorwasserstoffsäure in Dioxan/Diäthyläther);
— aus 2-[(2,6-Dichlorphenyl)imino]-1-hydroxyimidazolidin und 3-Brommethylfuran erhält man 2-[(2,6-Dichlorphenyl)imino]-1-(3-furylmethoxy)-imidazolidin-hydrochlorid, Smp. 204—206° (aus Acetonitril).

Beispiel 41

2,30 g (10 mMol) 2-[(2,6-Dichlorphenyl)imino]imidazolidin werden in 10 ml konz. Schwefelsäure gelöst und portionsweise bei Raumtemperatur mit 2,97 g (11 mMol) Kaliumpersulfat versetzt. Die Temperatur wird mit einem Kühlbad unter 50° gehalten. Die dunkle Lösung wird mit Wasser verdünnt und mit konz. Natronlauge stark alkalisch gestellt. Das nicht umgesetzte Ausgangsmaterial wird mit Äther extrahiert. Die wässerige Lösung wird mit Eisessig und dann mit Natriumbicarbonat auf pH 7 gestellt. Man filtriert die Lösung vom Rückstand und gibt eine Kaliumcarbonatlösung bis zum pH 10,5 zu. Das ausgefallene Produkt wird mit Essigester extrahiert und zweimal aus Acetonitril umkristallisiert, wobei man 2-[(2,6-Dichlorphenyl)imino]-1-hydroxyimidazolidin vom Schmelzpunkt 215° erhält, welches mit dem in Beispiel 3 erhaltenen Produkt identisch ist.

Beispiel 42

6,95 g Hydroxylamin-hydrochlorid (100 mMol) und 13,61 g Natriumacetattrihydrat (100 mMol) werden in 50 ml Wasser gelöst und mit 4,31 g Aziridin versetzt (der pH-Wert beträgt 7,61). Nach 10 Minuten ist die Temperatur auf 42° gestiegen und der pH-Wert auf 6,74 gefallen. Die wäßrige Lösung wird dann unter vermindertem Druck bei 30° eingedampft. Der Rückstand wird in Eisessig aufgenommen und mit 24,23 g (2,6-Dichlorphenyl)imidocarbonylchlorid (100 mMol) wie in Beispiel 34, zweiter Absatz, versetzt. Man erhält so 2-[(2,6-Dichlorphenyl)imino]-1-hydroxyimidazolidin, Smp. 221—222° (aus Acetonitril).

Beispiel 43

In analoger Weise wie in Beispiel 30 beschrieben wurden die folgenden Verbindungen hergestellt:

— Aus Äthyl 4-/[2-(2,6-dichlorphenyl)imino]-1-imidazolidinyl/oxy]butyrat und Methylamin erhält man 4-[/2-[(2,6-Dichlorphenyl)imino]-1-imidazolidinyl/oxy]-N-methylbutyramid, Smp. 147—149° (aus Methylenchlorid/Isopropyläther);
— aus Äthyl 4-[/2-[(2,6-dichlorphenyl)imino]-1-imidazolidinyl/oxy]butyrat und Dimethylamin erhält man 4-[/2-[(2,6-Dichlorphenyl)imino]-1-imidazolidinyl/oxy]-N,N-dimethylbutyramid, Smp. 126—128° (aus Methylenchlorid/Isopropyläther).

Beispiel 44

Zu 24,6 g O-Benzylhydroxylamin werden 25 ml 50%ige Tetrafluorborsäure zugegeben, wobei die Temperatur bis 70° steigt. Diese Lösung wird bei 50° mit einer Lösung von 13 ml Aziridin in 13 ml

Wasser so versetzt, daß die Temperatur 60° nicht übersteigt. Am Ende der exothermen Reaktion wird auf 5° abgekühlt und mit einer Lösung aus 56 g Kaliumcarbonat in 100 ml Wasser versetzt. Unter kräftigem Rühren werden 60 ml Toluol und 35 ml (2,6-Dichlorphenyl)imidocarbonylchlorid zugegeben. Unter $CO_2$-Entwicklung steigt die Temperatur auf 43°. Das Reaktionsgemisch wird während 90 Minuten weitergerührt und dann mit 150 ml Hexan und 200 ml Wasser versetzt. Die kristalline Masse wird sorgfältig mit Wasser und Hexan gewaschen und über Nacht unter vermindertem Druck getrocknet. Die getrocknete Substanz wird aus Isopropyläther umkristallisiert, wobei man 1-(Benzyloxy)-2-[(2,6-dichlorphenyl)imino]imidazolidin erhält, Smp. 115–117°.

Beispiel 45

In analoger Weise wie in Beispiel 40 beschrieben wurde die folgende Verbindung hergestellt, wobei jedoch anstelle von Methylenchlorid Toluol als Lösungsmittel und anstelle von 2 N-Natronlauge 28%ige Natronlauge verwendet wurde:

— Aus 2-[(2,6-Dichlorphenyl)imino]-1-hydroxyimidazolidin und 2-Chlormethylpyridin erhält man 2-/[/2-[(2,6-Dichlorphenyl)imino]-1-imidazolidinyl/oxy]methyl/pyridin, Smp. 116–118° (aus Isopropyläther); das entsprechende Dihydrochlorid schmilzt bei 175–176° unter Zersetzung (aus Methanol/Acetonitril).

Beispiel 46

In analoger Weise wie in Beispiel 20 beschrieben wurde die folgende Verbindung hergestellt:

— Aus 2-[(2,6-Dichlorphenyl)imino]-1-hydroxyimidazolidin und Pentin-3-yl-p-toluolsulfonat erhält man 2-[(2,6-Dichlorphenyl)imino]-1-(3-pentinyloxy)-imidazolidin, Smp. 104–105° (aus Isopropyläther).

Beispiel 47

In analoger Weise wie in Beispiel 11 beschrieben wurde die folgende Verbindung hergestellt:

— Aus (2,6-Dichlor-4-fluorphenyl)imidocarbonylchlorid und 1-(Benzyloxy)-2-[(2,6-dichlorphenyl)imino]-imidazolidin erhält man 1-(Benzyloxy)-2-[(2,6-dichlor-4-fluorphenyl)imino]imidazolidin, Smp. 112–113° (aus Cyclohexan).

Das Ausgangsmaterial kann wie folgt hergestellt werden:

97,8 g 4-Fluoranilin (0,88 Mol) werden in 600 ml Eisessig gelöst und mit einigen Tropfen konz. Schwefelsäure und dann mit 90 ml Essigsäureanhydrid langsam versetzt. Die Lösung wird 1 Stunde auf dem Dampfbad erwärmt und auf 20° abgekühlt. Danach wird während 2 Stunden Chlorgas eingeleitet, wobei die Temperatur auf 40° steigt. Die Lösung wird dann auf 80° erwärmt und Chlorgas während 2 weiteren Stunden eingeleitet. Die Lösung wird auf 20° abgekühlt und mit 150 ml konz. Salzsäure versetzt. Unter Kühlen werden 90 ml 30%iges Wasserstoffperoxyd so zugetropft, daß die Temperatur 25° nicht übersteigt. Die Lösung wird während 1 Stunde auf 80° erwärmt, abgekühlt, und das Lösungsmittel unter vermindertem Druck eingedampft. Fraktionierte Kristallisation des Rückstandes liefert 2,6-Dichlor-4-fluoracetanilid, Smp. 186–187° (aus Äthanol).

19,2 g 2,6-Dichlor-4-fluor-acetanilid werden in 48%iger Bromwasserstoffsäure 90 Minuten zum Rückfluß erhitzt. Die Lösung wird dann abgekühlt, und der kristalline Niederschlag abfiltriert, wobei man 2,6-Dichlor-4-fluoranilinhydrobromid erhält, das direkt in die nächste Stufe eingesetzt wird.

1,3 g 2,6-Dichlor-4-fluoranilin-hydrobromid werden in 0,5 g Triäthylamin und 3 ml Ameisensäure gelöst und während 4 Stunden zum Rückfluß erhitzt. Die Lösung wird auf −10° abgekühlt, und der entstandene Niederschlag abfiltriert, mit Wasser gewaschen und getrocknet, wobei man 2,6-Dichlor-4-fluorformanilid erhält, Smp. 165–168°.

1,0 g 2,6-Dichlor-4-fluorformanilid werden portionsweise zu einem Gemisch von 1,82 ml Thionylchlorid und 0,40 ml Sulfurylchlorid bei 10° gegeben. Die Lösung wird 10 Stunden zum Rückfluß erhitzt, danach 6 Stunden bei Raumtemperatur stehengelassen, dann unter vermindertem Druck eingedampft, dann dreimal mit Benzol behandelt und wieder eingedampft. Der ölige Rückstand wird im Hochvakuum destilliert, wobei man (2,6-Dichlor-4-fluorphenyl)imidocarbonylchlorid erhält, Sdp. 65°/0,005 mm Hg.

Beispiel 48

In analoger Weise wie in Beispiel 2 beschrieben wurde die folgende Verbindung hergestellt:

— Aus 1-(Benzyloxy)-2-[(2,6-dichlor-4-fluorphenyl)imino]-imidazolidin und Bromwasserstoffsäure erhält man 2-[(2,6-Dichlor-4-fluorphenyl)imino]-1-hydroxyimidazolidin-hydrobromid, Smp. 230° unter Zersetzung (aus 48%iger Bromwasserstoffsäure).

Beispiel 49

In analoger Weise wie in Beispiel 40 beschrieben wurden die folgenden Verbindungen hergestellt:

— Aus 2-[(2,6-Dichlorphenyl)imino]-1-hydroxyimidazolidin und 3-Chlormethylpyridin erhält man 3-/[/2-[(2,6-Dichlorphenyl)imino]-1-imidazolidinyl/oxy]methyl/pyridin, Smp. 151—152° (aus Methylenchlorid/Isopropyläther); das entsprechende Dihydrochlorid schmilzt bei 222° unter Zersetzung (aus Acetonitril/Dioxan);
— aus 2-[(2,6-Dichlorphenyl)imino]-1-hydroxyimidazolidin und 4-Chlormethylpyridin erhält man 4-/[/2-[(2,6-Dichlorphenyl)imino]-1-imidazolidinyl/oxy]methyl/pyridin, Smp. 160—161° (aus Acetonitril/Isopropyläther); das entsprechende Dihydrochlorid schmilzt bei 180° unter Zersetzung (aus Methanol/Acetonitril);
— aus 2-[(2,3-Dichlorphenyl)imino]-1-hydroxyimidazolidin und 2-Chlormethylpyridin erhält man 2-/[/2-[(2,3-Dichlorphenyl)imino]-1-imidazolidinyl/oxy]methyl/pyridin-dihydrobromid, Smp. 154—155° unter Zersetzung (aus Methanol/Aceton);
— aus 2-[(2,4-Dichlorphenyl)imino]-1-hydroxyimidazolidin und 2-Chlormethylpyridin erhält man 2-/[/2-[(2,4-Dichlorphenyl)imino]-1-imidazolidinyl/oxy]methyl/pyridin-dihydrochlorid, Smp. 179—180° unter Zersetzung (aus Aceton);
— aus 2-[(2,5-Dichlorphenyl)imino]-1-hydroxyimidazolidin und 2-Chlormethylpyridin erhält man 2-/[/2-[(2,5-Dichlorphenyl)imino]-1-imidazolidinyl/oxy]methyl/pyridin als ein Öl;
— aus 2-[(2,6-Dichlorphenyl)imino]-1-hydroxyimidazolidin und 2-Chlormethyl-5-methylpyridin erhält man 2-/[/2-[(2,6-Dichlorphenyl)imino]-1-imidazolidinyl/oxy]-methyl/-5-methylpyridin, Smp. 147—148° (aus Methylenchlorid/Isopropyläther); das entsprechende Dihydrobromid schmilzt bei 163—164° unter Zersetzung (aus Acetonitril);
— aus 2-[(2,6-Dichlorphenyl)imino]-1-hydroxyimidazolidin und 2-Chlormethyl-6-methylpyridin erhält man 2-/[/2-[(2,6-Dichlorphenyl)imino]-1-imidazolidinyl/oxy]-methyl/-6-methylpyridin, Smp. 132—133° (aus Aceton/Isopropyläther); das entsprechende Dihydrochlorid schmilzt bei 190° unter Zersetzung (aus Aceton);
— aus 2-[(2,6-Dichlorphenyl)imino]-1-hydroxyimidazolidin und 2-Chlormethyl-5-äthylpyridin erhält man 2-/[/2-[(2,6-Dichlorphenyl)imino]-1-imidazolidinyl/oxy]-methyl/-5-äthylpyridin, Smp. 103—104° (aus Isopropyläther);
— aus 2-[(2,6-Dichlorphenyl)imino]-1-hydroxyimidazolidin und 2-α-Chloräthylpyridin erhält man rac-2-/1-[/2-[(2,6-Dichlorphenyl)imino]-1-imidazolidinyl/oxy]äthyl/pyridin-dihydrochlorid, Smp. 218—219° (aus Acetonitril/Aceton);
— aus 2-[(2,6-Dichlorphenyl)imino]-1-hydroxyimidazolidin und 5-Brommethylisoxazol erhält man 5-/[/2-[(2,6-Dichlorphenyl)imino]-1-imidazolidinyl/oxy]methyl/isoxazol, Smp. 87—88° (aus Isopropyläther); das entsprechende Hydrochlorid schmilzt bei 171° unter Zersetzung (aus Aceton);
— aus 2-[(2-Chlorphenyl)imino]-1-hydroxyimidazolidin und 2-Chlormethylpyridin erhält man 2-/[/2-[(2-Chlorphenyl)imino]-1-imidazolidinyl/oxy]methyl/pyridindihydrochlorid, Smp. 175—176° (aus Acetonitril/Dioxan);
— aus 2-[(2-Bromphenyl)imino-1-hydroxyimidazolidin und 2-Chlormethylpyridin erhält man 2-/[/2-[(2-Bromphenyl)imino]-1-imidazolidinyl/oxy]methyl/pyridin-dihydrobromid, Smp. 186—187° (aus 30% Bromwasserstoff in Eisessig);
— aus 2-[(2,6-Dibromphenyl)imino]-1-hydroxyimidazolidin und 2-Chlormethylpyridin erhält man 2-/[/2-[(2,6-Dibromphenyl)imino]-1-imidazolidinyl/oxy]methyl/pyridin-dihydrochlorid, Smp. 187—188° unter Zersetzung (aus Methanol/Acetonitril);
— aus 1-Hydroxy-2-[(2,6-dimethylphenyl)imino]imidazolidin und 2-Chlormethylpyridin erhält man 2-/[/2-[(2,6-Dimethylphenyl)imino]-1-imidazolidinyl/oxy]methyl/pyridin-dihydrochlorid, Smp. 196—197° unter Zersetzung (aus Acetonitril);
— aus 2-[(6-Chlor-2-methylphenyl)imino]-1-hydroxyimidazolidin und 2-Chlormethylpyridin erhält man 2-/[/2-[(6-Chlor-2-methylphenyl)imino]-1-imidazolidinyl/oxy]methyl/pyridin-dihydrochlorid, Smp. 187° (aus Acetonitril);
— aus 2-[(6-Chlor-2-methylphenyl)imino]-1-hydroxyimidazolidin und 3-Chlormethylpyridin erhält man 3-/[/2-[(6-Chlor-2-methyl)imino]-1-imidazolidinyl/oxy]methyl/pyridin, Smp. 110—111° (aus Methylenchlorid/Isopropyläther); das entsprechende Dihydrochlorid schmilzt bei 221—222° unter Zersetzung (aus Acetonitril);

- aus 2-[(2,6-Dichlor-4-fluorphenyl)imino]-1-hydroxyimidazolidin und 2-Chlormethylpyridin erhält man 2-/[/2-[(2,6-Dichlor-4-fluorphenyl)imino]-1-imidazolidinyl/oxy]methyl/pyridin-dihydrobromid, Smp. 199—200° unter Zersetzung (aus Methanol/Acetonitril);
- aus 2-[(2,6-Dichlorphenyl)imino]-1-hydroxyimidazolidin und 5-Chlormethyl-2,2,8-trimethyl-4H-m-dioxino-[4,5-c]-pyridin erhält man 5-/[/2-[(2,6-Dichlorphenyl)imino]-1-imidazolidinyl/oxy]methyl/-2,2,8-trimethyl-4H-m-dioxino[4,5-c]pyridin, Smp. 190—192° (aus Methanol/Acetonitril);
- aus 2-[(2,6-Dichlorphenyl)imino]-1-hydroxyimidazolidin und 4-α-Chloräthylpyridin erhält man 4-/1-[/2-[(2,6-Dichlorphenyl)imino]-1-imidazolidinyl/oxy]äthyl/pyridin-dihydrochlorid, Smp. >300° (aus Methanol/Acetonitril);
- aus 2-[(2,6-Dichlorphenyl)imino]-1-hydroxyimidazolidin und 5,6-Dimethoxy-3-chlormethylpyridin erhält man 5-/[/2-[(2,6-Dichlorphenyl)imino]-1-imidazolidinyl/oxy]methyl/-2,3-dimethoxypyridin-dihydrochlorid, Smp. 172—175° unter Zersetzung (aus Acetonitril/Dioxan);
- aus 2-[(2,6-Dichlorphenyl)imino]-1-hydroxyimidazolidin und 3-α-Chloräthylpyridin erhält man 3-/1-[/2-[(2,6-Dichlorphenyl)imino]-1-imidazolidinyl/oxy]äthyl/pyridin-dihydrochlorid, Smp. 262° unter Zersetzung (aus Acetonitril/Dioxan).

## Beispiel 50

9,84 g (~40 mMol) 2-[(2,6-Dichlorphenyl)imino]-1-hydroxyimidazolidin werden in 100 ml 40%igem Tetrabutylammoniumhydroxid gelöst und mit 10,16 g (55 mMol) γ-Chlorpropylbenzyläther versetzt. Die Lösung wird über Nacht stehengelassen, danach mit Wasser verdünnt und mit Äther extrahiert. Die Ätherextrakte werden mit 1 N Schwefelsäure geschüttelt. Die wässerige Phase wird mit Ammoniak alkalisch gestellt und mit Äther extrahiert. Die Ätherextrakte werden über Natriumsulfat getrocknet und eingedampft. Der ölige Rückstand wird aus Methylenchlorid/Isopropyläther umkristallisiert, wobei man 1-[3-(Benzyloxy)propoxy]-2-[(2,6-dichlorphenyl)imino]imidazolidin erhält, Smp. 79—80°.

Für die vorstehend beschriebene Verbindung und ihre Herstellung wird kein Schutz begehrt.

In analoger Weise wurde die folgende erfindungsgemäße Verbindung hergestellt:

- Aus 2-[(2,6-Dichlorphenyl)imino]-1-hydroxyimidazolidin und Cyclohexylbromid erhält man 1-(Cyclohexyloxy)-2-[(2,6-dichlorphenyl)imino]-imidazolidin-fumarat, Smp. 163—164° (aus Aceton).

## Beispiel 51

3,7 g 5-/[/2-[(2,6-Dichlorphenyl)imino]-1-imidazolidinyl/oxy]methyl/-2,2,8-trimethyl-4H-m-dioxino[4,5-c]pyridin werden in 50 ml Äthanol gelöst, mit 50 ml 3 N Salzsäure bei Raumtemperatur versetzt und zwei Tage stehengelassen. Die Lösung wird eingedampft und der Rückstand aus Methanol/Acetonitril umkristallisiert, wobei man 5-/[/2-[(2,6-Dichlorphenyl)imino]-1-imidazolidinyl/oxy]methyl/-3-hydroxy-2-methyl-4-pyridinmethanol-dihydrochlorid erhält, Smp. 216—218°.

## Beispiel A

Herstellung von Lacktabletten nachstehender Zusammensetzung:

| | |
|---|---|
| 2-[(2,6-Dichlorphenyl)imino]-1-(furfuryloxy)-imidazolidin-hydrochlorid | 5,56 mg |
| Milchzucker, pulv. | 34,44 mg |
| Maisstärke, weiß | 59,0 mg |
| Talk | 0,5 mg |
| Magnesiumstearat | 0,5 mg |
| Kerngewicht | 100,0 mg |
| Lacktrockensubstanz ca. | 7,0 mg |
| Lacktablettengewicht ca. | 107,0 mg |

Die Mischung des Wirkstoffes mit dem Milchzucker (pulv.) und einem Teil der Maisstärke wird mit einem Kleister aus einem weiteren Teil der Maisstärke und Wasser befeuchtet, geknetet, granuliert, getrocknet und gesiebt. Dieses Granulat wird mit der restlichen Maisstärke dem Talk und dem Magnesiumstearat gemischt und zu Kernen à 100 mg verpreßt. Die Kerne werden mittels einer der üblichen Methoden mit ca. 7,0 mg Lacktrockensubstanz lackiert.

Beispiel B

Herstellung von Lacktabletten nachstehender Zusammensetzung:

| | |
|---|---|
| Äthyl 2-[/2-[(2,6-dichlorphenyl)imino]-1-imidazolidinyl/oxy]butyrat-hydrochlorid | 110,1 mg |
| Milchzucker, pulv. | 104,9 mg |
| Maisstärke, weiß | 125,0 mg |
| Talk | 9,0 mg |
| Magnesiumstearat | 1,0 mg |
| Kerngewicht | 350,0 mg |
| Lacktrockensubstanz ca. | 20,0 mg |
| Lacktablettengewicht ca. | 370,0 mg |

Die Mischung des Wirkstoffes mit dem Milchzucker (pulv.) und einem Teil der Maisstärke wird mit einem Kleister aus einem weiteren Teil der Maisstärke und Wasser befeuchtet, geknetet, granuliert, getrocknet und gesiebt. Dieses Granulat wird mit der restlichen Maisstärke, dem Talk und dem Magnesiumstearat gemischt und zu Kernen à 350 mg verpreßt. Die Kerne werden mittels einer der üblichen Methoden mit ca. 20 mg Lacktrockensubstanz lackiert.

**Patentansprüche für die Vertragsstaaten: BE, CH, FR, LU, NL, SE**

1. 2-Imino-imidazolidin-Derivate der allgemeinen Formel

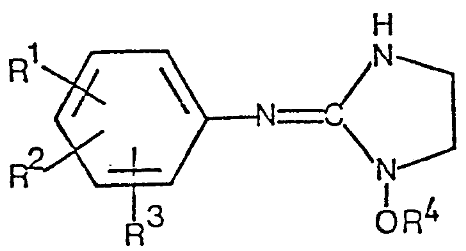

I

worin $R^1$, $R^2$ und $R^3$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy und $R^4$ Wasserstoff, $C_1-C_6$-Alkyl, $C_3-C_6$-Cycloalkyl, $C_2-C_6$-Alkenyl, $C_2-C_6$-Alkinyl, Di-$C_1-C_6$-alkylamino-$C_1-C_6$-alkyl, Carboxy-$C_1-C_6$-alkyl, Carboxy-$C_2-C_6$-alkenyl, Hydroxy-$C_1-C_6$-alkyl, Cyano-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, Mono-$C_1-C_6$-alkylaminocarbonyl-$C_1-C_6$-alkyl, Di-$C_1-C_6$-alkylaminocarbonyl-$C_1-C_6$-alkyl, $C_1-C_6$-Alkoxycarbonyl-$C_2-C_6$-alkenyl, $C_1-C_6$-Alkoxy-$C_1-C_6$-alkyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl, (2,2,8-Trimethyl)-4H-m-dioxino-[4,5-c]-pyridin-5-yl)methyl, [5-Hydroxy-4-(hydroxymethyl)-6-methyl-3-pyridyl]methyl, $\alpha$-Carboxybenzyl, $\alpha$-$C_1-C_6$-Alkoxycarbonyl-$\alpha$-$C_1-C_6$-alkylphenyl oder einen über eine $-CH(R^5)$-Gruppe gebundenen, gegebenenfalls durch $C_1-C_6$-Alkyl oder die Gruppe $-COOR$ substituierten Thienyl-, Furyl- oder Isoxazolylrest oder einen gegebenenfalls durch $C_1-C_6$-Alkyl, $C_1-C_6$-Alkoxy oder die Gruppe $-COOR$ substituierten Pyridylrest und $R^5$ Wasserstoff, Methyl, Äthyl oder n-Propyl und R Wasserstoff oder $C_1-C_6$-Alkyl bedeuten,
sowie pharmazeutisch anwendbare Säureadditionssalze davon.

2. Verbindungen gemäß Anspruch 1, worin $R^4$ Wasserstoff, $C_1-C_6$-Alkyl, $C_3-C_6$-Cycloalkyl, $C_2-C_6$-Alkenyl, $C_2-C_6$-Alkinyl, Di-$C_1-C_6$-alkylamino-$C_1-C_6$-alkyl, Carboxy-$C_1-C_6$-alkyl, Carboxy-$C_2-C_6$-alkenyl, Hydroxy-$C_1-C_6$-alkyl, Cyano-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, $C_1-C_6$-Alkoxycarbonyl-$C_2-C_6$-alkenyl, $C_1-C_6$-Alkoxy-$C_1-C_6$-alkyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl, Amino-$C_1-C_6$-alkyl, $\alpha$-Carboxybenzyl, $\alpha$-$C_1-C_6$-Alkoxycarbonyl-$\alpha$-$C_1-C_6$-alkylphenyl oder einen über eine $-CH(R^5)$-Gruppe gebundenen, gegebenenfalls durch $C_1-C_6$-Alkyl oder die Gruppe $-COOR$ substituierten Thienyl-, Furyl-, Isoxazolyl-oder Pyridylrest bedeutet und $R^1$, $R^2$, $R^3$, $R^5$ und R die in Anspruch 1 angegebene Bedeutung besitzen.

3. Verbindungen gemäß Anspruch 1 oder 2, worin $R^3$ Wasserstoff bedeutet.

4. Verbindungen gemäß einem der Ansprüche 1 bis 3, worin $R^1$ und $R^2$ in 2,6-Stellung des Phenylringes lokalisiert sind.

5. Verbindungen gemäß einem der Ansprüche 1 bis 4, worin $R^1$ und $R^2$ die gleiche Bedeutung haben.

6. Verbindungen gemäß einem der Ansprüche 1 bis 5, worin $R^1$ und $R^2$ Halogen bedeuten.

7. Verbindungen gemäß Anspruch 6, worin $R^1$ und $R^2$ Chlor bedeuten.

8. Verbindungen gemäß einem der Ansprüche 1 bis 7, worin $R^4$ $C_2-C_6$-Alkinyl, Carboxy-$C_1-C_6$-alkyl, $C_1-C_6$-Alkoxycarbonyl-$C_2-C_6$-alkenyl oder einen über eine $-CH(R^5)$-Gruppe gebundenen, gegebenenfalls durch $C_1-C_6$-Alkyl oder die Gruppe $-COOR$ substituierten Thienyl-, Furyl-, Isoxazolyl- oder Pyridylrest bedeutet und $R^5$ Wasserstoff, Methyl, Äthyl oder n-Propyl und R Wasserstoff oder $C_1-C_6$-Alkyl bedeuten.

9. Verbindungen gemäß Anspruch 8, worin $R^4$ Alkinyl mit 3–5 Kohlenstoffatomen, Carboxymethyl, Carboxypropyl, Äthoxycarbonyl-$C_2-C_6$-alkenyl oder einen über eine $-CH(R^5)$-Gruppe gebundenen Pyridyl- oder unsubstituierten Furyl- oder Thienylrest und $R^5$ Wasserstoff oder Methyl bedeuten.

10. Verbindungen gemäß Anspruch 9, worin $R^4$ Propargyl, Carboxymethyl, unverzweigtes Äthoxycarbonyl-$C_2-C_6$-alkenyl oder über eine $-CH(R^5)$-Gruppe gebundenes Pyridyl oder Furyl bedeutet.

11. Verbindungen gemäß einem der Ansprüche 1 bis 10, worin $R^3$ Wasserstoff, $R^1$ und $R^2$ Halogen, vorzugsweise Chlor, in 2,6-Stellung, $R^4$ Propargyl, Carboxymethyl, unverzweigtes Äthoxycarbonyl-$C_2-C_6$-alkenyl oder über eine $-CH(R^5)$-Gruppe gebundenes Pyridyl oder Furyl und $R^5$ Wasserstoff oder Methyl bedeuten.

12. 2-[(2,6-Dichlorphenyl)imino]-1-hydroxyimidazolidin.

13. 2-[(2,6-Dibromphenyl)imino]-1-hydroxyimidazolidin.

14. 1-Hydroxy-2-[(2-iodphenyl)imino]imidazolidin.

15. Äthyl 4-[/2-[(2,6-dichlorphenyl)imino]-1-imidazolidinyl/oxy]butyrat.

16. 4-[/2-[(2,6-Dichlorphenyl)imino]-1-imidazolidinyl/oxy]-buttersäure.

17. Äthyl 2-[/2-[(2,6-dichlorphenyl-imino]-1-imidazolidinyl/oxy]butyrat.

18. 2-[(2,6-Dichlorphenyl)imino]-1-(2-propinyloxy)imidazolidin.

19. [/2-[(2,6-Dichlorphenyl)imino]-1-imidazolidinyl/oxy]-essigsäure.

20. Äthyl 4-[/2-[(2,6-dichlorphenyl)imino]-1-imidazolidinyl/oxy]crotonat.

21. 2-[(2,6-Dichlorphenyl)imino]-1-äthoxyimidazolidin.

22. 1-(2-Butinyloxy)-2-[(2,6-dichlorphenyl)imino]imidazolidin.

23. 2-[(2,6-Dichlorphenyl)imino]-1-(furfuryloxy)imidazolidin.

24. 2-[/(2-[(2,6-Dichlorphenyl)imino]-1-imidazolinyl)oxy]methyl/pyridin.

25. 3-[/(2-[(2,6-Dichlorphenyl)imino]-1-imidazolinyl)oxy]methyl/pyridin.

26. 2-[/(2-[(2,6-Dichlor-4-fluorphenyl)imino]-1-imidazolinyl)oxy]methyl/pyridin.

27. 5-[/(2-[(2,6-Dichlorphenyl)imino]-1-imidazolinyl)oxy]methyl/-3-hydroxy-2-methyl-4-pyridinmethanol.

28. Salze der Verbindungen gemäß Ansprüche 12–15, 17, 18 und 20–27.

29. Verbindungen der allgemeinen Formel

$$H_2N-CH_2-CH_2-NH-OR^4 \qquad IV$$

worin $R^4$ Wasserstoff, $C_1-C_6$-Alkyl, $C_3-C_6$-Cycloalkyl, $C_2-C_6$-Alkenyl, $C_2-C_6$-Alkinyl, Di-$C_1-C_6$-alkylamino-$C_1-C_6$-alkyl, Carboxy-$C_1-C_6$-alkyl, Carboxy-$C_2-C_6$-alkenyl, Hydroxy-$C_1-C_6$-alkyl, Cyano-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, Mono-$C_1-C_6$-alkylaminocarbonyl-$C_1-C_6$-alkyl, Di-$C_1-C_6$-alkylaminocarbonyl-$C_1-C_6$-alkyl, $C_1-C_6$-Alkoxycarbonyl-$C_2-C_6$-alkenyl, $C_1-C_6$-Alkoxy-$C_1-C_6$-alkyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl, (2,2,8-Trimethyl-4H-m-dioxino[4,5-c]pyridin-5-yl)methyl, [5-Hydroxy-4-(hydroxymethyl)-6-methyl-3-pyridyl]methyl, $\alpha$-Carboxybenzyl, $\alpha$-$C_1-C_6$-Alkoxycarbonyl-$\alpha$-$C_1-C_6$-alkylphenyl oder einen über eine $-CH(R^5)$-Gruppe gebundenen, gegebenenfalls durch $C_1-C_6$-Alkyl oder die Gruppe $-COOR$ substituierten Thienyl-, Furyl- oder Isoxazolylrest oder einen gegebenenfalls durch $C_1-C_6$-Alkyl, $C_1-C_6$-Alkoxy oder die Gruppe $-COOR$ substituierten Pyridylrest und $R^5$ Wasserstoff, Methyl, Äthyl oder n-Propyl und R Wasserstoff oder $C_1-C_6$-Alkyl bedeuten, wenn sie zur Herstellung von Verbindungen nach Anspruch 1 verwendet werden.

30. Verbindungen der allgemeinen Formel

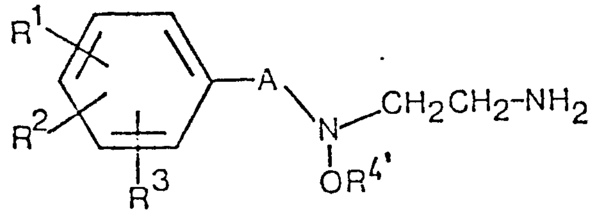

V

worin A die Gruppe

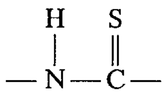

oder

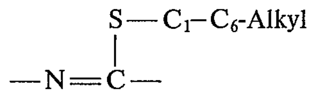

$R^1$, $R^2$ und $R^3$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy und $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest und $R^5$ Wasserstoff, Methyl, Äthyl oder n-Propyl bedeuten,
wenn sie zur Herstellung von Verbindungen nach Anspruch 1 verwendet werden.

31. Verbindungen der allgemeinen Formel

$$Z-CH_2CH_2-HN-OR^{4'} \qquad XII$$

worin $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest und $R^5$ Wasserstoff, Methyl, Äthyl oder n-Propyl und Z eine Phthalimido- oder Succinimidogruppe bedeuten,
wenn sie zur Herstellung von Verbindungen nach Anspruch 1 verwendet werden.

32. Verbindungen der allgemeinen Formel

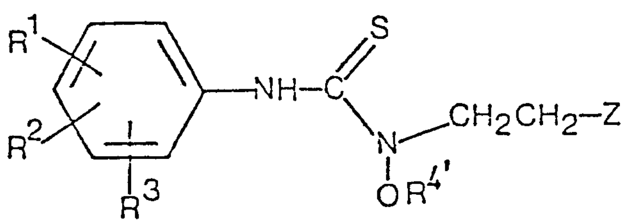

XIII

worin $R^1$, $R^2$ und $R^3$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy und $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest und $R^5$ Wasserstoff, Methyl, Äthyl oder n-Propyl und Z eine Phthalimido- oder Succinimidogruppe bedeuten,
wenn sie zur Herstellung von Verbindungen nach Anspruch 1 verwendet werden.

33. Verbindungen der allgemeinen Formel

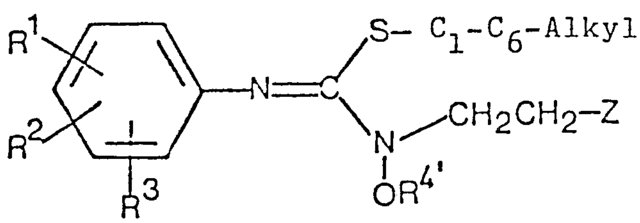

XIV

worin $R^1$, $R^2$ und $R^3$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy und $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest und $R^5$ Wasserstoff, Methyl, Äthyl oder n-Propyl und Z eine Phthalimido- oder Succinimidogruppe bedeuten,
wenn sie zur Herstellung von Verbindungen nach Anspruch 1 verwendet werden.

34. Verfahren zur Herstellung von 2-Imino-imidazolidin-Derivaten der in Anspruch 1 definierten allgemeinen Formel I und ihren pharmazeutisch anwendbaren Säureadditionssalzen, dadurch gekennzeichnet, daß man

a) eine Verbindung der allgemeinen Formel

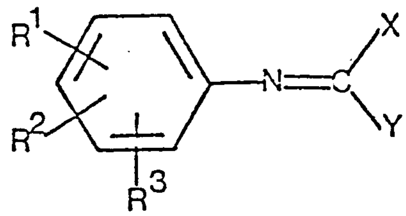

II

worin X und Y je Halogen, eine Sulfhydryl-, Amino-, Methoxy- oder Alkylthiogruppe mit 1–3 Kohlenstoffatomen bedeuten und $R^1$, $R^2$ und $R^3$ die in Anspruch 1 angegebene Bedeutung besitzen,
oder eine Verbindung der allgemeinen Formel

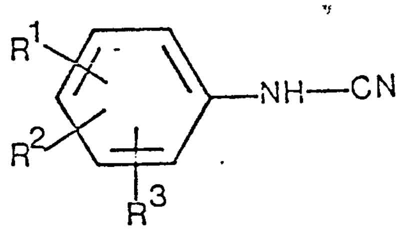

III

worin $R^1$, $R^2$ und $R^3$ die in Anspruch 1 angegebene Bedeutung besitzen, mit einer Verbindung der allgemeinen Formel

$$H_2N-CH_2-CH_2-NH-OR^4 \qquad IV$$

worin $R^4$ die in Anspruch 1 angegebene Bedeutung besitzt,
umsetzt, oder

b) zur Herstellung von Verbindungen der Formel I, worin $R^4$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl,$C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest bedeutet und $R^1$, $R^2$, $R^3$ und $R^5$ die in Anspruch 1 angegebene Bedeutung besitzen, eine Verbindung der allgemeinen Formel

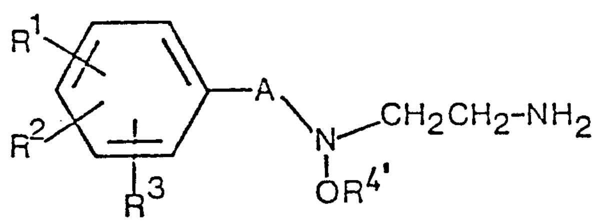

V

worin A die Gruppe

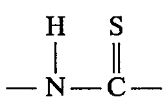

oder

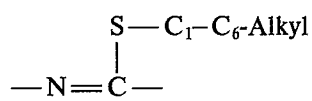

und $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl $-C_1-C_6$-alkyl, $C_1-C_6$-Alkylthio-$C_1-C_6$-alkyl, $C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest bedeutet und $R^1$, $R^2$, $R^3$ und $R^5$ die in Anspruch 1 angegebene Bedeutung besitzen,
cyclisiert, oder

c) zur Herstellung von Verbindungen der Formel I, worin $R^4$ Wasserstoff bedeutet und $R^1$, $R^{2'}$ und $R^3$ die in Anspruch 1 angegebene Bedeutung besitzen, in einer Verbindung der allgemeinen Formel

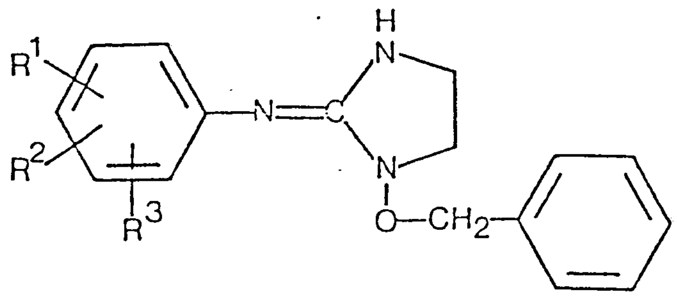

Ia

worin $R^1$, $R^2$ und $R^3$ die in Anspruch 1 angegebene Bedeutung besitzen, die Benzylgruppe abspaltet, oder

d) zur Herstellung von Verbindungen der allgemeinen Formel

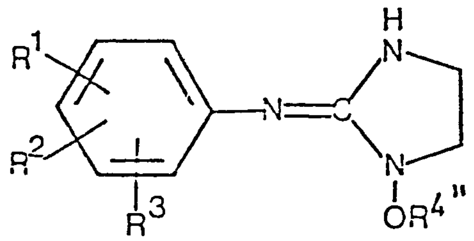

Ib

worin $R^{4''}$ $C_1-C_6$-Alkyl, $C_3-C_6$-Cycloalkyl, $C_2-C_6$-Alkenyl, $C_2-C_6$-Alkinyl, Di-$C_1-C_6$-alkylamino-$C_1-C_6$-alkyl, Carboxy-$C_1-C_6$-alkyl, Hydroxy-$C_1-C_6$-alkyl, Cyano-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, Mono-$C_1-C_6$-alkylaminocarbonyl-$C_1-C_6$-alkyl,Di-$C_1-C_6$-alkylaminocarbonyl-$C_1-C_6$-alkyl,$C_1-C_6$-Alkoxycarbonyl-$C_2-C_6$-alkenyl, $C_1-C_6$-Alkoxy-$C_1-C_6$-alkyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl oder einen über eine $-CH(R^5)$-Gruppe gebundenen, gegebenenfalls durch $C_1-C_6$-Alkyl oder die Gruppe $-COOR$ substituierten Thienyl-, Furyl-, Isoxazolyl- oder Pyridylrest bedeutet und R, $R^1$, $R^2$, $R^3$ und $R^5$ die in Anspruch 1 angegebene Bedeutung besitzen,
eine Verbindung der allgemeinen Formel

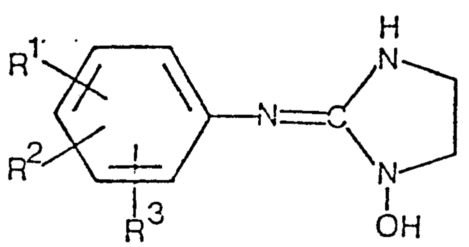

Ic

worin $R^1$, $R^2$ und $R^3$ die in Anspruch 1 angegebene Bedeutung besitzen, mit einer Verbindung der allgemeinen Formel

$$R^{4''}-X \qquad VI$$

worin X eine austretende Gruppe bedeutet und $R^{4''}$ die obige Bedeutung besitzt,
umsetzt, oder

e) zur Herstellung von Verbindungen der Formel I, worin $R^4$ Carboxy-$C_1-C_6$-alkyl oder Carboxy-$C_2-C_6$-alkenyl bedeutet und $R^1$, $R^2$ und $R^3$ die in Anspruch 1 angegebene Bedeutung besitzen, eine entsprechende Verbindung der Formel I, worin $R^4$ $C_1-C_6$-Alkoxycarbonyl-$C_1-C_6$-alkyl oder $C_1-C_6$-Alkoxycarbonyl-$C_2-C_6$-alkenyl bedeutet und $R^1$, $R^2$ und $R^3$ die in Anspruch 1 angegebene Bedeutung besitzen, hydrolysiert, oder

f) zur Herstellung von Verbindungen der Formel I, worin $R^4$ $C_1-C_6$-Alkoxycarbonyl-$C_1-C_6$-alkyl oder $C_1-C_6$-Alkoxycarbonyl-$C_2-C_6$-alkenyl bedeutet und $R^1$, $R^2$ und $R^3$ die in Anspruch 1 angegebene Bedeutung besitzen, eine entsprechende Verbindung der Formel I, worin $R^4$ $C_1-C_6$-Alkoxycarbonyl-$C_1-C_6$-alkyl oder $C_1-C_6$-Alkoxycarbonyl-$C_2-C_6$-alkenyl bedeutet, wobei jedoch der Alkoxyrest verschieden ist, und $R^1$, $R^2$ und $R^3$ die in Anspruch 1 angegebene Bedeutung besitzen, mit einem $C_1-C_6$-Alkanol umestert, oder

g) zur Herstellung von Verbindungen der obigen Formel I, worin $R^4$ Aminocarbonyl-$C_1-C_6$-alkyl bedeutet und $R^1$, $R^2$ und $R^3$ die in Anspruch 1 angegebene Bedeutung besitzen, eine

entsprechende Verbindung der Formel I, worin $R^4$ $C_1-C_6$-Alkoxycarbonyl-$C_1-C_6$-alkyl bedeutet und $R^1$, $R^2$ und $R^3$ die in Anspruch 1 angegebene Bedeutung besitzen, mit Ammoniak, primärem Amin oder sekundärem Amin umsetzt, oder

h) zur Herstellung von Verbindungen der Formel I, worin $R^4$ Wasserstoff bedeutet und $R^1$, $R^2$ und $R^3$ die in Anspruch 1 angegebene Bedeutung besitzen, eine Verbindung der allgemeinen Formel

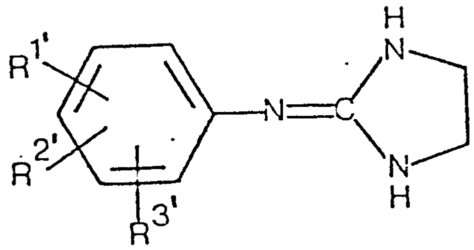

VII

worin $R^{1'}$, $R^{2'}$ und $R^{3'}$ unabhängig voneinander je Wasserstoff, $C_1-C_6$-Alkyl, $C_1-C_6$-Alkoxy, Halogen oder Trifluormethyl bedeuten,
mit Kaliumpersulfat in konz. Schwefelsäure oxydiert, oder

i) zur Herstellung von Verbindungen der obigen Formel I, worin $R^4$ [5-Hydroxy-4-(hydroxymethyl)-6-methyl-3-pyridyl]methyl bedeutet und $R^1$, $R^2$ und $R^3$ die in Anspruch 1 angegebene Bedeutung besitzen, in einer Verbindung der allgemeinen Formel

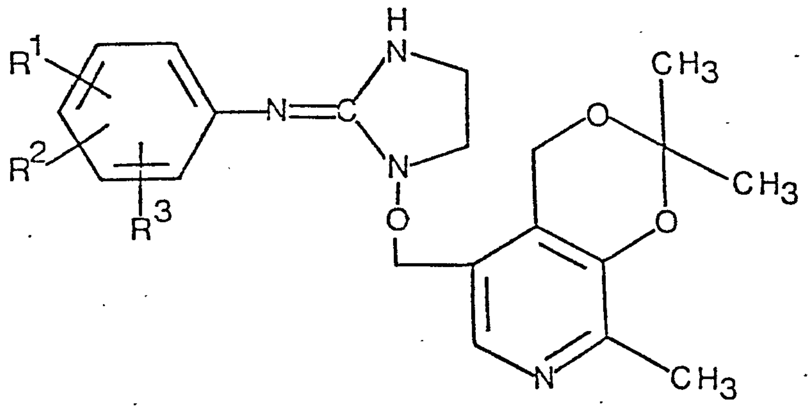

Id

worin $R^1$, $R^2$ und $R^3$ die in Anspruch 1 angegebene Bedeutung besitzen,
die Ketalgruppe abspaltet, und

j) erwünschtenfalls eine erhaltene Verbindung oder ein pharmazeutisch nicht anwendbares Säureadditionssalz davon in ein pharmazeutisch anwendbares Säureadditionssalz davon überführt.

35. Verfahren nach Anspruch 34 zur Herstellung von den in Anspruch 2 definierten Verbindungen der Formel I oder von deren pharmazeutisch anwendbaren Säureadditionssalzen, dadurch gekennzeichnet, daß man Verfahrensvarianten a), b), c), d), e), f), g) und j) von Anspruch 34 durchführt.

36. Verfahren zur Herstellung von Präparaten mit blutdrucksenkenden Eigenschaften, dadurch gekennzeichnet, daß man ein 2-Imino-imidazolidin-Derivat der Formel I in Anspruch 1 oder ein pharmazeutisch anwendbares Säureadditionssalz davon als wirksamen Bestandteil mit zur therapeutischen Verabreichung geeigneten, nicht-toxischen, inerten, an sich in solchen Präparaten üblichen festen und flüssigen Trägern und/oder Excipientien vermischt.

37. Präparate mit blutdrucksenkenden Eigenschaften enthaltend ein 2-Imino-imidazolidin-Derivat der Formel I in Anspruch 1 oder ein pharmazeutisch anwendbares Säureadditionssalz davon und einen Trägerstoff.

**Patentansprüche für den Vertragsstaat: DE**

1. 2-Imino-imidazolidin-Derivate der allgemeinen Formel

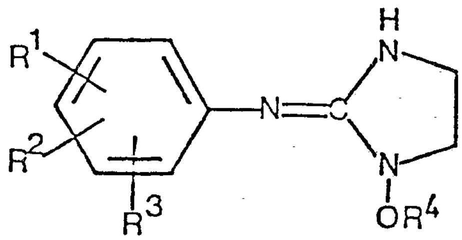

I

worin $R^1$, $R^2$ und $R^3$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy und $R^4$ Wasserstoff, $C_1-C_6$-Alkyl, $C_3-C_6$-Cycloalkyl, $C_1-C_6$-Alkenyl, $C_2-C_6$-Alkinyl, Di-$C_1-C_6$-alkylamino-$C_1-C_6$-alkyl, Carboxy-$C_1-C_6$-alkyl, Carboxy-$C_2-C_6$-alkenyl, Hydroxy-$C_1-C_6$-alkyl, Cyano-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, Mono-$C_1-C_6$-alkylaminocarbonyl-$C_1-C_6$-alkyl,Di-$C_1-C_6$-alkylaminocarbonyl-$C_1-C_6$-alkyl,$C_1-C_6$-Alkoxycarbonyl-$C_2-C_6$-alkenyl, $C_1-C_6$-Alkoxy-$C_1-C_6$-alkyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl, (2,2,8-Trimethyl)-4H-m-dioxino[4,5-c]-pyridin-5-yl)methyl, [5-Hydroxy-4-(hydroxymethyl)-6-methyl-3-pyridyl]methyl, $\alpha$-Carboxybenzyl, $\alpha$-$C_1-C_6$-Alkoxycarbonyl-$\alpha$-$C_1-C_6$-alkylphenyl oder einen über eine $-CH(R^5)$-Gruppe gebundenen, gegebenenfalls durch $C_1-C_6$-Alkyl oder die Gruppe $-COOR$ substituierten Thienyl-, Furyl- oder Isoxazolylrest oder einen gegebenenfalls durch $C_1-C_6$-Alkyl, $C_1-C_6$-Alkoxy oder die Gruppe $-COOR$ substituierten Pyridylrest und $R^5$ Wasserstoff, Methyl, Äthyl oder n-Propyl und R Wasserstoff oder $C_1-C_6$-Alkyl bedeuten, wobei $R^4$ von Wasserstoff, $C_1-C_6$-Alkyl, Hydroxy-$C_1-C_6$-alkyl, Benzyl, Phenäthyl und $C_1-C_6$-Alkoxy-$C_1-C_6$-alkyl verschieden ist, falls $R^1$, $R^2$ und $R^3$ unabhängig voneinander Wasserstoff, Methyl oder Halogen bedeuten,
sowie pharmazeutisch anwendbare Säureadditionssalze davon.

2. Verbindungen gemäß Anspruch 1, worin $R^4$ Wasserstoff, $C_1-C_6$-Alkyl, $C_3-C_6$-Cycloalkyl, $C_2-C_6$-Alkenyl, $C_2-C_6$-Alkinyl, Di-$C_1-C_6$-alkylamino-$C_1-C_6$-alkyl, Carboxy-$C_1-C_6$-alkyl, Carboxy-$C_2-C_6$-alkenyl, Hydroxy-$C_1-C_6$-alkyl, Cyano-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl,$C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, $C_1-C_6$-Alkoxycarbonyl-$C_2-C_6$-alkenyl, $C_1-C_6$-Alkoxy-$C_1-C_6$-alkyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl, Amino-$C_1-C_6$-alkyl, $\alpha$-Carboxybenzyl, $\alpha$-$C_1-C_6$-Alkoxycarbonyl-$\alpha$-$C_1-C_6$-alkylphenyl oder einen über eine $-CH(R^5)$-Gruppe gebundenen, gegebenenfalls durch $C_1-C_6$-Alkyl oder die Gruppe $-COOR$ substituierten Thienyl-, Furyl-, Isoxazolyl- oder Pyridylrest bedeutet und $R^1$, $R^2$, $R^3$, $R^5$ und R die in Anspruch 1 angegebene Bedeutung besitzen.

3. Verbindungen gemäß Anspruch 1 oder 2, worin $R^3$ Wasserstoff bedeutet.

4. Verbindungen gemäß einem der Ansprüche 1 bis 3, worin $R^1$ und $R^2$ in 2,6-Stellung des Phenylringes lokalisiert sind.

5. Verbindungen gemäß einem der Ansprüche 1 bis 4, worin $R^1$ und $R^2$ die gleiche Bedeutung haben.

6. Verbindungen gemäß einem der Ansprüche 1 bis 5, worin $R^1$ und $R^2$ Halogen bedeuten.

7. Verbindungen gemäß Anspruch 6, worin $R^1$ und $R^2$ Chlor bedeuten.

8. Verbindungen gemäß einem der Ansprüche 1 bis 7, worin $R^4$ $C_2-C_6$-Alkinyl, Carboxy-$C_1-C_6$-alkyl, $C_1-C_6$-Alkoxycarbonyl-$C_2-C_6$-alkenyl oder einen über eine $-CH(R^5)$-Gruppe gebundenen, gegebenenfalls durch $C_1-C_6$-Alkyl oder die Gruppe $-COOR$ substituierten Thienyl-, Furyl-, Isoxazolyl- oder Pyridylrest bedeutet und $R^5$ Wasserstoff, Methyl, Äthyl oder n-Propyl und R Wasserstoff oder $C_1-C_6$-Alkyl bedeuten.

9. Verbindungen gemäß Anspruch 8, worin $R^4$ Alkinyl mit 3–5 Kohlenstoffatomen, Carboxymethyl, Carboxypropyl, Äthoxycarbonyl-$C_2-C_6$-alkenyl oder einen über eine $-CH(R^5)$-Gruppe gebundenen Pyridyl- oder unsubstituierten Furyl- oder Thienylrest und $R^5$ Wasserstoff oder Methyl bedeuten.

10. Verbindungen gemäß Anspruch 9, worin $R^4$ Propargyl, Carboxymethyl, unverzweigtes Äthoxycarbonyl-$C_2-C_6$-alkenyl oder über eine $-CH(R^5)$-Gruppe gebundenes Pyridyl oder Furyl bedeutet.

11. Verbindungen gemäß einem der Ansprüche 1 bis 10, worin $R^3$ Wasserstoff, $R^{1'}$ und $R^{2'}$ Halogen, vorzugsweise Chlor, in 2,6-Stellung, $R^4$ Propargyl, Carboxymethyl, unverzweigtes Äthoxycarbonyl-$C_2-C_6$-alkenyl oder über eine $-CH(R^5)$-Gruppe gebundenes Pyridyl oder Furyl und $R^5$ Wasserstoff oder Methyl bedeuten.

12. Äthyl 4-[/2-[(2,6-dichlorphenyl)imino]-1-imidazolidinyl/oxy]butyrat.

13. 4-[/2-[(2,6-Dichlorphenyl)imino]-1-imidazolidinyl/oxy]buttersäure.

14. Äthyl 2-[/2-[(2,6-dichlorphenyl)imino]-1-imidazolidinyl/oxy]butyrat.
15. 2-[(2,6-Dichlorphenyl)imino]-1-(2-propinyloxy)imidazolidin.
16. [/2-[(2,6-Dichlorphenyl)imino]-1-imidazolidinyl/oxy]essigsäure.
17. Äthyl 4-[/2-[(2,6-dichlorphenyl)imino]-1-imidazolidinyl/oxy]crotonat.
18. 1-(2-Butinyloxy)-2-[(2,6-dichlorphenyl)imino]imidazolidin.
19. 2-[(2,6-Dichlorphenyl)imino]-1-(furfuryloxy)imidazolidin.
20. 2-[/(2-[(2,6-Dichlorphenyl)imino]-1-imidazolidinyl)oxy]methyl/pyridin.
21. 3-[/(2-[(2,6-Dichlorphenyl)imino]-1-imidazolidinyl)oxy]methyl/pyridin.
22. 2-[/(2-[(2,6-Dichlor-4-fluorphenyl)imino]-1-imidazolidinyl)oxy]methyl/pyridin.
23. 5-[/(2-[(2,6-Dichlorphenyl)imino]-1-imidazolidinyl)oxy]methyl/-3-hydroxy-2-methyl-4-pyridinmethanol.
24. Salze der Verbindungen gemäß Ansprüche 12, 14, 15 und 17 bis 23.
25. Verbindungen der allgemeinen Formel

$$H_2N-CH_2-CH_2-NH-OR^4 \qquad IV$$

worin $R^4$ Wasserstoff, $C_3-C_6$-Cycloalkyl, $C_2-C_6$-Alkenyl, $C_2-C_6$-Alkinyl, Di-$C_1-C_6$-alkylamino-$C_1-C_6$-alkyl, Carboxy-$C_1-C_6$-alkyl, Carboxy-$C_2-C_6$-alkenyl, Cyano-$C_1-C_6$-alkyl, Phenyl-$C_3-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, Mono-$C_1-C_6$-alkylaminocarbonyl-$C_1-C_6$-alkyl, Di-$C_1-C_6$-alkylaminocarbonyl-$C_1-C_6$-alkyl, $C_1-C_6$-Alkoxycarbonyl-$C_2-C_6$-alkenyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl, (2,2,8-Trimethyl-4H-m-dioxino[4,5-c]pyridin-5-yl)methyl, [5-Hydroxy-4-(hydroxymethyl)-6-methyl-3-pyridyl]methyl, α-Carboxybenzyl, α-$C_1-C_6$-Alkoxycarbonyl-α-$C_1-C_6$-alkylphenyl oder einen über eine $-CH(R^5)$-Gruppe gebundenen, gegebenenfalls durch $C_1-C_6$-Alkyl oder die Gruppe $-COOR$ substituierten Thienyl-, Furyl- oder Isoxazolylrest oder einen gegebenenfalls durch $C_1-C_6$-Alkyl, $C_1-C_6$-Alkoxy oder die Gruppe $-COOR$ substituierten Pyridylrest und $R^5$ Wasserstoff, Methyl, Äthyl oder n-Propyl und R Wasserstoff oder $C_1-C_6$-Alkyl bedeuten, wenn sie zur Herstellung von Verbindungen nach Anspruch 1 verwendet werden.

26. Verbindungen der allgemeinen Formel

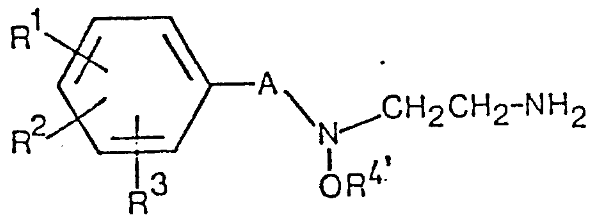

V

worin A die Gruppe

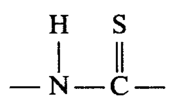

oder

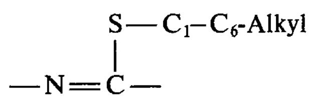

$R^1$, $R^2$ und $R^3$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy und $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxy-$C_1-C_6$ $C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest und $R^5$ Wasserstoff, Methyl, Äthyl oder n-Propyl bedeuten, wobei $R^{4'}$ von $C_1-C_6$-Alkyl, Benzyl, Phenäthyl und $C_1-C_6$-Alkoxy-$C_1-C_6$-alkyl verschieden ist, falls $R^1$, $R^2$ und $R^3$ unabhängig voneinander Wasserstoff, Methyl oder Halogen bedeuten,
wenn sie zur Herstellung von Verbindungen nach Anspruch 1 verwendet werden.

27. Verbindungen der allgemeinen Formel

$$Z-CH_2CH_2-HN-OR^{4'} \qquad XII$$

worin $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest und $R^5$ Wasserstoff, Methyl, Äthyl oder n-Propyl und Z eine Phthalimido- oder Succinimidogruppe bedeuten,

wenn sie zur Herstellung von Verbindungen nach Anspruch 1 verwendet werden.

28. Verbindungen der allgemeinen Formel

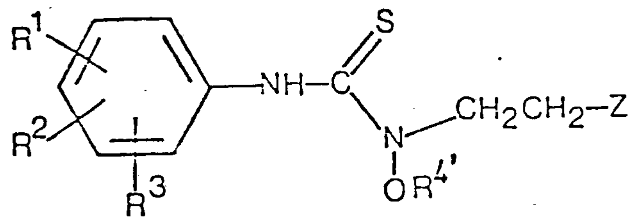

XIII

worin $R^1$, $R^2$ und $R^3$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy und $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl,$C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl oder Pyridylrest und $R^5$ Wasserstoff, Methyl, Äthyl oder n-Propyl und Z eine Phthalimido- oder Succinimidogruppe bedeuten, wobei $R^{4'}$ von $C_1-C_6$-Alkyl, Benzyl, Phenäthyl und $C_1-C_6$-Alkoxy-$C_1-C_6$-alkyl verschieden ist, falls $R^1$, $R^2$ und $R^3$ unabhängig voneinander Wasserstoff, Methyl oder Halogen bedeuten,
wenn sie zur Herstellung von Verbindungen nach Anspruch 1 verwendet werden.

29. Verbindungen der allgemeinen Formel

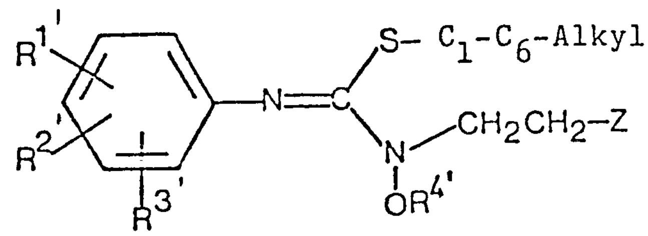

XIV'

worin $R^1$, $R^2$ und $R^3$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy und $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl,$C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest und $R^5$ Wasserstoff, Methyl, Äthyl oder n-Propyl und Z eine Phthalimido- oder Succinimidogruppe bedeuten, wobei $R^{4'}$ von $C_1-C_6$-Alkyl, Benzyl, Phenäthyl und $C_1-C_6$-Alkoxy-$C_1-C_6$-alkyl verschieden ist, falls $R^1$, $R^2$ und $R^3$ unabhängig voneinander Wasserstoff, Methyl oder Halogen bedeuten,
wenn sie zur Herstellung von Verbindungen nach Anspruch 1 verwendet werden.

30. Verfahren zur Herstellung von 2-Imino-imidazolidin-Derivaten der in Anspruch 1 definierten allgemeinen Formel I und ihren pharmazeutisch anwendbaren Säureadditionssalzen, dadurch gekennzeichnet, daß man

a) eine Verbindung der allgemeinen Formel

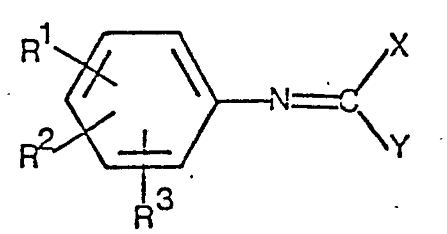

II

worin X und Y je Halogen, eine Sulfhydryl-, Amino-, Methoxy- oder Alkylthiogruppe mit 1–3 Kohlenstoffatomen bedeuten und $R^1$, $R^2$ und $R^3$ die in Anspruch 1 angegebene Bedeutung besitzen,
oder eine Verbindung der allgemeinen Formel

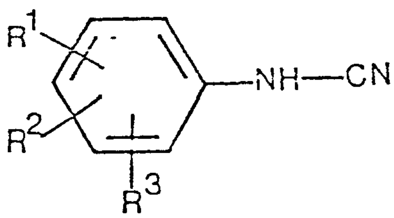

III

worin $R^1$, $R^2$ und $R^3$ die in Anspruch 1 angegebene Bedeutung besitzen, mit einer Verbindung der allgemeinen Formel

$$H_2N-CH_2-CH_2-NH-OR^4 \qquad IV$$

worin $R^4$ die in Anspruch 1 angegebene Bedeutung besitzt, umsetzt, oder

b) zur Herstellung von Verbindungen der Formel I, worin $R^4$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl,$C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest bedeutet und $R^1$, $R^2$, $R^3$ und $R^5$ die in Anspruch 1 angegebene Bedeutung besitzen, eine Verbindung der allgemeinen Formel

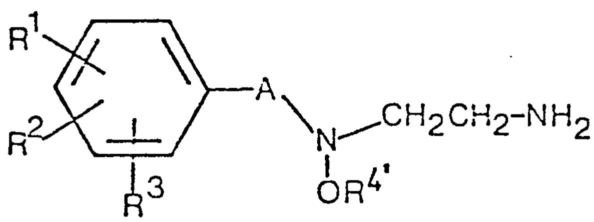

V

worin A die Gruppe

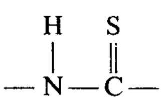

oder

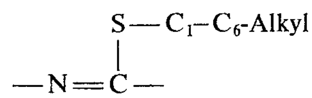

und $R^{4'}$ $C_1-C_6$-Alkyl, $C_3-C_6$-Cycloalkyl, $C_2-C_6$-Alkenyl, Di-$C_1-C_6$-alkylamino-$C_1-C_6$-alkyl, Phenyl-$C_1-C_6$-alkyl, im Phenylkern durch Methoxygruppen substituiertes Phenyl $-C_1-C_6$-alkyl, $C_1-C_6$-Alkylthio-$C_1-C_6$-alkyl, $C_1-C_6$-Alkoxy-$C_1-C_6$-alkoxy-$C_1-C_6$-alkyl, $C_1-C_6$-Alkoxy-$C_1-C_6$-alkyl oder einen über eine $-CH(R^5)$-Gruppe gebundenen, gegebenenfalls durch $C_1-C_6$-Alkyl substituierten Thienyl-, Furyl-, Isoxazolyl- oder Pyridylrest bedeutet und $R^1$, $R^2$, $R^3$ und $R^5$ die in Anspruch 1 angegebene Bedeutung besitzen, cyclisiert, oder

c) zur Herstellung von Verbindungen der Formel I, worin $R^4$ Wasserstoff bedeutet und $R^1$, $R^2$ und $R^3$ die in Anspruch 1 angegebene Bedeutung besitzen, in einer Verbindung der allgemeinen Formel

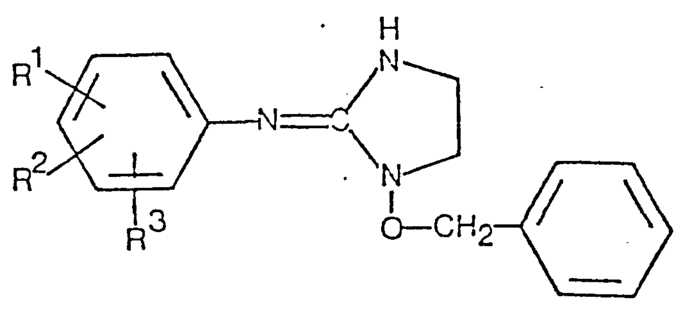

Ia

worin $R^1$, $R^2$ und $R^3$ die in Anspruch 1 angegebene Bedeutung besitzen, die Benzylgruppe abspaltet, oder

d) zur Herstellung von Verbindungen der allgemeinen Formel

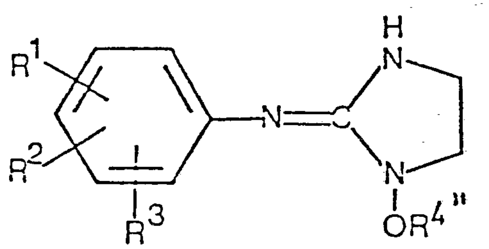

Ib

worin $R^{4''}$ $C_1-C_6$-Alkyl, $C_3-C_6$-Cycloalkyl, $C_2-C_6$-Alkenyl, $C_2-C_6$-Alkinyl, Di-$C_1-C_6$-alkylamino-$C_1-C_6$-alkyl, Carboxy-$C_1-C_6$-alkyl, Hydroxy-$C_1-C_6$-alkyl, Cyano-$C_1-C_6$-alkyl, Phenyl-$C_1-C_6$-alkyl, im Phenylkern durch Methoxygruppen substituiertes Phenyl-$C_1-C_6$-alkyl, $C_1-C_6$-Alkylthio-$C_1-C_6$-alkyl, $C_1-C_6$-Alkoxycarbonyl-$C_1-C_6$-alkyl, $C_1-C_6$-Alkoxy-$C_1-C_6$-alkoxy-$C_1-C_6$-alkyl, Aminocarbonyl-$C_1-C_6$-alkyl, Mono-$C_1-C_6$-alkylaminocarbonyl-$C_1-C_6$-alkyl,Di-$C_1-C_6$-alkylaminocarbonyl-$C_1-C_6$-alkyl,$C_1-C_6$-Alkoxycarbonyl-$C_2-C_6$-alkenyl, $C_1-C_6$-Alkoxy-$C_1-C_6$-alkyl, Amino-$C_1-C_6$-alkyl, Mono-$C_1-C_6$-alkylamino-$C_1-C_6$-alkyl, Di-$C_1-C_6$-alkylamino-$C_1-C_6$-alkyl oder einen über eine $-CH(R^5)$-Gruppe gebundenen, gegebenenfalls durch $C_1-C_6$-Alkyl oder die Gruppe $-COOR$ substituierten Thienyl-, Furyl-, Isoxazolyl- oder Pyridylrest bedeutet und R, $R^1$, $R^2$, $R^3$ und $R^5$ die in Anspruch 1 angegebene Bedeutung besitzen,
eine Verbindung der allgemeinen Formel

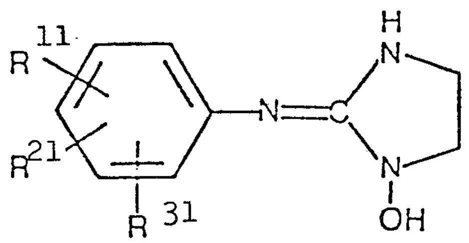

Ic

worin $R^{11}$, $R^{21}$ und $R^{31}$ unabhängig voneinander je Wasserstoff, $C_1-C_6$-Alkyl, $C_1-C_6$-Alkoxy, $C_1-C_6$-Alkylthio, Halogen, Trifluormethyl, Cyano oder Hydroxy bedeuten, mit einer Verbindung der allgemeinen Formel

$$R^{4''}-X \qquad \text{VI}$$

worin X eine austretende Gruppe bedeutet und $R^{4''}$ die obige Bedeutung besitzt,
umsetzt, oder

e) zur Herstellung von Verbindungen der Formel I, worin $R^4$ Carboxy-$C_1-C_6$-alkyl oder Carboxy-$C_2-C_6$-alkenyl bedeutet und $R^1$, $R^2$ und $R^3$ die in Anspruch 1 angegebene Bedeutung besitzen, eine entsprechende Verbindung der Formel I, worin $R^4$ $C_1-C_6$-Alkoxycarbonyl-$C_1-C_6$-alkyl oder $C_1-C_6$-Alkoxycarbonyl-$C_2-C_6$-alkenyl bedeutet und $R^1$, $R^2$ und $R^3$ die in Anspruch 1 angegebene Bedeutung besitzen, hydrolysiert, oder

f) zur Herstellung von Verbindungen der Formel I, worin $R^4$ $C_1-C_6$-Alkoxycarbonyl-$C_1-C_6$-alkyl oder $C_1-C_6$-Alkoxycarbonyl-$C_2-C_6$-alkenyl bedeutet und $R^1$, $R^2$ und $R^3$ die in Anspruch 1 angegebene Bedeutung besitzen, eine entsprechende Verbindung der Formel I, worin $R^4$ $C_1-C_6$-Alkoxycarbonyl-$C_1-C_6$-alkyl oder $C_1-C_6$-Alkoxycarbonyl-$C_2-C_6$-alkenyl bedeutet, wobei jedoch der Alkoxyrest verschieden ist, und $R^1$, $R^2$ und $R^3$ die in Anspruch 1 angegebene Bedeutung besitzen, mit einem $C_1-C_6$-Alkanol umestert, oder

g) zur Herstellung von Verbindungen der obigen Formel I, worin $R^4$ Aminocarbonyl-$C_1-C_6$-alkyl bedeutet und $R^1$, $R^2$ und $R^3$ die in Anspruch 1 angegebene Bedeutung besitzen, eine entsprechende Verbindung der Formel I, worin $R^4$ $C_1-C_6$-Alkoxycarbonyl-$C_1-C_6$-alkyl bedeutet und $R^1$, $R^2$ und $R^3$ die in Anspruch 1 angegebene Bedeutung besitzen, mit Ammoniak, primärem Amin oder sekundärem Amin umsetzt, oder

h) zur Herstellung von Verbindungen der Formel I, worin $R^4$ Wasserstoff bedeutet und $R^1$, $R^2$ und $R^3$ die in Anspruch 1 angegebene Bedeutung besitzen, eine Verbindung der allgemeinen Formel

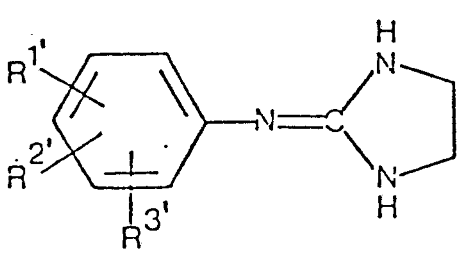

VII

worin $R^{1'}$, $R^{2'}$ und $R^{3'}$ unabhängig voneinander je $C_2-C_6$-Alkyl, $C_1-C_6$-Alkoxy oder Trifluormethyl bedeuten,
mit Kaliumpersulfat in konz. Schwefelsäure oxydiert, oder

i) zur Herstellung von Verbindungen der obigen Formel I, worin $R^4$ [5-Hydroxy-4-(hydroxymethyl)-6-methyl-3-pyridyl]methyl bedeutet und $R^1$, $R^2$ und $R^3$ die in Anspruch 1 angegebene Bedeutung besitzen, in einer Verbindung der allgemeinen Formel

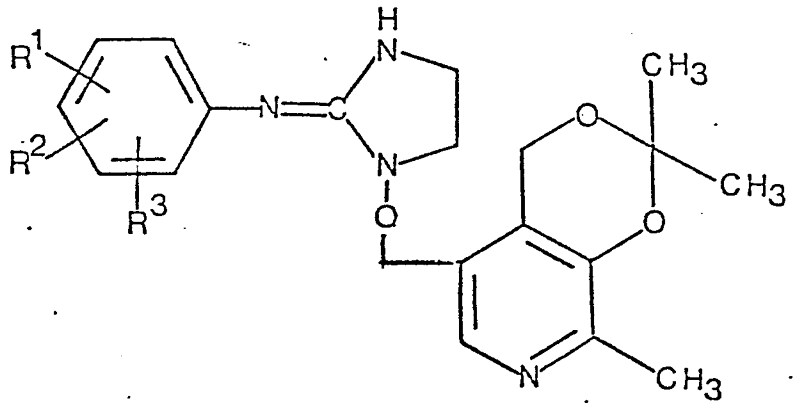

Id

worin $R^1$, $R^2$ und $R^3$ die in Anspruch 1 angegebene Bedeutung besitzen,
die Ketalgruppe abspaltet, und

j) erwünschtenfalls eine erhaltene Verbindung oder ein pharmazeutisch nicht anwendbares Säureadditionssalz davon in ein pharmazeutisch anwendbares Säureadditionssalz davon überführt.

31. Verfahren nach Anspruch 30 zur Herstellung von den in Anspruch 2 definierten Verbindungen der Formel I oder von deren pharmazeutisch anwendbaren Säureadditionssalzen, dadurch gekennzeichnet, daß man Verfahrensvarianten a), b), c), d), e), f), g) und j) von Anspruch 30 durchführt.

32. Verfahren zur Herstellung von Präparaten mit blutdrucksenkenden Eigenschaften, dadurch gekennzeichnet, daß man ein 2-Imino-imidazolidin-Derivat der Formel I in Anspruch 1 oder ein pharmazeutisch anwendbares Säureadditionssalz davon als wirksamen Bestandteil mit zur therapeutischen Verabreichung geeigneten, nicht-toxischen, inerten, an sich in solchen Präparaten üblichen festen und flüssigen Trägern und/oder Excipientien vermischt.

33. Präparate mit blutdrucksenkenden Eigenschaften enthaltend ein 2-Imino-imidazolidin-Derivat der Formel I in Anspruch 1 oder ein pharmazeutisch anwendbares Säureadditionssalz davon und einen Trägerstoff.

**Claims for the Contracting States: BE, CH, FR, LU, NL, SE**

1. 2-Imino-imidazolidine derivatives of the general formula

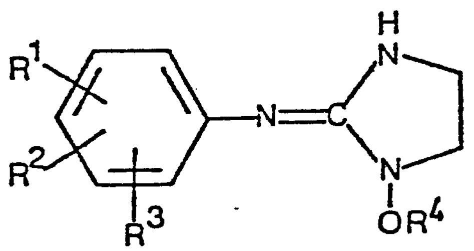

I

wherein $R^1$, $R^2$ and $R^3$ independently of one another each signify hydrogen, $C_1-C_6$-alkyl, $C_1-C_6$-alkoxy, $C_1-C_6$-alkylthio, halogen, trifluoromethyl, cyano or hydroxy and $R^4$ signifies hydrogen, $C_1-C_6$-alkyl, $C_3-C_6$-cycloalkyl, $C_2-C_6$-alkenyl, $C_2-C_6$-alkynyl, di-$C_1-C_6$-alkylamino-$C_1-C_6$-alkyl, carboxy-$C_1-C_6$-alkyl, carboxy-$C_2-C_6$-alkenyl, hydroxy-$C_1-C_6$-alkyl, cyano-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxycarbonyl-$C_1-C_6$-alkyl, $C_1-C_6$-alkoxy-$C_1-C_6$-alkoxy-$C_1-C_6$-alkyl, aminocarbonyl-$C_1-C_6$-alkyl, mono-$C_1-C_6$-alkylaminocarbonyl-$C_1-C_6$-alkyl, di-$C_1-C_6$-alkylaminocarbonyl-$C_1-C_6$-alkyl, $C_1-C_6$-alkoxycarbonyl-$C_2-C_6$-alkenyl, $C_1-C_6$-alkoxy-$C_1-C_6$-alkyl, amino-$C_1-C_6$-alkyl, mono-$C_1-C_6$-alkylamino-$C_1-C_6$-alkyl, di-$C_1-C_6$-alkylamino-$C_1-C_6$-alkyl, (2,2,8-trimethyl-4H-m-dioxino-[4,5-c]pyridin-5-yl)methyl, [5-hydroxy-4-(hydroxymethyl)-6-methyl-3-pyridyl]methyl, $\alpha$-carboxybenzyl, $\alpha$-$C_1-C_6$-alkoxycarbonyl-$\alpha$-$C_1-C_6$-alkylphenyl or a thienyl, furyl or isoxazolyl residue, which is

bonded via a $-CH(R^5)$ group and which is optionally substituted by $C_1-C_6$-alkyl or the group $-COOR$, or a pyridyl residue, which is bonded via a $-CH(R^5)$ group and which is optionally substituted by $C_1-C_6$-alkyl, $C_1-C_6$-alkoxy or the group $-COOR$, and $R^5$ signifies hydrogen, methyl, ethyl or n-propyl and R signifies hydrogen or $C_1-C_6$-alkyl,
as well as pharmaceutically usable acid addition salts thereof.

2. Compounds in accordance with claim 1, wherein $R^4$ signifies hydrogen, $C_1-C_6$-alkyl, $C_3-C_6$-cycloalkyl, $C_2-C_6$-alkenyl, $C_2-C_6$-alkynyl, di-$C_1-C_6$-alkylamino-$C_1-C_6$-alkyl, carboxy-$C_1-C_6$-alkyl, carboxy-$C_2-C_6$-alkenyl, hydroxy-$C_1-C_6$-alkyl, cyano-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxycarbonyl-$C_1-C_6$-alkyl, $C_1-C_6$-alkoxy-$C_1-C_6$-alkoxy-$C_1-C_6$-alkyl, aminocarbonyl-$C_1-C_6$-alkyl, $C_1-C_6$-alkoxycarbonyl-$C_2-C_6$-alkenyl, $C_1-C_6$-alkoxy-$C_1-C_6$-alkyl, amino-$C_1-C_6$-alkyl, mono-$C_1-C_6$-alkylamino-$C_1-C_6$-alkyl, di-$C_1-C_6$-alkylamino-$C_1-C_6$-alkyl, amino-$C_1-C_6$-alkyl, $\alpha$-carboxybenzyl, $\alpha$-$C_1-C_6$-alkoxycarbonyl-$\alpha$-$C_1-C_6$-alkylphenyl or a thienyl, furyl, isoxazolyl or pyridyl residue, which is bonded via a $-CH(R^5)$ group and which is optionally substituted by $C_1-C_6$-alkyl or the group $-COOR$, and $R^1$, $R^2$, $R^3$, $R^5$ and R have the significance given in claim 1.

3. Compounds in accordance with claim 1 or 2, wherein $R^3$ signifies hydrogen.

4. Compounds in accordance with one of claims 1 to 3, wherein $R^1$ and $R^2$ are located in the 2,6-position of the phenyl ring.

5. Compounds in accordance with one of claims 1 to 4, wherein $R^1$ and $R^2$ have the same significance.

6. Compounds in accordance with one of claims 1 to 5, wherein $R^1$ and $R^2$ signify halogen.

7. Compounds in accordance with claim 6, wherein $R^1$ and $R^2$ signify chlorine.

8. Compounds in accordance with one of claims 1 to 7, wherein $R^4$ signifies $C_2-C_6$-alkynyl, carboxy-$C_1-C_6$-alkyl, $C_1-C_6$-alkoxycarbonyl-$C_2-C_6$-alkenyl or a thienyl, furyl, isoxazolyl or pyridyl residue, which is bonded via a $-CH(R^5)$ group and which is optionally substituted by $C_1-C_6$-alkyl or the group $-COOR$, and $R^5$ signifies hydrogen, methyl, ethyl or n-propyl and R signifies hydrogen or $C_1-C_6$-alkyl.

9. Compounds in accordance with claim 8, wherein $R^4$ signifies alkynyl with 3–5 carbon atoms, carboxymethyl, carboxypropyl, ethoxycarbonyl-$C_2-C_6$-alkenyl or a pyridyl or unsubstituted furyl or thienyl residue, which is bonded via a $-CH(R^5)$ group, and $R^5$ signifies hydrogen or methyl.

10. Compounds in accordance with claim 9, wherein $R^4$ signifies propargyl, carboxymethyl, unbranched ethoxycarbonyl-$C_2-C_6$-alkenyl or pyridyl or furyl, which is bonded via a $-CH(R^5)$ group.

11. Compounds in accordance with one of claims 1 to 10, wherein $R^3$ signifies hydrogen, $R^1$ and $R^2$ signify halogen, preferably chlorine, in the 2,6-position, $R^4$ signifies propargyl, carboxymethyl, unbranched ethoxycarbonyl-$C_2-C_6$-alkenyl or pyridyl or furyl, which is bonded via a $-CH(R^5)$ group, and $R^5$ signifies hydrogen or methyl.

12. 2-[(2,6-Dichlorophenyl)imino]-1-hydroxyimidazolidine.

13. 2-[(2,6-Dibromophenyl)imino]-1-hydroxyimidazolidine.

14. 1-Hydroxy-2-[(2-iodophenyl)imino]imidazolidine.

15. Ethyl 4-[/2-[(2,6-dichlorophenyl)imino]-1-imidazolidinyl/oxy]butyrate.

16. 4-[/2-[(2,6-Dichlorophenyl)imino]-1-imidazolidinyl/oxy]butyric acid.

17. Ethyl 2-[/2-[(2,6-dichlorophenyl)imino]-1-imidazolidinyl/oxy]butyrate.

18. 2-[(2,6-Dichlorophenyl)imino]-1-(2-propynyloxy)imidazolidine.

19. [/2-[(2,6-Dichlorophenyl)imino]-1-imidazolidinyl/oxy]acetic acid.

20. Ethyl 4-[/2-[(2,6-dichlorophenyl)imino]-1-imidazolidinyl/oxy]crotonate.

21. 2-[(2,6-Dichlorophenyl)imino]-1-ethoxyimidazolidine.

22. 1-(2-Butynyloxy)-2-[(2,6-dichlorophenyl)imino]imidazolidine.

23. 2-[(2,6-Dichlorophenyl)imino]-1-(furfuryloxy)imidazolidine.

24. 2-[/⟨2-[(2,6-Dichlorophenyl)imino]-1-imidazolidinyl⟩oxy]methyl/pyridine.

25. 3-[/⟨2-[(2,6-Dichlorophenyl)imino]-1-imidazolidinyl⟩oxy]methyl/pyridine.

26. 2-[/⟨2-[(2,6-Dichloro-4-fluorophenyl)imino]-1-imidazolidinyl⟩oxy]methyl/pyridine.

27. 5-[/⟨2-[(2,6-Dichlorophenyl)imino]-1-imidazolidinyl⟩oxy]methyl/-3-hydroxy-2-methyl-4-pyridinemethanol.

28. Salts of the compounds in accordance with claims 12–15, 17, 18 and 20–27.

29. Compounds of the general formula

$$H_2N-CH_2-CH_2-NH-OR^4 \qquad IV$$

wherein $R^4$ signifies hydrogen, $C_1-C_6$-alkyl, $C_3-C_6$-cycloalkyl, $C_2-C_6$-alkenyl, $C_2-C_6$-alkynyl, di-$C_1-C_6$-alkylamino-$C_1-C_6$-alkyl, carboxy-$C_1-C_6$-alkyl, carboxy-$C_2-C_6$-alkenyl, hydroxy-$C_1-C_6$-alkyl, cyano-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxycarbonyl-$C_1-C_6$-alkyl, $C_1-C_6$-alkoxy-$C_1-C_6$-alkoxy-$C_1-C_6$-alkyl, aminocarbonyl-$C_1-C_6$-alkyl,mono-$C_1-C_6$-alkylaminocarbonyl-$C_1-C_6$-alkyl, di-$C_1-C_6$-alkylaminocarbonyl-$C_1-C_6$-alkyl, $C_1-C_6$-alkoxycarbonyl-$C_2-C_6$-alkenyl, $C_1-C_6$-alkoxy-$C_1-C_6$-alkyl, amino-$C_1-C_6$-alkyl, mono-$C_1-C_6$-alkylamino-$C_1-C_6$-alkyl, di-$C_1-C_6$-alkylamino-$C_1-C_6$-alkyl, (2,2,8-trimethyl-4H-m-dioxino[4,5-c]pyridin-5-yl)methyl, [5-hydroxy-4-(hydroxymethyl)-6-methyl-3-pyridyl]methyl, α-carboxybenzyl, α-$C_1-C_6$-alkoxycarbonyl-α-$C_1-C_6$-alkylphenyl or a thienyl, furyl or isoxazolyl residue, which is bonded via a $-CH(R^5)$ group and which is optionally substituted by $C_1-C_6$-alkyl or the group $-COOR$, or a pyridyl residue, which is bonded via a $-CH(R^5)$ group and which is optionally substituted by $C_1-C_6$-alkyl, $C_1-C_6$-alkoxy or the group $-COOR$, and $R^5$ signifies hydrogen, methyl, ethyl or n-propyl and R signifies hydrogen or $C_1-C_6$-alkyl,
when they are used for the manufacture of compounds according to claim 1.

30. Compounds of the general formula

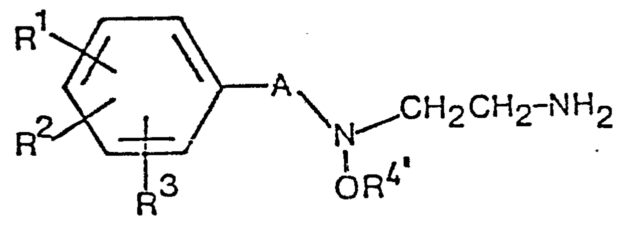

V

wherein A signifies the group

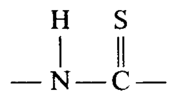

or

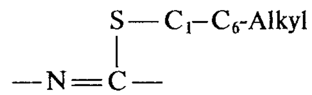

$R^1$, $R^2$ and $R^3$ independently of one another each signify hydrogen, $C_1-C_6$-alkyl, $C_1-C_6$-alkoxy, $C_1-C_6$-alkylthio, halogen, trifluoromethyl, cyano or hydroxy and $R^{4'}$ signifies $C_1-C_6$-alkyl, $C_3-C_6$-cycloalkyl, $C_2-C_6$-alkenyl, di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^5$ signifies hydrogen, methyl, ethyl or n-propyl,
when they are used for the manufacture of compounds according to claim 1.

31. Compounds of the general formula

$$Z-CH_2CH_2-HN-OR^{4'} \qquad XII$$

wherein $R^{4'}$ signifies $C_1-C_6$-alkyl, $C_3-C_6$-cycloalkyl, $C_2-C_6$-alkenyl,di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^5$ signifies hydrogen, methyl, ethyl or n-propyl and Z signifies a phthalimido or succinimido group,
when they are used for the manufacture of compounds according to claim 1.

32. Compounds of the general formula

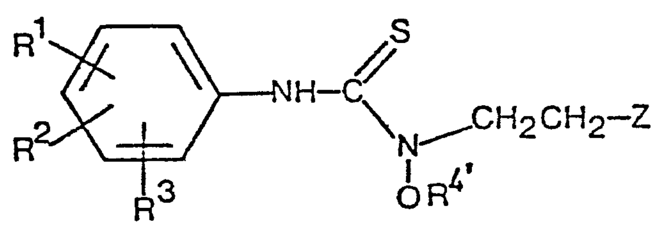

XIII

wherein $R^1$, $R^2$ and $R^3$ independently of one another each signify hydrogen, $C_1-C_6$-alkyl, $C_1-C_6$-alkoxy, $C_1-C_6$-alkylthio, halogen, trifluoromethyl, cyano or hydroxy and $R^{4'}$ signifies $C_1-C_6$-alkyl, $C_3-C_6$-cycloalkyl, $C_2-C_6$-alkenyl, di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^5$ signifies hydrogen, methyl, ethyl or n-propyl and Z signifies a phthalimido or succinimido group,
when they are used for the manufacture of compounds according to claim 1.

33. Compounds of the general formula

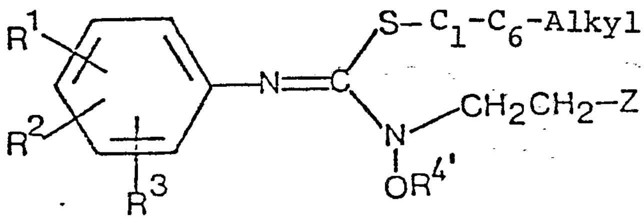

XIV

wherein $R^1$, $R^2$ and $R^3$ independently of one another each signify hydrogen, $C_1-C_6$-alkyl, $C_1-C_6$-alkoxy, $C_1-C_6$-alkylthio, halogen, trifluoromethyl, cyano or hydroxy and $R^{4'}$ signifies $C_1-C_6$-alkyl, $C_3-C_6$-cycloalkyl, $C_2-C_6$-alkenyl, di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^5$ signifies hydrogen, methyl, ethyl or n-propyl and Z signifies a phthalimido or succinimido group,
when they are used for the manufacture of compounds according to claim 1.

34. Process for the manufacture of 2-imino-imidazolidine derivatives of general formula I difined in claim 1 and their pharmaceutically usable acid addition salts, characterized by

a) reacting a compound of the general formula

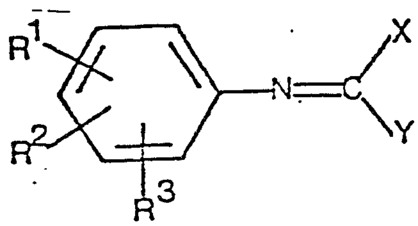

II

wherein X and Y each signify halogen, a sulphydryl, amino, methoxy or alkylthio group with 1–3 carbon atoms and $R^1$, $R^2$ and $R^3$ have the significance given in claim 1,
or a compound of the general formula

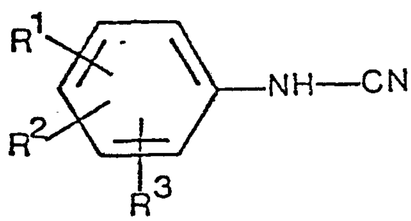

III

wherein $R^1$, $R^2$ and $R^3$ have the significance given in claim 1,
with a compound of the general formula

$$H_2N-CH_2-CH_2-NH-OR^4 \qquad IV$$

wherein $R^4$ has the significance given in claim 1,
or

b) for the manufacture of compounds of formula I wherein $R^4$ signifies $C_1-C_6$-alkyl, $C_3-C_6$-cycloalkyl, $C_2-C_6$-alkenyl, di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^1$, $R^2$, $R^3$ and $R^5$ have the significance given in claim 1, cyclizing a compound of the general formula

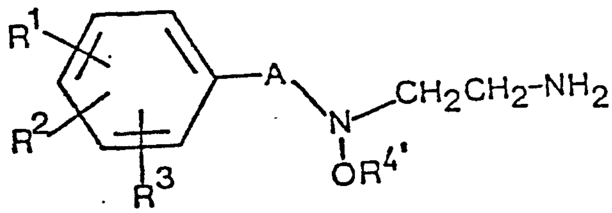

V

wherein A signifies the group

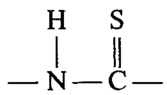

or

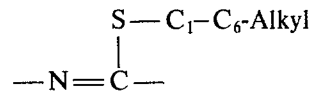

and $R^{4'}$ signifies $C_1-C_6$-alkyl, $C_3-C_6$-cycloalkyl, $C_2-C_6$-alkenyl, di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^1$, $R^2$, $R^3$ and $R^5$ have the significance given in claim 1,

or

c) for the manufacture of compounds of formula I wherein $R^4$ signifies hydrogen and $R^1$, $R^2$ and $R^3$ have the significance given in claim 1, cleaving off the benzyl group in a compound of the general formula

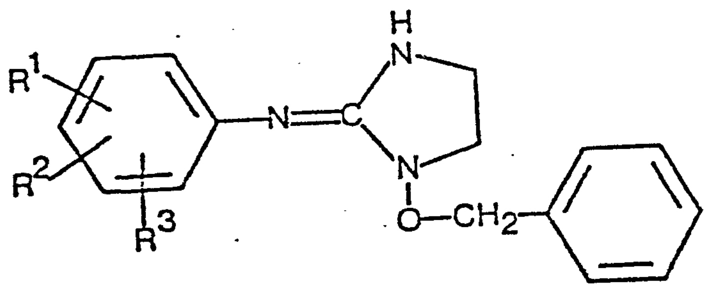

Ia

wherein $R^1$, $R^2$ and $R^3$ have the significance given in claim 1,

or

d) for the manufacture of compounds of the general formula

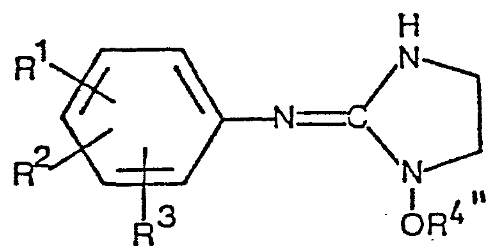

Ib

wherein $R^{4''}$ signifies $C_1-C_6$-alkyl, $C_3-C_6$-cycloalkyl, $C_2-C_6$-alkenyl, $C_2-C_6$-alkynyl, di-$C_1-C_6$-alkylamino-$C_1-C_6$-alkyl, carboxy-$C_1-C_6$-alkyl, hydroxy-$C_1-C_6$-alkyl, cyano-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxycarbonyl-$C_1-C_6$-alkyl, $C_1-C_6$-alkoxy-$C_1-C_6$-alkoxy-$C_1-C_6$-alkyl, aminocarbonyl-$C_1-C_6$-alkyl, mono-$C_1-C_6$-alkylaminocarbonyl-$C_1-C_6$-alkyl, di-$C_1-C_6$-alkylaminocarbonyl-$C_1-C_6$-alkyl, $C_1-C_6$-alkoxycarbonyl-$C_2-C_6$-alkenyl, $C_1-C_6$-alkoxy-$C_1-C_6$-alkyl, amino-$C_1-C_6$-alkyl, mono-$C_1-C_6$-alkylamino-$C_1-C_6$-alkyl, di-$C_1-C_6$-alkylamino-$C_1-C_6$-alkyl or a thienyl, furyl, isoxazolyl or pyridyl residue, which is bonded via a $-CH(R^5)$ group and which is optionally substituted by $C_1-C_6$-alkyl or the group $-COOR$, and R, $R^1$, $R^2$, $R^3$ and $R^5$ have the significance given in claim 1,

reacting a compound of the general formula

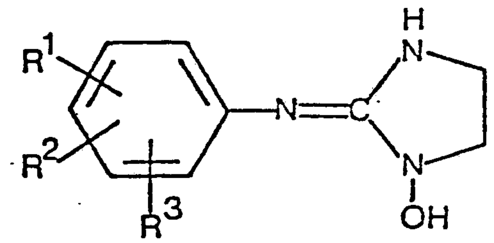

Ic

wherein $R^1$, $R^2$ and $R^3$ have the significance given in claim 1, with a compound of the general formula

$$R^{4''}-X \qquad VI$$

wherein X signifies a leaving group and $R^{4''}$ has the above significance, or

e) for the manufacture of compounds of formula I wherein $R^4$ signifies carboxy-$C_1-C_6$-alkyl or carboxy-$C_2-C_6$-alkenyl and $R^1$, $R^2$ and $R^3$ have the significance given in claim 1, hydrolyzing a corresponding compound of formula I wherein $R^4$ signifies $C_1-C_6$-alkoxycarbonyl-$C_1-C_6$-alkyl or $C_1-C_6$-alkoxycarbonyl-$C_2-C_6$-alkenyl and $R^1$, $R^2$ and $R^3$ have the significance given in claim 1, or

f) for the manufacture of compounds of formula I wherein $R^4$ signifies $C_1-C_6$-alkoxycarbonyl-$C_1-C_6$-alkyl or $C_1-C_6$-alkoxycarbonyl-$C_2-C_6$-alkenyl and $R^1$, $R^2$ and $R^3$ have the significance given in claim 1, trans-esterifying a corresponding compound of formula I wherein $R^4$ signifies $C_1-C_6$-alkoxycarbonyl-$C_1-C_6$-alkyl or $C_1-C_6$-alkoxycarbonyl-$C_2-C_6$-alkenyl, but in which the alkoxy residue is different, and $R^1$, $R^2$ and $R^3$ have the significance given in claim 1, with a $C_1-C_6$-alkanol, or

g) for the manufacture of compounds of formula I above wherein $R^4$ signifies aminocarbonyl-$C_1-C_6$-alkyl and $R^1$, $R^2$ and $R^3$ have the significance given in claim 1, reacting a corresponding compound of formula I wherein $R^4$ signifies $C_1-C_6$-alkoxycarbonyl-$C_1-C_6$-alkyl and $R^1$, $R^2$ and $R^3$ have the significance given in claim 1, with ammonia, a primary amine or a secondary amine, or

h) for the manufacture of compounds of formula I wherein $R^4$ signifies hydrogen and $R^1$, $R^2$ and $R^3$ have the significance given in claim 1, oxidizing a compound of the general formula

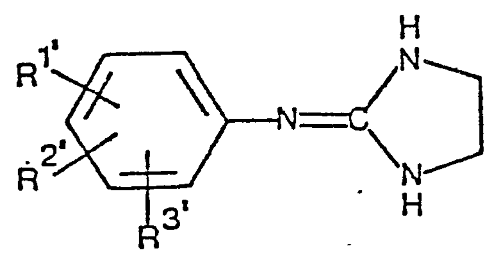

VII

wherein $R^{1'}$, $R^{2'}$ and $R^{3'}$ independently of one another each signify hydrogen, $C_1-C_6$-alkyl, $C_1-C_6$-alkoxy, halogen or trifluoromethyl, with potassium persulphate in conc. sulphuric acid, or

i) for the manufacture of compounds of formula I above wherein $R^4$ signifies [5-hydroxy-4-(hydroymethyl)-6-methyl-3-pyridyl]methyl and $R^1$, $R^2$ and $R^3$ have the significance given in claim 1, cleaving off the ketal group in a compound of the general formula

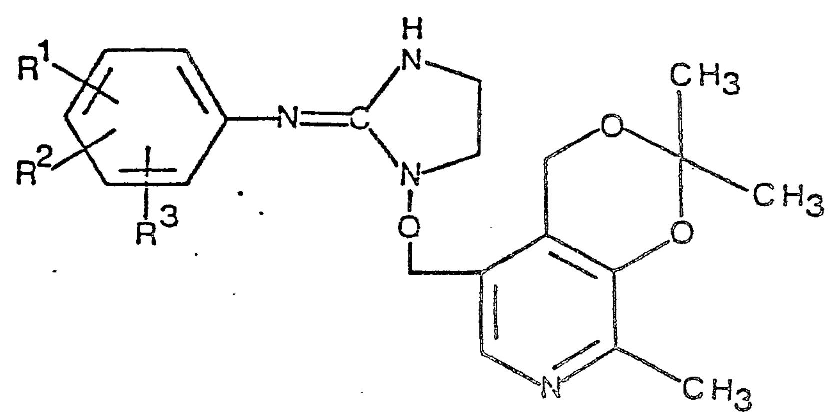

Id

wherein $R^1$, $R^2$ and $R^3$ have the significance given in claim 1,

and

j) if desired, converting a compound obtained or an acid addition salt thereof which is pharmaceutically non-usable into a pharmaceutically usable acid addition salt.

35. Process according to claim 34 for the manufacture of the compounds of formula I defined in claim 2 or of their pharmaceutically usable acid addition salts, characterized by carrying out process variants a), b), c), d), e), f), g) and j) of claim 34.

36. Process for the manufacture of preparations with hypotensive properties, characterized by mixing a 2-imino-imidazolidine derivative of formula I in claim 1 or a pharmaceutically usable acid addition salt thereof as the active ingredient with non-toxic, inert, solid and liquid carriers and/or excipients which are customary in such preparations and suitable for therapeutic administration.

37. Preparations with hypotensive properties, containing a 2-imino-imidazolidine derivative of formula I in claim 1 or a pharmaceutically usable acid addition salt thereof and a carrier material.

**Claims for the Contracting State: DE**

1. 2- Imino-imidazolidine derivatives of the general formula

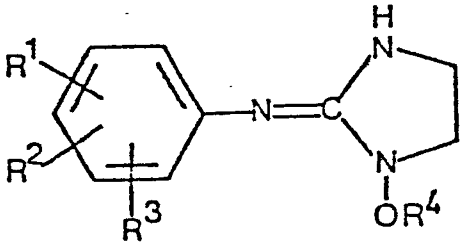

I

wherein $R^1$, $R^2$ and $R^3$ independently of one another each signify hydrogen, $C_1-C_6$-alkyl, $C_1-C_6$-alkoxy, $C_1-C_6$-alkylthio, halogen, trifluoromethyl, cyano or hydroxy and $R^4$ signifies hydrogen, $C_1-C_6$-alkyl, $C_3-C_6$-cycloalkyl, $C_2-C_6$-alkenyl, $C_2-C_6$-alkynyl, di-$C_1-C_6$-alkylamino-$C_1-C_6$-alkyl, carboxy-$C_1-C_6$-alkyl, carboxy-$C_2-C_6$-alkenyl, hydroxy-$C_1-C_6$-alkyl, cyano-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxycarbonyl-$C_1-C_6$-alkyl, $C_1-C_6$-alkoxy-$C_1-C_6$-alkoxy-$C_1-C_6$-alkyl, aminocarbonyl-$C_1-C_6$-alkyl, mono-$C_1-C_6$-alkylaminocarbonyl-$C_1-C_6$-alkyl, di-$C_1-C_6$-alkylaminocarbonyl-$C_1-C_6$-alkyl, $C_1-C_6$-alkoxycarbonyl-$C_2-C_6$-alkenyl, $C_1-C_6$-alkoxy-$C_1-C_6$-alkyl, amino-$C_1-C_6$-alkyl, mono-$C_1-C_6$-alkylamino-$C_1-C_6$-alkyl, di-$C_1-C_6$-alkylamino-$C_1-C_6$-alkyl, (2,2,8-trimethyl)-4H-m-dioxino-[4,5-c]-pyridin-5-yl)methyl, [5-hydroxy-4-(hydroxymethyl)-6-methyl-3-pyridyl]methyl, α-carboxybenzyl, α-$C_1-C_6$-alkoxycarbonyl-α-$C_1-C_6$-alkylphenyl or a thienyl, furyl or isoxazolyl residue, which is bonded via a $-CH(R^5)$ group and which is optionally substituted by $C_1-C_6$-alkyl or the group $-COOR$, or a pyridyl residue, which is bonded via a $-CH(R^5)$ group and which is optionally substituted by $C_1-C_6$-alkyl, $C_1-C_6$-alkoxy or the group $-COOR$, and $R^5$ signifies hydrogen, methyl, ethyl or n-propyl and R signifies hydrogen or $C_1-C_6$-alkyl, whereby $R^4$ is different from hydrogen, $C_1-C_6$-alkyl, hydroxy-$C_1-C_6$-alkyl, benzyl, phenethyl and $C_1-C_6$-alkoxy-$C_1-C_6$-alkyl where $R^1$, $R^2$ and $R^3$ independently of one another signify hydrogen, methyl or halogen,
as well as pharmaceutically usable acid addition salts thereof.

2. Compounds in accordance with claim 1, wherein $R^4$ signifies hydrogen, $C_1-C_6$-alkyl, $C_3-C_6$-cycloalkyl, $C_2-C_6$-alkenyl, $C_2-C_6$-alkynyl, di-$C_1-C_6$-alkylamino-$C_1-C_6$-alkyl, carboxy-$C_1-C_6$-alkyl, carboxy-$C_2-C_6$-alkenyl, hydroxy-$C_1-C_6$-alkyl, cyano-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxycarbonyl-$C_1-C_6$-alkyl, $C_1-C_6$-alkoxy-$C_1-C_6$-alkoxy-$C_1-C_6$-alkyl, aminocarbonyl-$C_1-C_6$-alkyl, $C_1-C_6$-alkoxycarbonyl-$C_2-C_6$-alkenyl, $C_1-C_6$-alkoxy-$C_1-C_6$-alkyl, amino-$C_1-C_6$-alkyl, mono-$C_1-C_6$-alkylamino-$C_1-C_6$-alkyl, di-$C_1-C_6$-alkylamino-$C_1-C_6$-alkyl, amino-$C_1-C_6$-alkyl, α-carboxybenzyl, α-$C_1-C_6$-alkoxycarbonyl-α-$C_1-C_6$-alkylphenyl or a thienyl, furyl, isoxazolyl or pyridyl residue, which is bonded via a $-CH(R^5)$ group and which is optionally substituted by $C_1-C_6$-alkyl or the group $-COOR$, and $R^1$, $R^2$, $R^3$, $R^5$ and R have the significance given in claim 1.

3. Compounds in accordance with claim 1 or 2, wherein $R^3$ signifies hydrogen.

4. Compounds in accordance with one of claims 1 to 3, wherein $R^1$ and $R^2$ are located in the 2,6-position of the phenyl ring.

5. Compounds in accordance with one of claims 1 to 4, wherein $R^1$ and $R^2$ have the same significance.

6. Compounds in accordance with one of claims 1 to 5, wherein $R^1$ and $R^2$ signify halogen.

7. Compounds in accordance with claim 6, wherein $R^1$ and $R^2$ signify chlorine.

8. Compounds in accordance with one of claims 1 to 7, wherein $R^4$ signifies $C_2-C_6$-alkynyl, carboxy-$C_1-C_6$-alkyl, $C_1-C_6$-alkoxycarbonyl-$C_2-C_6$-alkenyl or a thienyl, furyl, isoxazolyl or pyridyl

residue, which is bonded via a $-CH(R^5)$ group and which is optionally substituted by $C_1-C_6$-alkyl or the group $-COOR$, and $R^5$ signifies hydrogen, methyl, ethyl or n-propyl and R signifies hydrogen or $C_1-C_6$-alkyl.

9. Compounds in accordance with claim 8, wherein $R^4$ signifies alkynyl with 3—5 carbon atoms, carboxymethyl, carboxypropyl, ethoxycarbonyl-$C_2-C_6$-alkenyl or a pyridyl or unsubstituted furyl or thienyl residue, which is bonded via a $-CH(R^5)$ group, and $R^5$ signifies hydrogen or methyl.

10. Compounds in accordance with claim 9, wherein $R^4$ signifies propargyl, carboxymethyl, unbranched ethoxycarbonyl-$C_2-C_6$-alkenyl or pyridyl or furyl, which is bonded via a $-CH(R^5)$ group.

11. Compounds in accordance with one of claims 1 to 10, wherein $R^3$ signifies hydrogen, $R^1$ and $R^2$ signify halogen, preferably chlorine, in the 2,6-position, $R^4$ signifies propargyl, carboxymethyl, unbranched ethoxycarbonyl-$C_2-C_6$-alkenyl or pyridyl or furyl, which is bonded via a $-CH(R^5)$ group, and $R^5$ signifies hydrogen or methyl.

12. Ethyl 4-[/2-[(2,6-dichlorophenyl)imino]-1-imidazolidinyl/oxy]butyrate.
13. 4-[/2-[(2,6-Dichlorophenyl)imino]-1-imidazolidinyl/oxy]butyric acid.
14. Ethyl 2-[/2-[(2,6-dichlorophenyl)imino]-1-imidazolidinyl/oxy]butyrate.
15. 2-[(2,6-Dichlorophenyl)imino]-1-(2-propynyloxy)-imidazolidine.
16. [/2-[(2,6-Dichlorophenyl)imino]-1-imidazolidinyl/oxy]acetic acid.
17. Ethyl 4-[/2-[(2,6-dichlorophenyl)imino]-1-imidazolidinyl/oxy]crotonate.
18. 1-(2-Butynyloxy)-2-[(2,6-dichlorophenyl)imino]imidazolidine.
19. 2-[(2,6-Dichlorophenyl)imino]-1-(furfuryloxy)imidazolidine.
20. 2-[/⟨2-[(2,6-Dichlorophenyl)imino]-1-imidazolidinyl⟩oxy]methyl/pyridine.
21. 3-[/⟨2-[(2,6-Dichlorophenyl)imino]-1-imidazolidinyl⟩oxy]methyl/pyridine.
22. 2-[/⟨2-[(2,6-Dichloro-4-fluorophenyl)imino]-1-imidazolidinyl⟩oxy]methyl/pyridine.
23. 5-[/⟨2-[(2,6-Dichlorophenyl)imino]-1-imidazolidinyl⟩oxy]methyl/-3-hydroxy-2-methyl-4-pyridinemethanol.
24. Salts of the compounds in accordance with claims 12, 14, 15 and 17 to 23.
25. Compounds of the general formula

$$H_2N-CH_2-CH_2-NH-OR^4 \qquad IV$$

wherein $R^4$ signifies hydrogen, $C_3-C_6$-cycloalkyl, $C_2-C_6$-alkenyl, $C_2-C_6$-alkynyl, di-$C_1-C_6$-alkylamino-$C_1-C_6$-alkyl, carboxy-$C_1-C_6$-alkyl, carboxy-$C_2-C_6$-alkenyl, cyano-$C_1-C_6$-alkyl, phenyl-$C_3-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxycarbonyl-$C_1-C_6$-alkyl, $C_1-C_6$-alkoxy-$C_1-C_6$-alkoxy-$C_1-C_6$-alkyl, aminocarbonyl-$C_1-C_6$-alkyl, mono-$C_1-C_6$-alkylaminocarbonyl-$C_1-C_6$-alkyl, di-$C_1-C_6$-alkylaminocarbonyl-$C_1-C_6$-alkyl, $C_1-C_6$-alkoxycarbonyl-$C_2-C_6$-alkenyl, amino-$C_1-C_6$-alkyl, mono-$C_1-C_6$-alkylamino-$C_1-C_6$-alkyl, di-$C_1-C_6$-alkylamino-$C_1-C_6$-alkyl, (2,2,8-trimethyl-4H-m-dioxino[4,5-c]pyridin-5-yl)methyl, [5-hydroxy-4-hydroxymethyl)-6-methyl-3-pyridyl]methyl, $\alpha$-carboxybenzyl, $\alpha$-$C_1-C_6$-alkoxycarbonyl-$\alpha$-$C_1-C_6$-alkylphenyl or a thienyl, furyl or isoxazolyl residue, which is bonded via a $-CH(R^5)$ group and which is optionally substituted by $C_1-C_6$-alkyl or the group $-COOR$, or a pyridyl residue, which is bonded via a $-CH(R^5)$ group and which is optionally substituted by $C_1-C_6$-alkyl, $C_1-C_6$-alkoxy or the group $-COOR$, and $R^5$ signifies hydrogen, methyl, ethyl or n-propyl and R signifies hydrogen or $C_1-C_6$-alkyl,
when they are used for the manufacture of compounds according to claim 1.

26. Compounds of the general formula

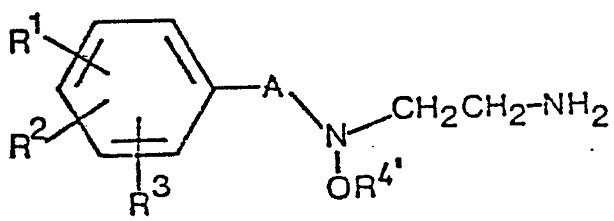

V

wherein A signifies the group

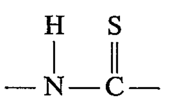

or

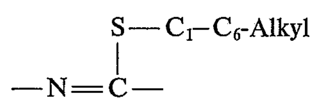

$R^1$, $R^2$ and $R^3$ independently of one another each signify hydrogen, $C_1-C_6$-alkyl, $C_1-C_6$-alkoxy, $C_1-C_6$-alkylthio, halogen, trifluormethyl, cyano or hydroxy and $R^{4'}$ signifies a $C_1-C_6$-alkyl, $C_3-C_6$-cycloalkyl, $C_2-C_6$-alkenyl, di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl,

$C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^5$ signifies hydrogen, methyl, ethyl or n-propyl, whereby $R^{4'}$ is different from $C_1-C_6$-alkyl, benzyl, phenethyl and $C_1-C_6$-alkoxy-$C_1-C_6$-alkyl where $R^1$, $R^2$ and $R^3$ independently of one another signify hydrogen, methyl or halogen,
when they are used for the manufacture of compounds according to claim 1.

27. Compounds of the general formula

$$Z-CH_2CH_2-HN-OR^{4'} \qquad XII$$

wherein $R^{4'}$ signifies $C_1-C_6$-alkyl, $C_3-C_6$-cycloalkyl, $C_2-C_6$-alkenyl,di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^5$ signifies hydrogen, methyl, ethyl or n-propyl and Z signifies a phthalimido or succinimido group,
when they are used for the manufacture of compounds according to claim 1.

28. Compounds of the general formula

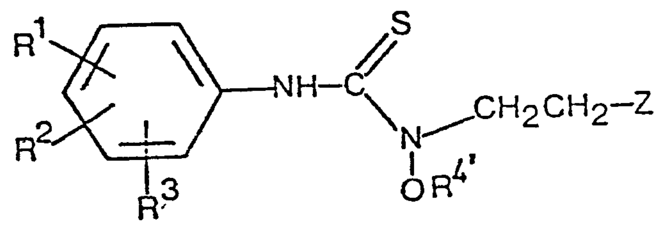

XIII

wherein $R^1$, $R^2$ and $R^3$ independently of one another each signify hydrogen, $C_1-C_6$-alkyl, $C_1-C_6$-alkoxy, $C_1-C_6$-alkylthio, halogen, trifluoromethyl, cyano or hydroxy and $R^{4'}$ signifies $C_1-C_6$-alkyl, $C_3-C_6$-cycloalkyl, $C_2-C_6$-alkenyl, di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^5$ signifies hydrogen, methyl, ethyl or n-propyl and Z signifies a phthalimido or succinimido group, whereby $R^{4'}$ is different from $C_1-C_6$-alkyl, benzyl, phenethyl and $C_1-C_6$-alkoxy-$C_1-C_6$-alkyl where $R^1$, $R^2$ and $R^3$ independently of one another signify, hydrogen, methyl or halogen,
when they are used for the manufacture of compounds according to claim 1.

29. Compounds of the general formula

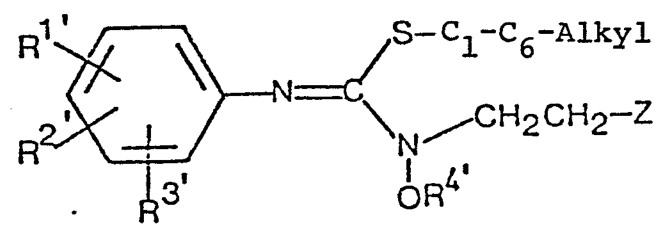

XIV'

wherein $R^{1'}$, $R^{2'}$ and $R^{3'}$ independently of one another each signify hydrogen, $C_1-C_6$-alkyl, $C_1-C_6$-alkoxy, $C_1-C_6$-alkylthio, halogen, trifluoromethyl, cyano or hydroxy and $R^{4'}$ signifies $C_1-C_6$-alkyl, $C_3-C_6$-cycloalkyl, $C_2-C_6$-alkenyl, di-$C_1-C_6$-alkylamino-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^5$ signifies hydrogen, methyl, ethyl or n-propyl and Z signifies a phthalimido or succinimido group, whereby $R^{4'}$ is different from $C_1-C_6$-alkyl, benzyl, phenethyl and $C_1-C_6$-alkoxy-$C_1-C_6$-alkyl where $R^1$, $R^2$ and $R^3$ independently of one another signify hydrogen, methyl or halogen,
when they are used for the manufacture of compounds according to claim 1.

30. Process for the manufacture of 2-imino-imidazolidine derivatives of general formula I defined in claim 1 and their pharmaceutically usable acid addition salts, characterized by

a) reacting a compound of the general formula

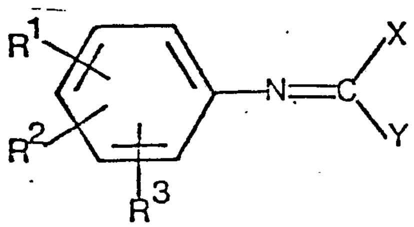

II

wherein X and Y each signify halogen, a sulphydryl, amino, methoxy or alkylthio group with 1–3 carbon atoms and $R^1$, $R^2$ and $R^3$ have the significance given in claim 1,
or a compound of the general formula

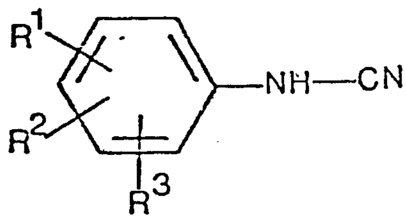

III

wherein $R^1$, $R^2$ and $R^3$ have the significance given in claim 1,
with a compound of the general formula

$$H_2N-CH_2-CH_2-NH-OR^4 \qquad IV$$

wherein $R^4$ has the significance given in claim 1,
or

b) for the manufacture of compounds of formula I wherein $R^4$ signifies $C_1-C_6$-alkyl, $C_3-C_6$-cycloalkyl, $C_2-C_6$-alkenyl, di-$C_1-C_6$-alkylamino-$C_1-C_6$-alkyl, phenyl-$C_1-C_6$-alkyl, phenyl-$C_1-C_6$ substituted in the phenyl nucleus by methoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded via a $-CH(R^5)$ group and which is optionally substituted by $C_1-C_6$-alkyl, and $R^1$, $R^2$, $R^3$ and $R^5$ have the significance given in claim 1, cyclizing a compound of the general formula

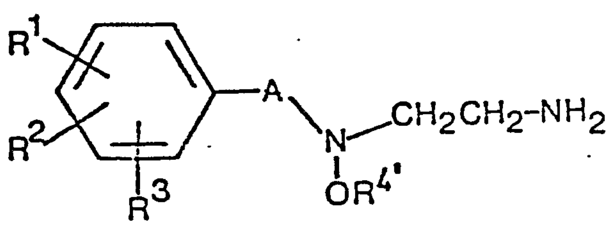

V

wherein A signifies the group

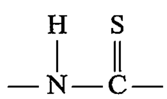

or

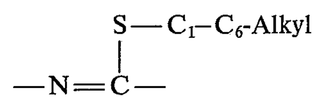

and $R^{4'}$ signifies a $C_1-C_6$-alkyl, $C_3-C_6$-cycloalkyl, $C_2-C_6$-alkenyl,di-$C_1-C_6$-alkylamino-$C_1-C_6$-alkyl, phenyl-$C_1-C_6$-alkyl, phenyl-$C_1-C_6$-alkyl substituted in the phenyl nucleus by alkoxy groups, $C_1-C_6$-alkylthio-$C_1-C_6$-alkyl, $C_1-C_6$-alkoxy-$C_1-C_6$-alkoxy-$C_1-C_6$-alkyl, $C_1-C_6$-alkoxy-$C_1-C_6$-alkyl or a thienyl, furyl, isoxazolyl or pyridyl residue, which is bonded by a $-CH(R^5)$ group and which is optionally substituted by $C_1-C_6$-alkyl, and $R^1$, $R^2$, $R^3$ and $R^5$ have the significance given in claim 1,
or

c) for the manufacture of compounds of formula I wherein $R^4$ signifies hydrogen and $R^1$, $R^2$ and $R^3$ have the significance given in claim 1, cleaving off the benzyl group in a compound of the general formula

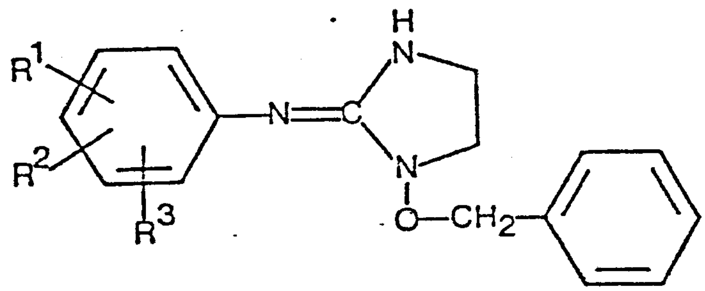

Ia

wherein $R^1$, $R^2$ and $R^3$ have the significance given in claim 1,
or

d) for the manufacture of compounds of the general formula

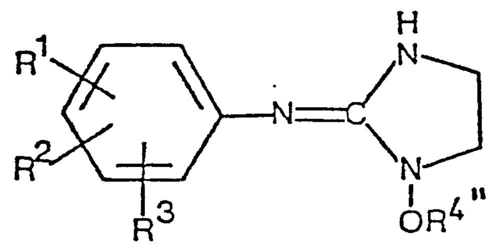

Ib

wherein $R^{4''}$ signifies $C_1-C_6$-alkyl, $C_3-C_6$-cycloalkyl, $C_2-C_6$-alkenyl, $C_2-C_6$-alkynyl, di-$C_1-C_6$-alkylamino-$C_1-C_6$-alkyl, carboxy-$C_1-C_6$-alkyl, hydroxy-$C_1-C_6$-alkyl, cyano-$C_1-C_6$-alkyl, phenyl-$C_1-C_6$-alkyl, phenyl-$C_1-C_6$-alkyl substituted in the phenyl nucleus by methoxy groups, $C_1-C_6$-alkylthio-$C_1-C_6$-alkyl, $C_1-C_6$-alkoxycarbonyl-$C_1-C_6$-alkyl, $C_1-C_6$-alkoxy-$C_1-C_6$-alkoxy-$C_1-C_6$-alkyl, aminocarbonyl-$C_1-C_6$-alkyl, mono-$C_1-C_6$-alkylaminocarbonyl-$C_1-C_6$-alkyl, di-$C_1-C_6$-alkylaminocarbonyl-$C_1-C_6$-alkyl, $C_1-C_6$-alkoxycarbonyl-$C_2-C_6$-alkenyl, $C_1-C_6$-alkoxy-$C_1-C_6$-alkyl, amino-$C_1-C_6$-alkyl, mono-$C_1-C_6$-alkylamino-$C_1-C_6$-alkyl, di-$C_1-C_6$-alkylamino-$C_1-C_6$-alkyl or a thienyl, furyl, isoxazolyl or pyridyl residue, which is bonded via a $-CH(R^5)$ group and which is optionally substituted by $C_1-C_6$-alkyl or the group $-COOR$, and R, $R^1$, $R^2$, $R^3$ and $R^5$ have the significance given in claim 1,
reacting a compound of the general formula

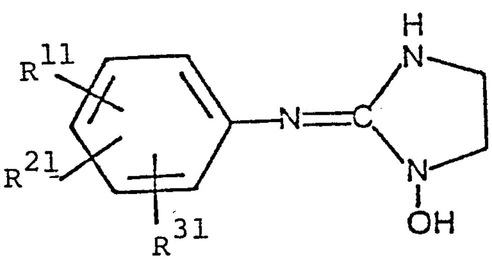

Ic

wherein $R^{11}$, $R^{21}$ and $R^{31}$ independently of one another each signify hydrogen, $C_1-C_6$-alkyl, $C_1-C_6$-alkoxy, $C_1-C_6$-alkylthio, halogen, trifluoromethyl, cyano or hydroxy,
with a compound of the general formula

$$R^{4''}-X \qquad VI$$

wherein X signifies a leaving group and $R^{4''}$ has the above significance,
or

e) for the manufacture of compounds of formula I wherein $R^4$ signifies carboxy-$C_1-C_6$-alkyl or carboxy-$C_2-C_6$-alkenyl and $R^1$, $R^2$ and $R^3$ have the significance given in claim 1, hydrolyzing a corresponding compound of formula I wherein $R^4$ signifies $C_1-C_6$-alkoxycarbonyl-$C_1-C_6$-alkyl or $C_1-C_6$-alkoxycarbonyl-$C_2-C_6$-alkenyl and $R^1$, $R^2$ and $R^3$ have the significance given in claim 1, or

f) for the manufacture of compounds of formula I wherein $R^4$ signifies $C_1-C_6$-alkoxycarbonyl-$C_1-C_6$-alkyl or $C_1-C_6$-alkoxycarbonyl-$C_2-C_6$-alkenyl and $R^1$, $R^2$ and $R^3$ have the significance given in claim 1, trans-esterifying a corresponding compound of formula I wherein $R^4$ signifies $C_1-C_6$-alkoxycarbonyl-$C_1-C_6$-alkyl or $C_1-C_6$-alkoxycarbonyl-$C_2-C_6$-alkenyl, but in which, the alkoxy residue is different, and $R^1$, $R^2$ and $R^3$ have the significance given in claim 1, with a $C_1-C_6$-alkanol, or

g) for the manufacture of compounds of formula I above wherein $R^4$ signifies aminocarbonyl-$C_1-C_6$-alkyl and $R^1$, $R^2$ and $R^3$ have the significance given in claim 1, reacting a corresponding compound

of formula I wherein $R^4$ signifies $C_1-C_6$-alkoxycarbonyl-$C_1-C_6$-alkyl and $R^1$, $R^2$ and $R^3$ have the significance given in claim 1, with ammonia, a primary amine or a secondary amine, or

h) for the manufacture of compounds of formula I wherein $R^4$ signifies hydrogen and $R^1$, $R^2$ and $R^3$ have the significance given in claim 1, oxidizing a compound of the general formula

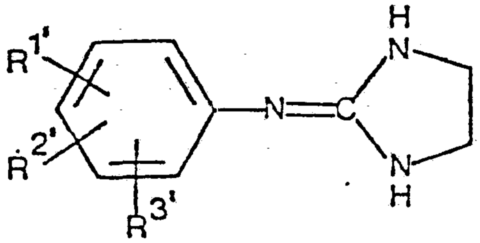

VII

wherein $R^{1'}$, $R^{2'}$ and $R^{3'}$ independently of one another each signify $C_2-C_6$-alkyl, $C_1-C_6$-alkoxy or trifluoromethyl,
with potassium persulphate in conc. sulphuric acid, or

i) for the manufacture of compounds of formula I above wherein $R^4$ signifies [5-hydroxy-4-(hydroxymethyl)-6-methyl-3-pyridyl]methyl and $R^1$, $R^2$ and $R^3$ have the significance given in Calim 1, cleaving off the ketal group in a compound of the general formula

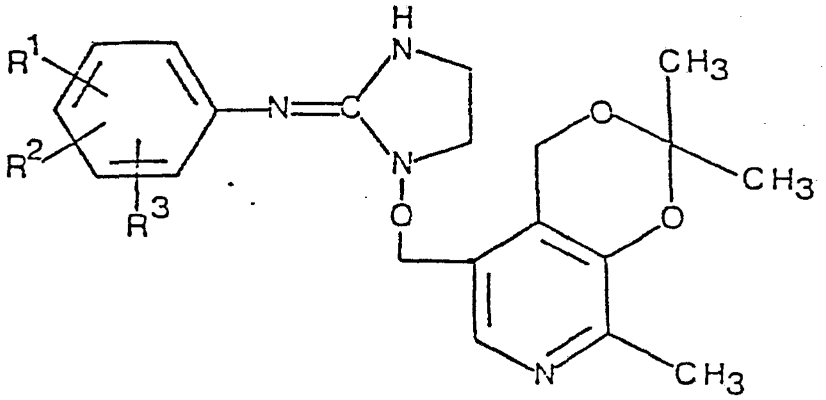

Id

wherein $R^1$, $R^2$ and $R^3$ have the significance given in claim 1,
and

j) if desired, converting a compound obtained or an acid addition salt thereof which is pharmaceutically non-usable into a pharmaceutically usable acid addition salt thereof.

31. Process according to claim 30 for the manufacture of the compounds of formula I defined in claim 2 or of their pharmaceutically usable acid addition salts, characterized by carrying out process variants a), b), c), d), e), f), g) and j) of claim 30.

32. Process for the manufacture of preparations with hypotensive properties, characterized by mixing a 2-imino-imidazolidine derivative of formula I in claim 1 or a pharmaceutically usable acid addition salt thereof as the active ingredient with non-toxic, inert, solid and liquid carriers and/or excipients which are usual in such preparations and which are suitable for therapeutic administration.

33. Preparations with hypotensive properties, containing a 2-imino-imidazolidine derivative of formula I in claim 1 or a pharmaceutically usable acid addition salt thereof and a carrier material.

**Revendications pour les Etats contractants: BE, CH, FR, LU, NL, SE**

1. Dérivés de 2-imino-imidazolidine de formule générale

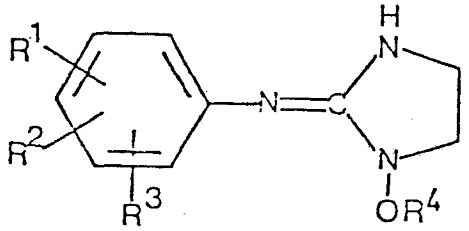

I

où $R^1$, $R^2$ et $R^3$ représentent chacun indépendamment l'un de l'autre un hydrogène, un alcoyle en $C_1$ à $C_6$, un alcoxy en $C_1$ à $C_6$, un alcoylthio en $C_1$ à $C_6$, un halogène, un trifluorométhyle, un cyano ou un hydroxy et $R^4$ représente un hydrogène, un alcoyle en $C_1$ à $C_6$, un cycloalcoyle en $C_3$ à $C_6$, un alcényle en $C_2$ à $C_6$, un alcynyle en $C_2$ à $C_6$, un di-alcoylamino en $C_1$ à $C_6$-alcoyle en $C_1$ à $C_6$, un carboxyalcoyle en $C_1$ à $C_6$, un carboxy-alcényle en $C_2$ à $C_6$, un hydroxy-alcoyle en $C_1$ à $C_6$, un cyano-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxycarbonyle en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un aminocarbonyl-alcoyle en $C_1$ à $C_6$, un mono-alcoylaminocarbonyle en $C_1$ à $C_6$-alcoyle en $C_1$ à $C_6$, un di-alcoylaminocarbonyle en $C_1$ à $C_6$-alcoyle en $C_1$ à $C_6$, und alcoxycarbonyle en $C_1$ à $C_6$-alcényle en $C_2$ à $C_6$, un alcoxy en $C_1$ à $C_6$-alcoyle en $C_1$ à $C_6$, un amino-alcoyle en $C_1$ à $C_6$, un mono-alcoylamino en $C_1$ à $C_6$-alcoyle en $C_1$ à $C_6$, un dialcoylamino en $C_1$ à $C_6$-alcoyle en $C_1$ à $C_6$, un (2,2,8-triméthyl-4H-m-dioxino-[4,5-c]-pyridin-5-yl)méthyle, un [5-hydroxy-4-(hydroxyméthyl)-6-méthyl-3-pyridyl]méthyle, un $\alpha$-carboxybenzyle, un $\alpha$-alcoxycarbonyle en $C_1$ à $C_6$-$\alpha$-alcoylphényle en $C_1$ à $C_6$ ou un radical thiényle, furyle ou isoxazolyle lié par l'intermédiaire d'un groupe $-CH(R^5)$ et éventuellement substitué par un alcoyle en $C_1$ à $C_6$ ou le groupe $-COOR$ ou un radical pyridyle lié par l'intermédiaire d'un groupe $-CH(R^5)$ et éventuellement substitué par un alcoyle en $C_1$ à $C_6$, un alcoxy en $C_1$ à $C_6$ ou le groupe $-COOR$ et $R^5$ représente un hydrogène, un méthyle, un éthyle, ou un n-propyle et R représente un hydrogène ou un alcoyle en $C_1$ à $C_6$,
ainsi que leurs sels d'addition acide pharmaceutiquement acceptables.

2. Composés selon la revendication 1 où $R^4$ représente un hydrogène, un alcoyle en $C_1$ à $C_6$, un cycloalcoyle en $C_3$ à $C_6$, un alcényle en $C_2$ à $C_6$, un alcynyle en $C_2$ à $C_6$, un di-alcoylamino en $C_1$ à $C_6$-alcoyle en $C_1$ à $C_6$, un carboxy-alcoyle en $C_1$ à $C_6$, un carboxy-alcényle en $C_2$ à $C_6$, un hydroxy-alcoyle en $C_1$ à $C_6$, un cyano-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxycarbonyle en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un aminocarbonyl-alcoyle en $C_1$ à $C_6$, un alcoxycarbonyle en $C_1$ à $C_6$-alcényle en $C_2$ à $C_6$, un alcoxy en $C_1$ à $C_6$-alcoyle en $C_1$ à $C_6$, un amino-alcoyle en $C_1$ à $C_6$, un mono-alcoylamino en $C_1$ à $C_6$-alcoyle en $C_1$ à $C_6$, un di-alcoylamino en $C_1$ à $C_6$-alcoyle en $C_1$ à $C_6$, un amino-alcoyle en $C_1$ à $C_6$, un $\alpha$-carboxybenzyle, un $\alpha$-alcoxy-carbonyle en $C_1$ à $C_6$-$\alpha$-alcoylphényle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ ou le groupe $-COOR$, et $R^1$, $R^2$, $R^3$, $R^5$ et R possèdent la signification donnée dans la revendication 1.

3. Composés selon la revendication 1 ou la revendication 2, où $R^3$ représente un hydrogène.

4. Composés selon l'une des revendications 1 à 3, où $R^1$ et $R^2$ sont localisés en position 2,6 du noyau phényle.

5. Composés selon l'une des revendications 1 à 4, où $R^1$ et $R^2$ ont la même signification.

6. Composés selon l'une des revendications 1 à 5, où $R^1$ et $R^2$ représentent un halogène.

7. Composés selon la revendication 6, où $R^1$ et $R^2$ représentent un chlore.

8. Composés selon l'une des revendications 1 à 7, où $R^4$ représente un alcynyle en $C_2$ à $C_6$, un carboxyalcoyle en $C_1$ à $C_6$, un alcoxycarbonyle en $C_1$ à $C_6$-alcényle en $C_2$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ ou le groupe $-COOR$ et $R^5$ représente un hydrogène, un méthyle, un éthyle ou un n-propyle et R représente un hydrogène ou un alcoyle en $C_1$ à $C_6$.

9. Composés selon la revendication 8, où $R^4$ représente un alcynyle en $C_3$ à $C_5$, un carboxyméthyle, un carboxypropyle, un éthoxycarbonyl-alcényle en $C_2$ à $C_6$ ou un radical pyridyle ou un radical furyle ou thiényle non substitué lié par l'intermédiaire d'un groupe $-CH(R^5)$ et $R^5$ représente un hydrogène ou un méthyle.

10. Composés selon la revendication 9, où $R^4$ représente un propargyle, un carboxyméthyle, un éthoxycarbonyl-alcényle en $C_2$ à $C_6$ non ramifié ou un pyridyle ou un furyle lié par l'intermédiaire d'un groupe $-CHR(R^5)$.

11. Composés selon l'une des revendications 1 à 10, où $R^3$ représente un hydrogène, $R^1$ et $R^2$ un halogahalogène, de préférence le chlore, en position 2,6, $R^4$ un propargyle, un carboxyméthyle, un éthoxycarbonyl-alcényle en $C_2$ à $C_6$ non ramifié ou un pyridyle ou un furyle lié par l'intermédiaire d'un groupe $-CH(R^5)$ et $R^5$ représente un hydrogène ou un méthyle.

12. 2-[(2,6-dichlorphényl)imino]-1-hydroxyimidazolidine.

13. 2-[(2,6-dibromophényl)imino]-1-hydroxyimidazolidine.

14. 1-hydroxy-2-[(2-iodophényl)imino]imidazolidine.

15. 4-[/2-[(2,6-dichlorophényl)imino]-1-imidazolidinyl/oxy]butyrate d'éthyle.

16. Acide 4-[/2-[(2,6-dichlorophényl)imino]-1-imidazolidinyl/oxy]-butyrique.

17. 2-[/2-[(2,6-dichlorophényl)imino]-1-imidazolidinyl/oxy]butyrate d'éthyle.

18. 2-[(2,6-dichlorphényl)imino]-1-(2-propinyloxy)imidazolidine.

19. Acide [/2-[(2,6-dichlorophényl)imino]-1-imidazolidinyl/oxy]-acétique.

20. 4-[/2-[(2,6-dichlorophényl)imino]-1-imidazolidinyl/oxy]crotonate d'éthyle.

21. 2-[(2,6-dichlorophényl)imino]-1-éthoxyimidazolidine.

22. 1-(2-butynyloxy)-2-[(2,6-dichlorophényl)imino]imidazolidine.

23. 2-[(2,6-dichlorophényl)imino]-1-(furfuryloxy)imidazolidine.
24. 2-[/⟨2-[(2,6-dichlorophényl)imino]-1-imidazolinyl⟩-oxy]méthyl/pyridine.
25. 3-[/⟨2-[(2,6-dichlorophényl)imino]-1-imidazolinyl⟩oxy]méthyl/pyridine.
26. 2-[/⟨2-[(2,6-dichloro-4-fluorophényl)imino]-1-imidazolinyl⟩oxy]méthyl/pyridine.
27. 5-[/⟨2-[(2,6-dichlorophényl)imino]-1-imidazolinyl⟩oxy]méthyl/-3-hydroxy-2-méthyl-4-pyridine-méthanol.
28. Sels des composés selon les revendications 12—15, 17, 18 et 20—27.
29. Composés de formule générale

$$H_2N-CH_2-CH_2-NH-OR^4 \qquad IV$$

où $R^4$ représente un hydrogène, un alcoyle en $C_1$ à $C_6$, un cycloalcoyle en $C_3$ à $C_6$, un alcényle en $C_2$ à $C_6$, un alcynyle en $C_2$ à $C_6$, un di-alcoylamino en $C_1$ à $C_6$-alcoyle en $C_1$ à $C_6$, un carboxy-alcoyle en $C_1$ à $C_6$, un carboxy-alcényle en $C_2$ à $C_6$, un hydroxy-alcoyle en $C_1$ à $C_6$, un cyano-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxycarbonyle en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un aminocarbonyl-alcoyle en $C_1$ à $C_6$, un mono-alcoylaminocarbonyle en $C_1$ à $C_6$-alcoyle en $C_1$ à $C_6$, un di-alcoylaminocarbonyle en $C_1$ à $C_6$-alcoyle en $C_1$ à $C_6$, un alcoxycarbonyle en $C_1$ à $C_6$-alcényle en $C_2$ à $C_6$, un alcoxy en $C_1$ à $C_6$-alcoyle en $C_1$ à $C_6$, un amino-alcoyle en $C_1$ à $C_6$, un monoalcoylamino en $C_1$ à $C_6$-alcoyle en $C_1$ à $C_6$, un di-alcoylamino en $C_1$ à $C_6$-alcoyle en $C_1$ à $C_6$, un (2,2,8-triméthyl-4H-m-dioxino[4,5-c]pyridin-5-yl)méthyle, un [5-hydroxy-4-(hydroxyméthyl)-6-méthyl-3-pyridyl]méthyle, un $\alpha$-carboxybenzyle, un $\alpha$-alcoxycarbonyle en $C_1$ à $C_6$-$\alpha$-alcoylphényle en $C_1$ à $C_6$ ou un radical thiényle, furyle, ou isoxazolyle lié par l'intermédiaire d'un groupe $-CH(R^5)$ et éventuellement substitué par un alcoyle en $C_1$ à $C_6$ ou le groupe $-COOR$ ou un radical pyridyle lié par l'intermédiaire d'un groupe $-CH(R^5)$ et éventuellement substitué par un alcoyle en $C_1$ à $C_6$, un alcoxy en $C_1$ à $C_6$ ou le groupe $-COOR$ et $R^5$ représente un hydrogène, un méthyle, un éthyle ou un n-propyle et R représente un hydrogène ou un alcoyle en $C_1$ à $C_6$,
lorsqu'on les utilise pour préparer des composés selon la revendication 1.
30. Composés de formule générale

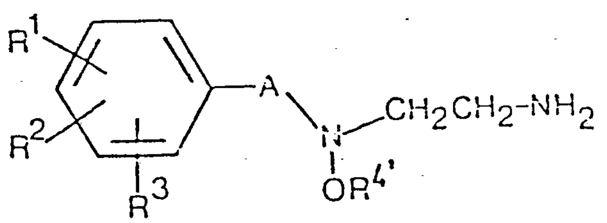

V

où A représente le groupe

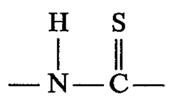

ou

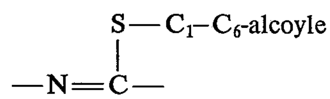

$R^1$, $R^2$ et $R^3$ représentent chacun indépendamment l'un de l'autre un hydrogène, un alcoyle en $C_1$ à $C_6$, un alcoxy en $C_1$ à $C_6$, un alcoylthio en $C_1$ à $C_6$, un halogène, un trifluorométhyle, un cyano ou un hydroxy et $R^{4'}$ représente un alcoyle en $C_1$ à $C_6$, un cycloalcoyle en $C_3$ à $C_6$, un alcényle en $C_2$ à $C_6$, un dialcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ et $R^5$ représente un hydrogène, un méthyle, un éthyle ou un n-propyle,
lorsqu'on les utilise pour préparer des composés selon la revendication 1.
31. Composés de formule générale

$$Z-CH_2CH_2-HN-OR^{4'} \qquad XII$$

où $R^{4'}$ représente un alcoyle en $C_1$ à $C_6$, un cycloalcoyle en $C_3$ à $C_6$, un alcényle en $C_2$ à $C_6$, un di-alcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement

substitué par un alcoyle en $C_1$ à $C_6$ et $R^5$ représente un hydrogène, un méthyle, un éthyle ou un n-propyle et Z représente un groupe phthalimido ou succinimido,
lorsqu'on les utilise pour préparer des composés selon la revendication 1.

32. Composés de formule générale

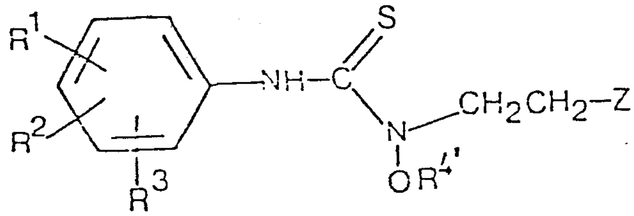

XIII

où $R^1$, $R^2$ et $R^3$ représentent chacun indépendamment l'un de l'autre un hydrogène, un alcoyle en $C_1$ à $C_6$, un alcoxy en $C_1$ à $C_6$, un alcoylthio en $C_1$ à $C_6$, un halogène, un trifluorométhyle, un cyano ou un hydroxy et $R^{4'}$ représente un alcoyle en $C_1$ à $C_6$, un cycloalcoyle en $C_3$ à $C_6$, un alcényle en $C_2$ à $C_6$, un dialcoylamino en $C_1$ à $C_6$-alcoyle en $C_1$ à $C_6$, un phényl-alcoyle en $C_1$ à $C_6$, un phénylalcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ et $R^5$ représente un hydrogène, un méthyle, un éthyle ou un n-propyle et Z représente un groupe phtalimido ou succinimido,
lorsqu'on les utilise pour préparer des composés selon la revendication 1.

33. Composés de formule générale

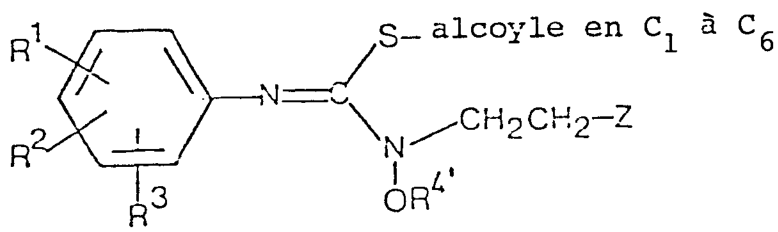

XIV

où $R^1$, $R^2$, et $R^3$ représentent chacun indépendamment l'un de l'autre un hydrogène, un alcoyle en $C_1$ à $C_6$, un alcoxy en $C_1$ à $C_6$, un alcoylthio en $C_1$ à $C_6$, un halogène, un trifluorométhyle, un cyano ou un hydroxy et $R^{4'}$ représente un alcoyle en $C_1$ à $C_6$, un cycloalcoyle en $C_3$ à $C_6$, un alcényle en $C_2$ à $C_6$, un dialcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ et $R^5$ représente un hydrogène, un méthyle, un éthyle ou un n-propyle et Z représente un groupe phtalimido ou succinimido,
lorsqu'on les utilise pour préparer des composés selon la revendication 1.

34. Procédé de préparation de dérivés de 2-imino-imidazolidine de formule générale I définie dans la revendication 1 et de leurs sels d'addition acide pharmaceutiquement acceptables, caractérisé en ce que

a) on fait réagir un composé de formule générale

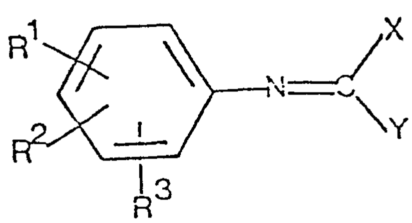

II

où X et Y représentent chacun un halogène, un groupe sulfhydryle, amino, méthoxy ou alcoylthio ayant de 1 à 3 atomes de carbone et $R^1$, $R^2$ et $R^3$ ont la signification donnée dans la revendication 1, ou un composé de formule générale

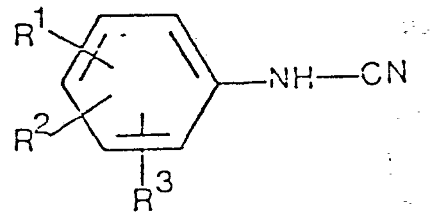

III

où $R^1$, $R^2$ et $R^3$ ont la signification donnée dans la revendication 1, avec un composé de formule générale

$$H_2N-CH_2-CH_2-NH-OR^4 \qquad IV$$

où $R^4$ a la signification donnée dans la revendication 1,

ou

b) pour préparer des composés de formule I où $R^4$ représente un alcoyle en $C_1$ à $C_6$, un cycloalcoyle en $C_3$ à $C_6$, un alcényle en $C_2$ à $C_6$, un di-alcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ et $R^1$, $R^2$, $R^3$ et $R^5$ ont la signification donnée dans la revendication 1, on cyclise un composé de formule générale

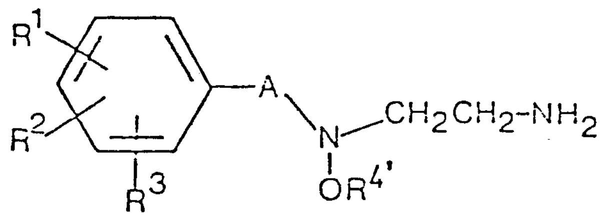

V

où A représente le groupe

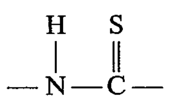

ou

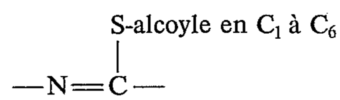

et $R^{4'}$ représente un alcoyle en $C_1$ à $C_6$, un cycloalcoyle en $C_3$ à $C_6$, un alcényle en $C_2$ à $C_6$, un di-alcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle, ou pyridyle lié par l'intermédiaire d'un groupe $-CH(R^5)$ et éventuellement substitué par un alcoyle en $C_1$ à $C_6$ et $R^1$, $R^2$, $R^3$ et $R^5$ ont la signification donnée dans la revendication 1,

ou

c) pour préparer des composés de formule I où $R^4$ représente un hydrogène et $R^1$, $R^2$ et $R^3$ ont la signification donnée dans la revendication 1, on sépare le groupe benzyle dans un composé de formule générale

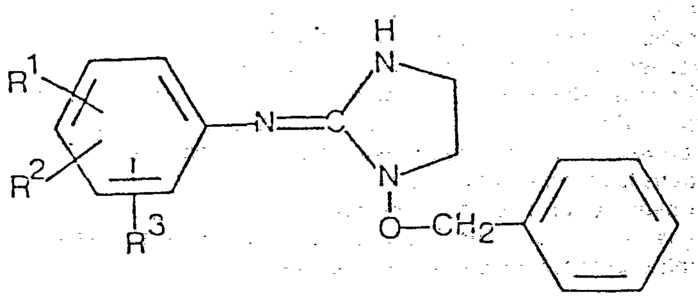

Ia

où $R^1$, $R^2$ et $R^3$ ont la signification donnée dans la revendication 1,

ou

d) pour préparer des composés de formule générale

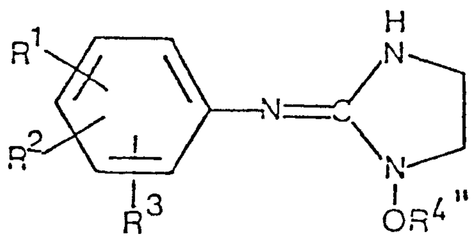

Ib

où $R^{4''}$ représente un alcoyle en $C_1$ à $C_6$, un cycloalcoyle en $C_3$ à $C_6$, un alcényle en $C_2$ à $C_6$, un alcynyle en $C_2$ à $C_6$, un di-alcoylamino en $C_1$ à $C_6$-alcoyle en $C_1$ à $C_6$, un carboxy-alcoyle en $C_1$ à $C_6$, un hydroxy-alcoyle en $C_1$ à $C_6$, un cyano-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxycarbonyle en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un aminocarbonyl-alcoyle en $C_1$ à $C_6$, un mono-alcoylaminocarbonyle en $C_1$ à $C_6$-alcoyle en $C_1$ à $C_6$, un di-alcoylaminocarbonyle en $C_1$ à $C_6$-alcoyle en $C_1$ à $C_6$, un alcoxycarbonyle en $C_1$ à $C_6$-alcényle en $C_2$ à $C_6$, un alcoxy en $C_1$ à $C_6$-alcoyle en $C_1$ à $C_6$, un amino-alcoyle en $C_1$ à $C_6$, un mono-alcoylamino en $C_1$ à $C_6$-alcoyle en $C_1$ à $C_6$, un di-alcoylamino en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ ou le groupe $-COOR$ et R, $R^1$, $R^2$, $R^3$ et $R^5$ ont la signification donnée dans la revendication 1, on fait réagir un composé de formule générale

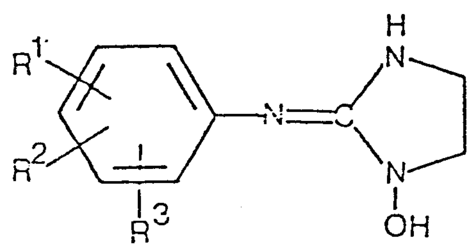

Ic

où $R^1$, $R^2$ et $R^3$ ont la signification donnée dans la revendication 1, avec un composé de formule générale

$$R^{4''}-X \qquad VI$$

où X représente un groupe sortant et $R^{4''}$ a la signification donnée ci-dessus, ou

e) pour préparer des composés de formule I où $R^4$ représente un carboxy-alcoyle en $C_1$ à $C_6$ ou un carboxyalcényle en $C_2$ à $C_6$ et $R_1$, $R_2$ et $R_3$ ont la signification donnée dans la revendication 1, on hydrolyse un composé de formule I correspondant, où $R^4$ représente un alcoxycarbonyle en $C_1$ à $C_6$-alcoyle en $C_1$ à $C_6$ ou un alcoxycarbonyle en $C_1$ à $C_6$-alcényle en $C_2$ à $C_6$ et $R^1$, $R^2$ et $R^3$ ont la signification donnée dans la revendication 1, ou

f) pour préparer des composés de formule I, où $R^4$ représente un alcoxycarbonyle en $C_1$ à $C_6$-alcoyle en $C_1$ à $C_6$ ou un alcoxycarbonyle en $C_1$ à $C_6$-alcényle en $C_2$ à $C_6$ et $R^1$, $R^2$ et $R^3$ ont la signification donnée dans la revendication 1, on transestérifie avec un alcanol en $C_1$ à $C_6$ un composé de formule I correspondant où $R^4$ représente un alcoxycarbonyle en $C_1$ à $C_6$-alcoyle en $C_1$ à $C_6$ ou un alcoxycarbonyle en $C_1$ à $C_6$-alcényle en $C_2$ à $C_6$, où cependant le radical alcoxy est différent, et où $R^1$, $R^2$ et $R^3$ ont la signification donnée dans la revendication 1, où

g) pour préparer des composés de formule I ci-dessus, où $R^4$ représente un aminocarbonyl-alcoyle en $C_1$ à $C_6$ et $R^1$, $R^2$ et $R^3$ ont la signification donnée dans la revendication 1, on fait réagir avec de l'ammoniac, une amine primaire ou une amine secondaire un composé de formule I correspondant, où $R^4$ représente un alcoxy-carbonyle en $C_1$ à $C_6$-alcoyle en $C_1$ à $C_6$ et $R^1$, $R^2$ et $R^3$ ont la signification donnée dans la revendication 1, ou

h) pour préparer des composés de formule I où $R^4$ représente un hydrogène et où $R^1$, $R^2$ et $R^3$ ont la signification donnée dans la revendication 1, on oxyde avec du persulfate de potassium dans l'acide sulfurique concentré un composé de formule générale

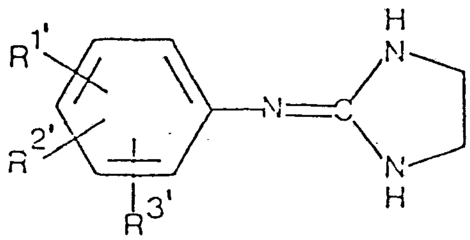

VII

où $R^{1'}$, $R^{2'}$, $R^{3'}$ représentent chacun indépendamment l'un de l'autre un hydrogène, un alcoyle en $C_1$ à $C_6$, un alcoxy en $C_1$ à $C_6$, un halogène ou un trifluorométhyle, ou

i) pour préparer des composés de formule I ci-dessus, où $R^4$ représente un [5-hydroxy-4-(hydroxyméthyl)-6-méthyl-3-pyridyl]-méthyle et $R^1$, $R^2$ et $R^3$ ont la signification donnée dans la revendication 1, on sépare le groupe cétal dans un composé de formule générale

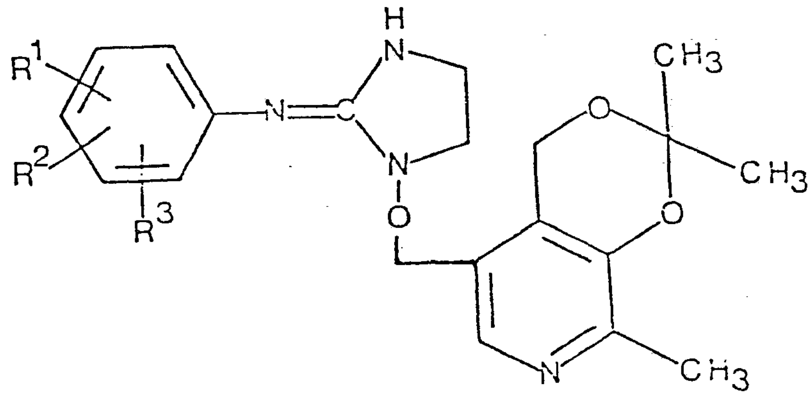

Id

où $R^1$, $R^2$ et $R^3$ ont la signification donnée dans la revendication 1, et

j) si on le désire on transforme un composé obtenu ou un sel d'addition acide de ce corps non pharmaceutiquement acceptable en un sel d'addition acide pharmaceutiquement acceptable de ce corps.

35. Procédé selon la revendication 34 de préparation de composés de formule I définie dans la revendication 2 ou de leurs sels d'addition acide pharmaceutiquement acceptables, caractérisé en ce qu'on effectue les variantes a), b), c), d), e), f), g) et j) du procédé de la revendication 34.

36. Procédé de préparation de préparations ayant des propriétés d'abaissement de la tension artérielle, caractérisé en ce qu'on mélange un dérivé de 2-imino-imidazolidine de formule I de la revendication 1 ou un de ses sels d'addition acide pharmaceutiquement acceptable comme composant actif avec des supports et/ou excipients solides et liquides appropriés à l'administration thérapeutique, non toxiques, inertes, habituels dans de telles préparations.

37. Préparations à effet d'abaissement de la tension artérielle contenant un dérivé de 2-imino-imidazolidine de formule I de la revendication 1 ou un de ses sels d'addition acide pharmaceutiquement acceptables et un support.

**Revendications pour l'Etat contractant: DE**

1. Dérivés de 2-imino-imidazolidine de formule générale

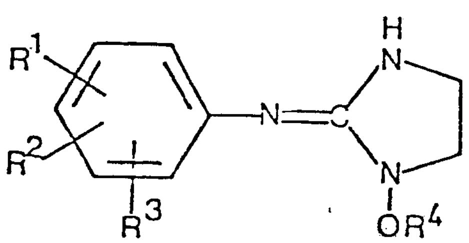

I

où $R^1$, $R^2$ et $R^3$ représentent chacun indépendamment l'un de l'autre un hydrogène, un alcoyle en $C_1$ à $C_6$, un alcoxy en $C_1$ à $C_6$, un alcoylthio en $C_1$ à $C_6$, un halogène, un trifluorométhyle, un cyano ou un hydroxy et $R^4$ représente un hydrogène, un alcoyle en $C_1$ à $C_6$, un cycloalcoyle en $C_3$ à $C_6$, un alcényle en $C_2$ à $C_6$, un alcynyle en $C_2$ à $C_6$, un di-alcoylamino en $C_1$ à $C_6$-alcoyle en $C_1$ à $C_6$, un carboxyalcoyle en $C_1$ à $C_6$, un carboxy-alcényle en $C_2$ à $C_6$, un hydroxy-alcoyle en $C_1$ à $C_6$, un cyano-alcoyle en $C_1$ à $C_6$, un

phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxycarbonyle en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un aminocarbonyl-alcoyle en $C_1$ à $C_6$, un mono-alcoylaminocarbonyle en $C_1$ à $C_6$-alcoyle en $C_1$ à $C_6$, un di-alcoylaminocarbonyle en $C_1$ à $C_6$-alcoyle en $C_1$ à $C_6$, un alcoxycarbonyle en $C_1$ à $C_6$-alcényle en $C_2$ à $C_6$, un alcoxy en $C_1$ à $C_6$-alcoyle en $C_1$ à $C_6$, un amino-alcoyle en $C_1$ à $C_6$, un mono-alcoylamino en $C_1$ à $C_6$-alcoyle en $C_1$ à $C_6$, un dialcoylamino en $C_1$ à $C_6$-alcoyle en $C_1$ à $C_6$, un (2,2,8-triméthyl-4H-m-dioxino-[4,5-c]-pyridin-5-yl)méthyle, un [5-hydroxy-4-(hydroxyméthyl)-6-méthyl-3-pyridyl]méthyle, un $\alpha$-carboxybenzyle, un $\alpha$-alcoxycarbonyle en $C_1$ à $C_6$-$\alpha$-alcoylphényle en $C_1$ à $C_6$ ou un radical thiényle, furyle ou isoxazolyle lié par l'intermédiaire d'un groupe $-CH(R^5)$ et éventuellement substitué par un alcoyle en $C_1$ à $C_6$ ou le groupe $-COOR$ ou un radical pyridyle lié par l'intermédiaire d'un groupe $-CH(R^5)$ et éventuellement substitué par un alcoyle en $C_1$ à $C_6$, un alcoxy en $C_1$ à $C_6$ ou le groupe $-COOR$ et $R^5$ représente un hydrogène, un méthyle, un éthyle, ou un n-propyle et R représente un hydrogène ou un alcoyle en $C_1$ à $C_6$, $R^4$ étant différent d'un hydrogène, d'un alcoyle en $C_1$ à $C_6$, d'un hydroxy-alcoyle en $C_1$ à $C_6$, d'un benzyle, d'un phénéthyle et d'un alcoxy en $C_1$ à $C_6$-alcoyle en $C_1$ à $C_6$ en cas que $R^1$, $R^2$ et $R^3$ représentent chacun indépendamment l'un de l'autre un hydrogène, un méthyle ou un halogène, ainsi que leurs sels d'addition acide pharmaceutiquement acceptables.

2. Composés selon la revendication 1 où $R^4$ représente un hydrogène, un alcoyle en $C_1$ à $C_6$, un cycloalcoyle en $C_3$ à $C_6$, un alcényle en $C_2$ à $C_6$, un alcynyle en $C_2$ à $C_6$, un di-alcoylamino en $C_1$ à $C_6$-alcoyle en $C_1$ à $C_6$, un carboxy-alcoyle en $C_1$ à $C_6$, un carboxy-alcényle en $C_2$ à $C_6$, un hydroxy-alcoyle en $C_1$ à $C_6$, un cyano-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxycarbonyle en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un aminocarbonyl-alcoyle en $C_1$ à $C_6$, un alcoxycarbonyle en $C_1$ à $C_6$-alcényle en $C_2$ à $C_6$, un alcoxy en $C_1$ à $C_6$-alcoyle en $C_1$ à $C_6$, un amino-alcoyle en $C_1$ à $C_6$, un mono-alcoylamino en $C_1$ à $C_6$-alcoyle en $C_1$ à $C_6$, un di-alcoylamino en $C_1$ à $C_6$-alcoyle en $C_1$ à $C_6$, un amino-alcoyle en $C_1$ à $C_6$, un $\alpha$-carboxybenzyle, un $\alpha$-alcoxy-carbonyle en $C_1$ à $C_6$-$\alpha$-alcoylphényle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ ou le groupe $-COOR$, et $R^1$, $R^2$, $R^3$, $R^5$ et R possèdent la signification donnée dans la revendication 1.

3. Composés selon la revendication 1 ou la revendication 2, où $R^3$ représente un hydrogène.

4. Composés selon l'une des revendications 1 à 3, où $R^1$ et $R^2$ sont localisés en position 2,6 du noyau phényle.

5. Composés selon l'une des revendications 1 à 4, où $R^1$ et $R^2$ ont la même signification.

6. Composés selon l'une des revendications 1 à 5, où $R^1$ et $R^2$ représentent un halogène.

7. Composés selon la revendication 6, où $R^1$ et $R^2$ représentent un chlore.

8. Composés selon l'une des revendications 1 à 7, où $R^4$ représente un alcynyle en $C_2$ à $C_6$, un carboxyalcoyle en $C_1$ à $C_6$, un alcoxycarbonyle en $C_1$ à $C_6$-alcényle en $C_2$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ ou le groupe $-COOR$ et $R^5$ représente un hydrogène, un méthyle, un éthyle ou un n-propyle et R représente un hydrogène ou un alcoyle en $C_1$ à $C_6$.

9. Composés selon la revendication 8, où $R^4$ représente un alcynyle en $C_3$ à $C_5$, un carboxyméthyle, un carboxypropyle, un éthoxycarbonyl-alcényle en $C_2$ à $C_6$ ou un radical pyridyle ou un radical furyle ou thiényle non substitué lié par l'intermédiaire d'un groupe $-CH(R^5)$ et $R^5$ représente un hydrogène ou un méthyle.

10. Composés selon la revendication 9, où $R^4$ représente un propargyle, un carboxyméthyle, un éthoxycarbonyl-alcényle en $C_2$ à $C_6$ non ramifié ou un pyridyle ou un furyle lié par l'intermédiaire d'un groupe $-CH(R^5)$.

11. Composés selon l'une des revendications 1 à 10, où $R^3$ représente un hydrogène, $R^1$ et $R^2$ un halogène, de préférence le chlore, en position 2,6, $R^4$ un propargyle, un carboxyméthyle, un éthoxycarbonyl-alcényle en $C_2$ à $C_6$ non ramifié ou un pyridyle ou un furyle lié par l'intermédiaire d'un groupe $-CH(R^5)$ et $R^5$ représente un hydrogène ou un méthyle.

12. 4-[/2-[(2,6-dichlorophényl-imino]-1-imidazolidinyl/oxy]butyrate d'éthyle.

13. Acide 4-[/2-[(2,6-dichlorophényl)imino]-1-imidazolidinyl/oxy]-butyrique.

14. 2-[/2-[(2,6-dichlorophényl)imino]-1-imidazolidinyl/oxy]butyrate d'éthyle.

15. 2-[(2,6-dichlorophényl)imino]-1-(2-propinyloxy)imidazolidine.

16. Acide [/2-[(2,6-dichlorophényl)imino]-1-imidazolidinyl/oxy]-acétique.

17. 4-[/2-[(2,6-dichlorophényl)imino]-1-imidazolidinyl/oxy]crotonate d'éthyle.

18. 1-(2-butynyloxy)-2-[(2,6-dichlorophényl)imino]imidazolidine.

19. 2-[(2,6-dichlorophényl)imino]-1-(furfuryloxy)imidazolidine.

20. 2-[/⟨2-[(2,6-dichlorophényl)imino]-1-imidazolinyl⟩-oxy]méthyl/pyridine.

21. 3-[/⟨2-[(2,6-dichlorophényl)imino]-1-imidazolinyl⟩oxy]méthyl/pyridine.

22. 2-[/⟨2-[(2,6-dichloro-4-fluorophényl)imino]-1-imidazolinyl⟩oxy]méthyl/pyridine.

23. 5-[/⟨2-[(2,6-dichlorophényl)imino]-1-imidazolinyl⟩oxy]méthyl/-3-hydroxy-2-méthyl-4-pyridine-méthanol.

24. Sels des composés selon les revendications 12, 14, 15 et 17–23.

25. Composés de formule générale

$H_2N-CH_2-CH_2-NH-OR^4$ IV

où $R^4$ représente un hydrogène, un cycloalcoyle en $C_3$ à $C_6$, un alcényle en $C_2$ à $C_6$, un alcynyle en $C_2$ à $C_6$, un di-alcoylamino en $C_1$ à $C_6$-alcoyle en $C_1$ à $C_6$, un carboxy-alcoyle en $C_1$ à $C_6$, un carboxy-alcényle en $C_2$ à $C_6$, un cyano-alcoyle en $C_1$ à $C_6$, un phényl-alcoyle en $C_3$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxycarbonyle en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un aminocarbonyl-alcoyle en $C_1$ à $C_6$, un monoalcoylaminocarbonyle en $C_1$ à $C_6$-alcoyle en $C_1$ à $C_6$, un di-alcoylaminocarbonyle en $C_1$ à $C_6$-alcoyle en $C_1$ à $C_6$, un alcoxycarbonyle en $C_1$ à $C_6$-alcényle en $C_2$ à $C_6$, un amino-alcoyle en $C_1$ à $C_6$, un monoalcoylamino en $C_1$ à $C_6$-alcoyle en $C_1$ à $C_6$, un di-alcoylamino en $C_1$ à $C_6$-alcoyle en $C_1$ à $C_6$, un (2,2,8-triméthyl-4H-m-dioxino[4,5-c]pyridin-5-yl)méthyle, un [5-hydroxy-4-(hydroxyméthyl)-6-méthyl-3-pyridyl]méthyle, un $\alpha$-carboxybenzyle, un $\alpha$-alcoxycarbonyle en $C_1$ à $C_6$-$\alpha$-alcoylphényle en $C_1$ à $C_6$ ou un radical thiényle, furyle, ou isoxazolyle lié par l'intermédiaire d'un groupe $-CH(R^5)$ et éventuellement substitué par un alcoyle en $C_1$ à $C_6$ ou le groupe $-COOR$ ou un radical pyridyle lié par l'intermédiaire d'un groupe $-CH(R^5)$ et éventuellement substitué par un alcoyle en $C_1$ à $C_6$, un alcoxy en $C_1$ à $C_6$ ou le groupe $-COOR$ et $R^5$ représente un hydrogène, un méthyle, un éthyle ou un n-propyle et R représente un hydrogène ou un alcoyle en $C_1$ à $C_6$, lorsqu'on les utilise pour préparer des composés selon la revendication 1.

26. Composés de formule générale

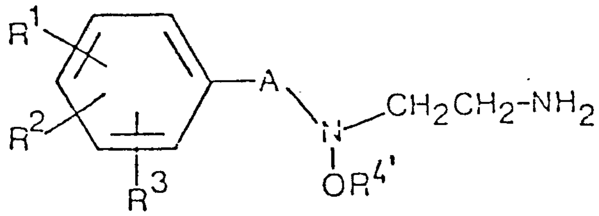

V

où A représente le groupe

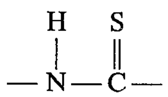

ou

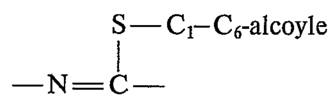

$R^1$, $R^2$ et $R^3$ représentent chacun indépendamment l'un de l'autre un hydrogène, un alcoyle en $C_1$ à $C_6$, un alcoxy en $C_1$ à $C_6$, un alcoylthio en $C_1$ à $C_6$, un halogène, un trifluorométhyle, un cyano ou un hydroxy et $R^{4'}$ représente un alcoyle en $C_1$ à $C_6$, un cycloalcoyle en $C_3$ à $C_6$, un alcényle en $C_2$ à $C_6$, un dialcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ et $R^5$ représente un hydrogène, un méthyle, un éthyle ou un n-propyle, $R^{4'}$ étant différent d'un alcoyle en $C_1$ à $C_6$, d'un benzyle, d'un phénéthyle ou d'un alcoxy en $C_1$ à $C_6$-alcoyle en $C_1$ à $C_6$ en cas que $R^1$, $R^2$ et $R^3$ représentent chacun indépendamment l'un de l'autre un hydrogène, un méthyle ou un halogène, lorsqu'on les utilise pour préparer des composés selon la revendication 1.

27. Composés de formule générale

$Z-CH_2CH_2-HN-OR^{4'}$ XII

où $R^{4'}$ représente un alcoyle en $C_1$ à $C_6$, un cycloalcoyle en $C_3$ à $C_6$, un alcényle en $C_2$ à $C_6$, un di-alcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ et $R^5$ représente un hydrogène, un méthyle, un éthyle ou un n-propyle et Z représente un groupe phtalimido ou succinimido, lorsqu'on les utilise pour préparer des composés selon la revendication 1.

28. Composés de formule générale

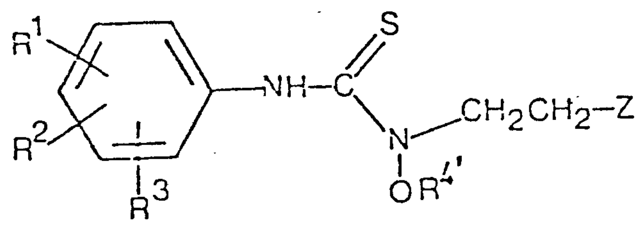

XIII

où $R^1$, $R^2$ et $R^3$ représentent chacun indépendamment l'un de l'autre un hydrogène, un alcoyle en $C_1$ à $C_6$, un alcoxy en $C_1$ à $C_6$, un alcoylthio en $C_1$ à $C_6$, un halogène, un trifluorométhyle, un cyano ou un hydroxy et $R^{4'}$ représente un alcoyle en $C_1$ à $C_6$, un cycloalcoyle en $C_3$ à $C_6$, un alcényle en $C_2$ à $C_6$, un dialcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ et $R^5$ représente un hydrogène, un méthyle, un éthyle ou un n-propyle et Z représente un groupe phtalimido ou succinimido, $R^{4'}$ étant différent d'un alcoyle en $C_1$ à $C_6$, d'un benzyle, d'un phénéthyle ou d'un alcoxy en $C_1$ à $C_6$-alcoyle en $C_1$ à $C_6$ en cas que $R^1$, $R^2$ et $R^3$ représentent chacun indépendamment l'un de l'autre un hydrogène, un méthyle ou un halogène,

lorsqu'on les utilise pour préparer des composés selon la revendication 1.

29. Composés de formule générale

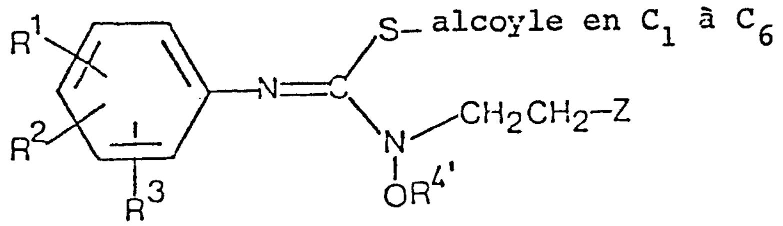

XIV

où $R^1$, $R^2$, et $R^3$ représentent chacun indépendamment l'un de l'autre un hydrogène, un alcoyle en $C_1$ à $C_6$, un alcoxy en $C_1$ à $C_6$, un alcoylthio en $C_1$ à $C_6$, un halogène, un trifluorométhyle, un cyano ou un hydroxy et $R^{4'}$ représente un alcoyle en $C_1$ à $C_6$, un cycloalcoyle en $C_3$ à $C_6$, un alcényle en $C_2$ à $C_6$, un dialcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ et $R^5$ représente un hydrogène, un méthyle, un éthyle ou un n-propyle et Z représente un groupe phtalimido ou succinimido, $R^{4'}$ étant différent d'un alcoyle en $C_1$ à $C_6$, d'un benzyle, d'un phénéthyle ou d'un alcoxy en $C_1$ à $C_6$-alcoyle en $C_1$ à $C_6$ en cas que $R^1$, $R^2$ et $R^3$ représentent chacun indépendamment l'un de l'autre un hydrogène, un méthyle ou un halogène,

lorsqu'on les utilise pour préparer des composés selon la revendication 1.

30. Procédé de préparation de dérivés de 2-imino-imidazolidine de formule générale I définie dans la revendication 1 et de leurs sels d'addition acide pharmaceutiquement acceptables, caractérisé en ce que

a) on fait réagir un composé de formule générale

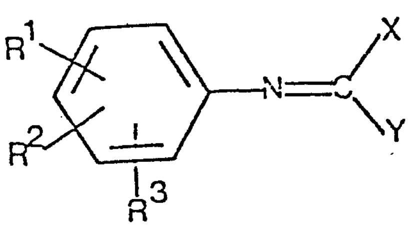

II

où X et Y représentent chacun un halogène, un groupe sulfhydryle, amino, méthoxy ou alcoylthio ayant de 1 à 3 atomes de carbone et $R^1$, $R^2$ et $R^3$ ont la signification donnée dans la revendication 1, ou un composé de formule générale

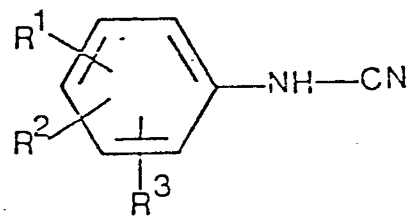

III

où $R^1$, $R^2$ et $R^3$ ont la signification donnée dans la revendication 1, avec un composé de formule générale

$$H_2N-CH_2-CH_2-NH-OR^4 \qquad IV$$

où $R^4$ a la signification donnée dans la revendication 1,
ou

b) pour préparer des composés de formule I où $R^4$ représente un alcoyle en $C_1$ à $C_6$, un cycloalcoyle en $C_3$ à $C_6$, un alcényle en $C_2$ à $C_6$, un di-alcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ et $R^1$, $R^2$, $R^3$ et $R^5$ ont la signification donnée dans la revendication 1, on cyclise un composé de formule générale

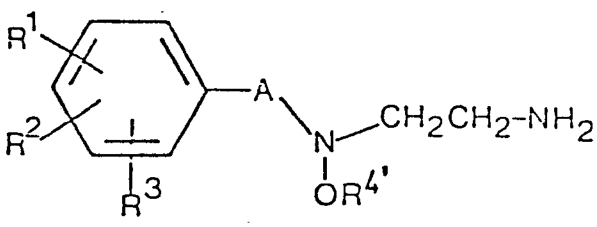

V

où A représente le groupe

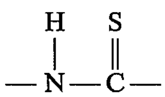

ou

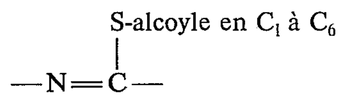

et $R^{4'}$ représente un alcoyle en $C_1$ à $C_6$, un cycloalcoyle en $C_3$ à $C_6$, un alcényle en $C_2$ à $C_6$, un di-alcoylamino en $C_1$ à $C_6$-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un alcoxy en $C_1$ à $C_6$-alcoyle en $C_1$ à $C_6$ ou un radical thiényle, furyle, isoxazolyle, ou pyridyle lié par l'intermédiaire d'un groupe $-CH(R^5)$ et éventuellement substitué par un alcoyle en $C_1$ à $C_6$ et $R^1$, $R^2$, $R^3$ et $R^5$ ont la signification donnée dans la revendication 1, ou

c) pour préparer des composés de formule I où $R^4$ représente un hydrogène et $R^1$, $R^2$ et $R^3$ ont la signification donnée dans la revendication 1, on sépare le groupe benzyle dans un composé de formule générale

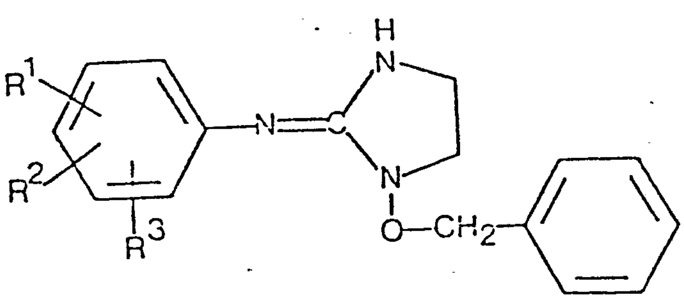

Ia

où $R^1$, $R^2$ et $R^3$ ont la signification donnée dans la revendication 1,
ou

d) pour préparer des composés de formule générale

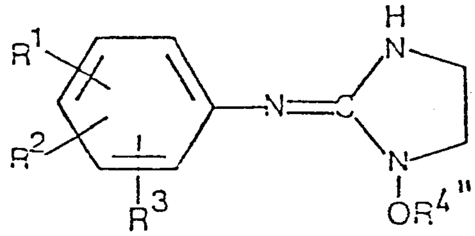

Ib

où $R^{4''}$ représente un alcoyle en $C_1$ à $C_6$, un cycloalcoyle en $C_3$ à $C_6$, un alcényle en $C_2$ à $C_6$, un alcynyle en $C_2$ à $C_6$, un di-alcoylamino en $C_1$ à $C_6$-alcoyle en $C_1$ à $C_6$, un carboxy-alcoyle en $C_1$ à $C_6$, un hydroxy-alcoyle en $C_1$ à $C_6$, un cyano-alcoyle en $C_1$ à $C_6$, un phényl-alcoyle en $C_1$ à $C_6$, un phényl-alcoyle en $C_1$ à $C_6$ substitué dans le noyau phényle par des groupes méthoxy, un alcoylthio en $C_1$ à $C_6$-alcoyle en $C_1$ à $C_6$, un alcoxycarbonyle en $C_1$ à $C_6$-alcoyle en $C_1$ à $C_6$, un alcoxy en $C_1$ à $C_6$-alcoxy en $C_1$ à $C_6$-alcoyle en $C_1$ à $C_6$, un amino-carbonyl-alcoyle en $C_1$ à $C_6$, un mono-alcoylaminocarbonyle en $C_1$ à $C_6$-alcoyle en $C_1$ à $C_6$, un di-alcoylaminocarbonyle en $C_1$ à $C_6$-alcoyle en $C_1$ à $C_6$, un alcoxycarbonyle en $C_1$ à $C_6$-alcényle en $C_2$ à $C_6$, un alcoxy en $C_1$ à $C_6$-alcoyle en $C_1$ à $C_6$, un amino-alcoyle en $C_1$ à $C_6$, un mono-alcoylamino en $C_1$ à $C_6$-alcoyle en $C_1$ à $C_6$, un di-alcoylamino en $C_1$ à $C_6$-alcoyle en $C_1$ à $C_6$ ou un radical thiényle, furyle, isoxazolyle ou pyridyle lié par l'intermédiaire d'un groupe $-CH(R^5)$ et éventuellement substitué par un alcoyle en $C_1$ à $C_6$ ou le groupe $-COOR$ et R, $R^1$, $R^2$, $R^3$ et $R^5$ ont la signification donnée dans la revendication 1, on fait réagir un composé de formule générale

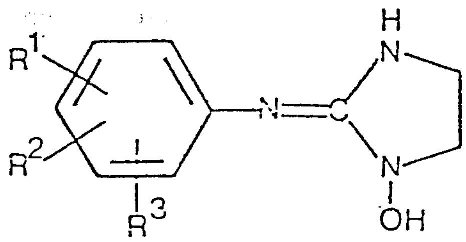

Ic

où $R^1$, $R^2$ et $R^3$ ont la signification donnée dans la revendication 1, avec un composé de formule générale

$R^{4''}-X$ VI

où X représente un groupe sortant et $R^{4''}$ a la signification donnée ci-dessus,
ou

e) pour préparer des composés de formule I où $R^4$ représente un carboxy-alcoyle en $C_1$ à $C_6$ ou un carboxyalcényle en $C_2$ à $C_6$ et $R_1$, $R_2$ et $R_3$ ont la signification donnée dans la revendication 1, on hydrolyse un composé de formule I correspondant, où $R^4$ représente un alcoxycarbonyle en $C_1$ à $C_6$-alcoyle en $C_1$ à $C_6$ ou un alcoxycarbonyle en $C_1$ +a $C_6$-alcényle en $C_2$ à $C_6$ et $R^1$, $R^2$ et $R^3$ ont la signification donnée dans la revendication 1, ou

f) pour préparer des composés de formule I, où $R^4$ représente un alcoxycarbonyle en $C_1$ à $C_6$-alcoyle en $C_1$ à $C_6$ ou un alcoxycarbonyle en $C_1$ à $C_6$-alcényle en $C_2$ à $C_6$ et $R^1$, $R^2$ et $R^3$ ont la signification donnée dans la revendication 1, on transestérifie avec un alcanol en $C_1$ à $C_6$ un composé de formule I correspondant où $R^4$ représente un alcoxycarbonyle en $C_1$ à $C_6$-alcoyle en $C_1$ à $C_6$ ou un alcoxycarbonyle en $C_1$ à $C_6$-alcényle en $C_2$ à $C_6$, où cependant le radical alcoxy est différent, et où $R^1$, $R^2$ et $R^3$ ont la signification donnée dans la revendication 1, où

g) pour préparer des composés de formule I ci-dessus, où $R^4$ représente un aminocarbonyl-alcoyle en $C_1$ à $C_6$ et $R^1$, $R^2$ et $R^3$ ont la signification donnée dans la revendication 1, on fait réagir avec de l'ammoniac, une amine primaire ou une amine secondaire un composé de formule I correspondant, où $R^4$ représente un alcoxy-carbonyle en $C_1$ à $C_6$-alcoyle en $C_1$ à $C_6$ et $R^1$, $R^2$ et $R^3$ ont la signification donnée dans la revendication 1, ou

h) pour préparer des composés de formule I où $R^4$ représente un hydrogène et où $R^1$, $R^2$ et $R^3$ ont la signification donnée dans la revendication 1, on oxyde avec du persulfate de potassium dans l'acide sulfurique concentré un composé de formule générale

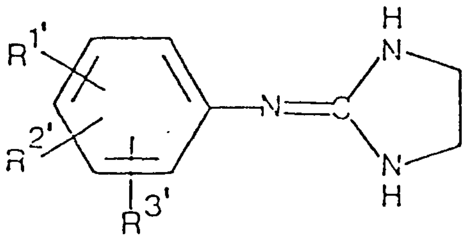

VII

où $R^{1'}$, $R^{2'}$, $R^{3'}$ représentent chacun indépendamment l'un de l'autre un alcoyle en $C_2$ à $C_6$, un alcoxy en $C_1$ à $C_6$ ou un trifluorométhyle, ou

i) pour préparer des composés de formule I ci-dessus, où $R^4$ représente un [5-hydroxy-4-(hydroxyméthyl)-6-méthyl-3-pyridyl]-méthyle et $R^1$, $R^2$ et $R^3$ ont la signification donnée dans la revendication 1, on sépare le groupe cétal dans un composé de formule générale

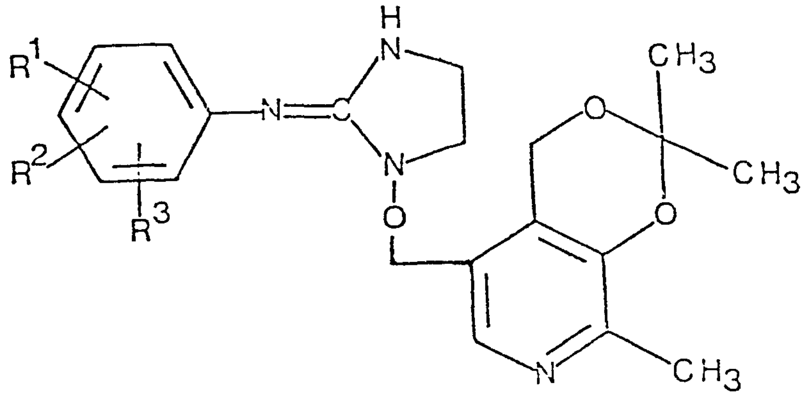

Id

où $R^1$, $R^2$ et $R^3$ ont la signification donnée dans la revendication 1, et

j) si on le désire on transforme un composé obtenu ou un sel d'addition acide de ce corps non pharmaceutiquement acceptable en un sel d'addition acide pharmaceutiquement acceptable de ce corps.

31. Procédé selon la revendication 30 de préparation de composés de formule I définie dans la revendication 2 ou de leurs sels d'addition acide pharmaceutiquement acceptables, caractérisé en ce qu'on effectue les variantes a), b), c), d), e), f), g) et j) du procédé de la revendication 30.

32. Procédé de préparation de préparations ayant des propriétés d'abaissement de la tension artérielle, caractérisé en ce qu'on mélange un dérivé de 2-imino-imidazolidine de formule I de la revendication I ou un de ses sels d'addition acide pharmaceutiquement acceptable comme composant actif avec des supports et/ou excipients solides et liquides appropriés à l'administration thérapeutique, non toxiques, inertes, habituels dans de telles préparations.

33. Préparations à effet d'abaissement de la tension artérielle contenant un dérivé de 2-imino-imidazolidine de formule I de la revendication 1 ou un de ses sels d'addition acide pharmaceutiquement acceptables et un support.